# FASCICULE DE BREVET EUROPEEN

(11) **EP 3 191 515 B1**
(45) Date de publication et mention de la délivrance du brevet: **30.12.2020**
(21) Numéro de dépôt: 15771200.1
(22) Date de dépôt: 10.09.2015
(51) Int. Cl.: C07K 16/26, A61K 39/395, A61K 38/24, A61P 5/06, A61P 5/24, A61P 15/08

(54) **LIGANDS POTENTIALISANTS DE LA BIOACTIVITE DES GONADOTROPHINES**
LIGANDEN ZUR VERSTÄRKUNG DER BIOAKTIVITÄT VON GONADOTROPINEN
LIGANDS THAT POTENTIATE THE BIOACTIVITY OF GONADOTROPINS

(30) Priorité: 10.09.2014 FR 1458469
(43) Date de publication de la demande: 19.07.2017
(73) Titulaire: IGYXOS, 37380 Nouzilly (FR)
(72) Inventeur: KARA, Elodie, 37250 Veigne (FR); DECOURTYE, Jérémye, 37000 Tours (FR); CASTERET, Sophie, 37190 Valleres (FR); MAUREL, Marie-Christine, 37000 Tours (FR)
(74) Mandataire: Novagraaf Technologies
(86) Numéro de dépôt international: PCT/FR2015/052413
(87) Numéro de publication internationale: WO 2016/038308

(56) Documents cités:
- WO-A1-97/12038
- WO-A1-2012/066519
- US-A1- 2002 102 613
- GLENCROSS R G ET AL: "Monoclonal antibody enhancement of FSH-induced uterine growth in snell dwarf mice", JOURNAL OF ENDOCRINOLOGY, SOCIETY FOR ENDOCRINOLOGY, GB, vol. 136, no. 3, 1 mars 1993 (1993-03-01), pages R5-R7, XP009145848, ISSN: 0022-0795, DOI: 10.1677/JOE.0.136R005
- ALFREDO ULLOA-AGUIRRE ET AL: "Novel pathways in gonadotropin receptor signaling and biased agonism", REVIEWS IN ENDOCRINE AND METABOLIC DISORDERS, KLUWER ACADEMIC PUBLISHERS, BO, vol. 12, no. 4, 28 avril 2011 (2011-04-28) , pages 259-274, XP019972343, ISSN: 1573-2606, DOI: 10.1007/S11154-011-9176-2
- DATABASE MEDLINE [Online] US NATIONAL LIBRARY OF MEDICINE (NLM), BETHESDA, MD, US; novembre 1987 (1987-11), MILLER K F ET AL: "Immunoaffinity chromatography of bovine FSH using monoclonal antibodies.", XP002751132, Database accession no. NLM3125301

## Description

### Domaine technique

La présente invention se rapporte à des anticorps dirigés contre l'hormone folliculo-stimulante (FSH) capables de potentialiser la bioactivité des gonadotrophines.

La présente invention trouve ses applications principalement en médecine humaine et vétérinaire, pour induire l'ovulation chez un mammifère femelle.

Dans la description ci-dessous, les références entre crochets ([ ]) renvoient à la liste des références présentée à la fin du texte.

### Etat de la technique

Les gonadotrophines (ou gonadotropines) sont des hormones glycoprotéiques complexes jouant un rôle central dans la régulation de la reproduction chez les vertébrés en agissant sur les fonctions des gonades (ovaires et testicules). Deux de ces hormones sont sécrétées chez tous les vertébrés : l'hormone lutéinisante (LH) et l'hormone folliculo-stimulante (FSH). Chez deux groupes de mammifères, équidés et primates, il existe en outre une gonadotrophine chorionique (CG) sécrétée par le placenta : la choriogonadotropine humaine (hCG) et la choriogonadotropine équine (eCG) qui agissent toutes deux via des récepteurs LH.

L'hormone lutéinisante (LH) est produite par les cellules gonadotropes du lobe antérieur de l'hypophyse sous stimulation de la GnRH, elle-même produite par l'hypothalamus. La LH stimule la production de testostérone chez les mâles, tandis qu'elle intervient dans les modifications du cycle ovarien chez les femelles où elle est responsable de la croissance folliculaire terminale et de l'ovulation puis de la transformation du follicule ovulatoire rompu en corps jaune. Pendant la phase lutéale du cycle menstruel, la LH stimule la sécrétion de progestérone par le corps jaune, indispensable au développement précoce et à l'implantation de l'embryon. La LH est constituée d'une sous-unité α commune à toutes les hormones glycoprotéiques d'une même espèce (comme la FSH, la CG et l'hormone thyréostimulante, la TSH) et d'une sous-unité β responsable de la spécificité d'activité de l'hormone ; activité qui n'existe que si les deux sous-unités sont associées de manière non covalente sous-forme d'un dimère.

L'hormone folliculo-stimulante (ou FSH) est produite par l'anté-hypophyse sous stimulation de la GnRH produite par l'hypothalamus. Chez les mâles, elle stimule les cellules de Sertoli indispensables à la spermatogénèse. Chez les femelles, elle est responsable du recrutement des follicules primordiaux, immatures, de leur croissance et de leur différentiation en follicules pré-ovulatoires en stimulant les récepteurs FSH des cellules de la granulosa. La FSH est constituée de deux sous-unités α et β, et a une structure semblable à celle de la LH. Seul le dimère est capable de stimuler les récepteurs FSH.

Chez les femelles, les taux de LH et de FSH sont cycliques : très faibles en période de repos sexuel ou en dehors de la période ovulatoire, avec un pic de sécrétion en période préovulatoire.

Les gonadotrophines sont utilisées en médecine vétérinaire et humaine, pour induire l'ovulation chez les mammifères femelles. Bien qu'efficaces, ces traitements présentent un risque sanitaire du fait de l'utilisation d'hormones extraites à partir de fluides biologiques (sang, urine) ou de tissus (hypophyses), particulièrement dans le domaine vétérinaire. C'est le cas de la chorionic gonadotropine équine (eCG) extraite à partir de sang de juments gravides, et de la LH et FSH porcines extraites à partir d'hypophyses de porc. Dans le domaine vétérinaire, on utilise également une hCG extraite à partir d'urine de femmes enceintes, le Chorulon® (Laboratoire MSD).

Dans le domaine de la clinique humaine, et particulièrement de la Procréation Médicalement Assistée (ou PMA), on utilise des hormones extraites à partir d'urine de femmes ménopausées telles que Fostimon® (Laboratoire Genévrier) qui est une FSH purifiée et Menopur® (Laboratoire Ferring Pharmaceuticals) qui est une hMG (human menoposal gonadotropin), mélange de FSH et de LH et la Gonadotropine Chorionique Endo5000 qui est une hCG purifiée (Laboratoire Schering-Plough). On utilise également des FSH humaines recombinantes, telles que Gonal-F® (Laboratoire Merck Serono) et Puregon® (Laboratoire Merck Shering-Plough); des hCG et LH recombinantes telle que Ovidrel® et Luveris® (Laboratoire Merck Serono).

En outre l'usage répété de ces hormones induit le plus souvent une réaction immunitaire qui vient neutraliser l'effet des hormones conduisant ainsi à une baisse d'efficacité thérapeutique. Cependant, il a également été mis en évidence dans quelques cas que la réaction immunitaire pouvait produire des anticorps capables de potentialiser l'activité de l'hormone lorsqu'elle était co-administrée (Brevet EP 1518863) [1]. Depuis, il a également été mis en évidence trois anticorps monoclonaux anti-LH capables de potentialiser son action ainsi que celle de la FSH pour deux d'entre eux (Demande Internationale WO 2012/066519) [2].

### Description de l'invention

Les Inventeurs ont maintenant obtenu des anticorps monoclonaux produits contre la sous-unité β de la FSH, capables de potentialiser son action ainsi que celle de la LH et de la hCG.

Ces anticorps monoclonaux sont dénommés CF12.

L'hybridome qui a produit l'anticorps CF12 a été déposé conformément au Traité de Budapest, le 03/10/2013 auprès de la CNCM (Collection Nationale de Culture de Microorganismes, Institut Pasteur, 25 rue du Docteur Roux, 75724 Paris Cedex 15, France), sous le numéro CNCM I-4803.

Les séquences nucléotidiques des régions variables des chaines lourdes et légères de l'anticorps CF12 ont été déterminées, les séquences peptidiques correspondantes déduites. Elles sont présentées dans le tableau 1 ci-dessous.

**Tableau 1**

| Anticorps monoclonal CF12 | |
|---|---|
| Chaîne lourde (VH) | |
| Séquence nucléotidique (SEQ ID NO : 1) | CAGGGTCAGATGCAGCAGTCTGGAGCTGAGCTGGTGAAGCCT GGGGCTTCAGTGAAGCTGTCCTGCAAGACTTCTGGCTTCACCT TCAGCAGTAGCTATATAAGTTGGTTGAAGCAAAAGCCTGGACAG AGTCTTGAGTGGATTGCATGGATTTATGCTGGAACTGGTGGTAC TAGCTATAATCAGAAGTTCACAGGCAAGGCCCAACTGACTGTAG ACACATCCTCCAGCACAGCCTACATGCAATTCAGCAGCCTGAC AACTGAGGACTCTGCCATCTATTACTGTGCAAGACACGGGTCCT ACTTTGACTACTGGGGCCAAGGCACCACTCTCACAGTCTCCTC A |
| Séquence peptidique (SEQ ID NO : 2) | QGQMQQSGAELVKPGASVKLSCKTSGFTFSSSYISWLKQKPGQS LEWIAWIYAGTGGTSYNQKFTGKAQLTVDTSSSTAYMQFSSLTTE DSAIYYCARHGSYFDYWGQGTTLTVSS |

| Chaîne légère (VL) | |
|---|---|
| Séquence nucléotidique (SEQ ID NO : 3) | GACATTGTGCTGACCCAATCTCCAGCTTCTTTGGCTGTGTCTCT AGGGCAGAGGGCCACCATCTCCTGCAAGGCCAGCCAAAGTGTT GATTATGATGGTGATAGTTATATGAACTGGTACCAACAGAAACC AGGACAGCCACCCAAACTCCTCATCTATGCTGCATCCAATCTAG AATCTGGGATCCCAGCCAGGTTTAGTGGCAGTGGGTCTGGGAC AGACTTCACCCTCAACATCCATCCTGTGGAGGAGGAGGATGCT GCAACCTATTACTGTCAGCAAAGTAATGAGGATCCGTACACGTT CGGAGGGGGGACCAAGCTGGAAATAAAA |
| Séquence peptidique (SEQ ID NO : 4) | DIVLTQSPASLAVSLGQRATISCKASQSVDYDGDSYMNWYQQKPG QPPKLLIYAASNLESGIPARFSGSGSGTDFTLNIHPVEEEDAATYYC QQSNEDPYTFGGGTKLEIK |

Les séquences codant pour les CDRs (régions déterminant la complémentarité) ont été déterminées à partir des séquences des régions variables des chaînes lourdes (VH-CDR) et légères (VL-CDR) de l'anticorps CF12 ci-dessus. Les séquences peptidiques correspondantes ont été déduites, et sont présentées respectivement dans le tableau 2 ci-dessous.

**Tableau 2**

| Anticorps monoclonal CF12 | |
|---|---|
| VH-CDR1 (SEQ ID NO : 5) | GFTFSSSY |
| VH-CDR2 (SEQ ID NO : 6) | IYAGTGGT |
| VH-CDR3 (SEQ ID NO : 7) | ARHGSYFDY |
| VL-CDR1 (SEQ ID NO : 8 | QSVDYDGDSY |
| VL-CDR2 | AAS |
| VL-CDR3 (SEQ ID NO : 9) | QQSNEDPYT |

Un ligand de l'hormone folliculo-stimulante (FSH) potentialisant la bioactivité de la FSH, de l'hormone lutéinisante (LH) et de la gonadotropine chorionique (CG), caractérisé en ce qu'il comprend le paratope d'un anticorps anti-sous-unité β de la FSH, est décrit.

On entend par « anticorps anti-sous-unité β de la FSH » au sens de la présente invention, tout anticorps obtenu par immunisation d'un animal à partir de premières injections de FSH suivies de plusieurs rappels avec injection de la sous-unité β de la FSH. Les injections peuvent être réalisées à partir de FSH de différents mammifères, par exemple de FSH ovine, humaine, bovine, caprine ou porcine, équine, canine, murine etc... et des sous-unités β de la FSH d'origine homologue ou hétérologue. Ainsi l'anticorps monoclonal CF12 a été obtenu suite à une immunisation à partir de FSH humaine et de sous-unité β de la FSH humaine.

La présente invention a donc pour objet un ligand de l'hormone folliculo-stimulante (FSH) potentialisant la bioactivité de la FSH, de l'hormone lutéinisante (LH) et de la gonadotropine chorionique (CG), caractérisé en ce que le ligand est un anticorps ou un fragment de celui-ci et en ce que :
le domaine variable de la chaine lourde contient les CDRs suivants :
   - VH-CDR1, défini par la séquence GFTFSSSY (SEQ ID NO : 5) ;
   - VH-CDR2, défini par la séquence IYAGTGGT (SEQ ID NO : 6) ;
   - VH-CDR3, défini par la séquence ARHGSYFDY (SEQ ID NO : 7) ; et
le domaine variable de la chaine légère contient les CDRs suivants :
   - VL-CDR1, défini par la séquence QSVDYDGDSY (SEQ ID NO : 8) ;
   - VL-CDR2, défini par la séquence AAS ;
   - VL-CDR3, défini par la séquence QQSNEDPYT (SEQ ID NO : 9).

On entend par « CDR » au sens de la présente invention, les trois régions hypervariables des régions variables des chaînes lourdes et légères d'un anticorps qui constituent les éléments du paratope et permettent de déterminer la complémentarité de l'anticorps avec l'épitope de l'antigène. Ces trois régions hypervariables sont encadrées par quatre régions constantes qui constituent la « charpente » (FR ou framework regions) et donnent une configuration stable au domaine variable.

En particulier, la présente invention a donc pour objet un ligand selon la revendication 2.

Un ligand selon la présente invention est par exemple :
- l'anticorps monoclonal CF12 produit par l'hybridome CNCM I-4803 ;
- un fragment VH ou VL d'un anticorps ci-dessus utilisé seul ou en mélange ;
- un fragment Fab, Fab', F(ab')2, Fv, dsFv ou scFv, d'un anticorps ci-dessus. De préférence, il s'agit d'un fragment Fab ou d'un fragment scFv, par exemple un fragment scFv de séquence peptidique SEQ ID NO : 11 ;
- une forme bi-, tri- ou tétravalente (diabodies, triabodies, tétrabodies) de deux, trois ou quatre fragments de scFv, respectivement.
- Un anticorps recombinant comprenant le paratope d'un anticorps ci-dessus et dont les régions constantes ont été modifiées de sorte à minimiser l'immunogénicité vis-à-vis de l'animal ou de l'homme auquel il est destiné, est décrit. Par exemple, il s'agit d'un anticorps chimérique (humanisé, ovinisé, caprinisé, bovinisé, porcinisé etc...) ou entièrement humanisé, ovinisé, caprinisé, bovinisé, porcinisé

A titre d'exemple non limitatif, les séquences nucléotidiques de scFv dérivés de l'anticorps CF12 ont été déterminées, les séquences peptidiques correspondantes déduites, et sont présentées respectivement dans le tableau 3 ci-dessous.

**Tableau 3**

| scFv CF12 | |
|---|---|
| Séquence nucléotidique (SEQ ID NO : 10) | CAGGTGCAGCTGCAGCAGTCGGGTGGCGCAGAGCTGGTGAAA CCGGGTGCGAGCGTTAAACTGAGCTGCAAAACTAGCGGCTTTA CCTTTAGCTCGTCATATATTTCGTGGCTGAAGCAGAAACCGGGC CAGTCACTGGAATGGATTGCGTGGATCTACGCAGGCACGGGTG GCACCTCATATAATCAGAAATTCACCGGTAAAGCGCAACTGACG GTCGATACCAGCAGCAGCACGGCGTACATGCAGTTCAGCTCGC TGACCACTGAAGATAGCGCAATCTACTATTGTGCACGCCATGGT TCGTACTTCGACTATTGGGGCCAGGGCACCACCCTGACCGTTT CAAGCGGTGGTGGTGGTAGCGGTGGTGGTGGTTCAGGTGGCG GCGGCTCAGATATTCAGATGACCCAGACCCCTGCGAGCCTGGC AGTGTCACTGGGCCAACGCGCAACCATCTCGTGTAAAGCCTCG CAGAGCGTGGATTATGACGGCGATAGCTACATGAACTGGTATC AGCAAAAGCCTGGTCAACCGCCGAAGCTGCTGATTTACGCCGC CAGCAACCTGGAATCGGGCATCCCGGCCCGTTTTAGCGGCTCA GGCTCGGGTACTGACTTCACGCTGAACATTCACCCGGTAGAAG AAGAAGACGCGGCCACGTATTACTGCCAGCAAAGCAATGAAGA CCCGTACACTTTTGGCGGCGGCACGAAACTCGAGATCAAACAC CATCACCATCACCATTAACTCGAGATCAAGTAA |
| Séquence peptidique (SEQ ID NO 11) | QVQLQQSGGAELVKPGASVKLSCKTSGFTFSSSYISWLKQKPGQS LEWIAWIYAGTGGTSYNQKFTGKAQLTVDTSSSTAYMQFSSLTTE DSAIYYCARHGSYFDYWGQGTTLTVSSGGGGSGGGGSGGGGSD IQMTQTPASLAVSLGQRATISCKASQSVDYDGDSYMNWYQQKPG QPPKLLIYAASNLESGIPARFSGSGSGTDFTLNIHPVEEEDAATYYC QQSNEDPYTFGGGTKLEIKHHHHHH |

Une séquence nucléotidique codant pour un ligand selon l'invention, est décrite.

Un vecteur recombinant, en particulier un vecteur d'expression, comprenant une séquence nucléotidique telle que décrite ci-dessus, est décrit.

Une cellule hôte comprenant une séquence nucléotidique telle que décrite ci-dessus ou un vecteur recombinant tel que décrit ci-dessus, est décrite. Par exemple, il s'agit de l'hybridome CNCM I-4803 ou d'une cellule transformée par une séquence nucléotidique ou un vecteur recombinant selon l'invention.

Un procédé de production d'un ligand selon l'invention, caractérisé en ce qu'il comprend la mise en culture dans un milieu approprié de cellules hôte telles que décrites ci-dessus, et la récupération dudit ligand à partir de ladite culture, est décrit.

Les Inventeurs ont mis en évidence que l'anticorps CF12 potentialise peu bien que significativement la FSH porcine, ovine et bovine contrairement à la FSH humaine qu'il potentialise fortement. En outre, les Inventeurs ont mis en évidence que le scFv dérivé de l'anticorps CF12, possède les mêmes propriétés de liaison et de potentialisation que les anticorps dont ils dérivent.

La présente invention a également pour objet un complexe formé d'un ligand et d'une gonadotrophine, ou d'un peptide actif de celle-ci, capable de se lier audit ligand et dont l'activité est potentialisée par ledit ligand. Par exemple, il s'agit du complexe d'un ligand avec la LH, avec l'hormone gonadotropine chorionique (CG), ou avec la FSH extraites de tissus ou de fluides biologiques, ou recombinantes, ou d'un peptide actif de celles-ci capable de se lier audit ligand et dont l'activité est potentialisée par ledit ligand.

La présente invention a également pour objet un ligand ou complexe selon l'invention pour utilisation comme médicament, en particulier pour potentialiser la bioactivité de la FSH, de la LH, et de la gonadotropine chorionique (CG) pour induire une ovulation voire une polyovulation chez un mammifère femelle ou pour réduire les problèmes d'infertilité ou d'hypofertilité hormono dépendants chez un mammifère mâle ou femelle. Ledit médicament permet également d'augmenter le taux de progestérone endogène circulant sécrété par un ou plusieurs corps jaunes chez un mammifère femelle, favorisant ainsi le développement embryonnaire précoce et diminuant le risque d'avortement.

Un procédé de production carnée, où ledit procédé comprend l'administration de ligand et/ou de complexe de l'invention à un mammifère femelle animal non humain, est décrit.

La présente invention a également pour objet un ligand et/ou complexe de l'invention pour une utilisation dans le traitement de l'infertilité ou de l'hypofertilité hormono dépendante chez un mammifère. Dans le cas d'un mammifère femelle souffrant d'infertilité ou d'hypofertilité, l'administration du ligand ou complexe de l'invention va permettre de stimuler une procréation naturelle, médicalement assistée ou artificielle. Il est à noter que l'administration du ligand ou complexe de l'invention à un mammifère femelle sain va également permettre de déclencher l'ovulation dans le cadre d'une procréation naturelle ou artificielle.

On entend par « infertilité/hypofertilité hormono dépendante » au sens de la présente invention, une infertilité/hypofertilité due à une insuffisance hormonale par exemple de faibles concentrations circulantes de FSH et LH ou une absence de ces hormones résultant par exemple d'une cause externe (par exemple les pesticides) ou interne (par exemple, insuffisance hypophysaire ou hypothalamique ou d'un problème de réceptivité des gonades à la LH et/ou la FSH due à une anomalie des récepteurs ou des gonadotropines LH, FSH, CG par exemple une mutation ou un polymorphisme des récepteurs).

Les ligands et complexes de l'invention peuvent être utilisés chez l'homme ou l'animal, en particulier les ovins, bovins, caprins, équins, porcins, murins, canins, camelins etc...

Les ligands, les hormones ou les complexes selon l'invention peuvent être administrés soit séparément, soit séquentiellement, soit conjointement, par injection, par exemple intramusculaire, intraveineuse, intrapéritonéale, sous-cutanée, transcutanée, intradermique, intraorbitaire, intraoculaire, ophtalmique, ou par voie transoculaire, sans altérer leur effet potentialisant.

La présente invention a également pour objet une composition pharmaceutique comprenant un ligand ou complexe de l'invention et un véhicule pharmaceutiquement acceptable. Ladite composition pharmaceutique peut comprendre en outre une FSH et/ou une LH et/ou une hormone gonadotropine chorionique (CG).

D'autres avantages pourront encore apparaître à l'homme du métier à la lecture des exemples ci-dessous, illustrés par les figures annexées, donnés à titre illustratif.

### Brève description des figures

- La figure 1 illustre l'effet potentialisant *in vitro* de l'anticorps monoclonal CF12 sur la bioactivité de la FSH humaine (hFSH) sur des cellules de granulosa bovines.
- La figure 2 représente l'effet potentialisant *in vitro* de l'anticorps monoclonal CF12 sur la bioactivité de la FSH humaine (hFSH) sur une lignée cellulaire HEK 293 transfectée de façon stable avec le récepteur FSH humain.
- La figure 3 représente l'effet potentialisant *in vitro* de l'anticorps monoclonal CF12 sur la bioactivité de la FSH humaine (hFSH) sur une lignée cellulaire HEK 293 transfectée de façon stable avec le récepteur FSH humain et le vecteur Glosensor®.
- La figure 4 représente l'effet potentialisant *in vitro* de l'anticorps monoclonal CF12 et du scFv CF12 sur la bioactivité de la FSH humaine (hFSH) sur une lignée cellulaire HEK 293 transfectée de façon stable avec le récepteur FSH humain et le vecteur Glosensor®.
- La figure 5 représente l'effet potentialisant *in vitro* de l'anticorps monoclonal CF12 sur la bioactivité de la FSH ovine (oFSH) sur une lignée cellulaire HEK 293 transfectée de façon stable avec le récepteur FSH humain et le vecteur Glosensor®.
- La figure 6 représente l'effet potentialisant *in vitro* de l'anticorps monoclonal CF12 sur la bioactivité de la FSH porcine (pFSH) sur une lignée cellulaire HEK 293 transfectée de façon stable avec le récepteur FSH humain et le vecteur Glosensor®.
- La figure 7 représente l'effet potentialisant *in vitro* de l'anticorps monoclonal CF12 sur la bioactivité des FSH humaines (hFSH) Gonal-F® (B) et Fostimon® (C) sur des cellules de granulosa humaines.
- La figure 8 représente l'effet potentialisant *in vitro* de l'anticorps monoclonal CF12 sur la bioactivité de la FSH humaine (hFSH) Gonal-F® sur des cellules de granulosa humaines.
- La figure 9 représente l'effet potentialisant *in vivo* de l'anticorps monoclonal CF12 sur la bioactivité des FSH humaines (hFSH) Gonal-F®, Puregon® et Fostimon® (A) et de la FSH ovine (oFSH) (B) chez le rat femelle.
- La figure 10 représente l'effet potentialisant *in vivo* du scFv CF12 sur la bioactivité des FSH humaines (hFSH) Gonal-F® (A et B), Puregon® et Fostimon® (A) et de l'anticorps monoclonal CF12 sur la bioactivité de la FSH humaine (hFSH) Gonal-F selon différents modes d'administration (C) chez le rat femelle.
- La figure 11 représente l'effet potentialisant *in vivo* de l'anticorps monoclonal CF12 sur la bioactivité des choriogonadotropines humaines (hCG) Chorulon® et Endo 5000® chez le rat mâle.
- La figure 12 représente l'effet potentialisant *in vivo* de l'anticorps monoclonal CF12 sur la bioactivité des gonadotropines endogènes chez la brebis en période de saison sexuelle.
- La figure 13 représente l'effet potentialisant *in vivo* de l'anticorps monoclonal CF12 injecté seul après 25 UI de hFSH, sur la stimulation folliculaire chez la guenon.
- La figure 14 représente l'effet potentialisant *in vivo* de l'anticorps monoclonal CF12 injecté seul après 37,5 UI de hFSH, sur la stimulation folliculaire chez la guenon
- La figure 15 représente l'effet potentialisant *in vivo* de l'anticorps monoclonal CF12 injecté seul après 37,5 UI de hFSH, sur la sécrétion d'oestradiol et de progestérone chez la guenon
- La figure 16 représente l'épitope conformationnel du ligand CF12, sur les hormones hFSH, hCG, hLH, oLH, pLH, oFSH, pFSH et le récepteur FSH humain.
- La figure 17 représente l'effet potentialisant *in vitro* de différents fragments de l'anticorps monoclonal CF12 sur la bioactivité de la hFSH
- La figure 18 représente l'effet potentialisant *in vivo* chez la ratte de différents fragments de l'anticorps monoclonal CF12 sur la bioactivité de la hFSH

### EXEMPLES

### EXEMPLE 1 : OBTENTION DES LIGANDS DE L'INVENTION, ET LEUR CARACTERISATION

### 1/ Stratégie d'immunisation des souris

Les injections ont toutes été réalisées par voie intra-péritonéale sur des souris (Balb/C). Cinq souris ont été utilisées.

### Stratégie d'immunisation des souris pour l'anticorps CF12

L'immunisation a été réalisée avec plusieurs injections de FSH humaine recombinante (hFSHr). Une première injection (J0) a été réalisée avec 50µg de hFSHr avec adjuvant de Freund complet. Plusieurs injections de rappel ont été ensuite réalisées selon la séquence suivante :
- J21 et J35 : injection de rappel de 50µg de hFSHr avec adjuvant de Freund incomplet ;
- J55, J56 et J57 : injection de 30µg de sous-unité béta de hFSHr sans adjuvant ;
- J58 : fusion.

### 2/ Isotypage

L'isotypage de l'anticorps CF12 a été réalisé avec le kit d'isotypage FastElysa commercialisé par RD Biotech (référence RDB 3255) en suivant les recommandations du fabriquant.

L'anticorps CF12 est une immunoglobine de classe IgM et d'isotype Kappa. Les valeurs des densités optiques (DO) obtenues ont été 0,639 et 0,6 respectivement.

### 3/ Séquencage

Les séquences nucléotidiques de la partie variable des chaînes lourdes (VH) et légères (VL) de l'anticorps CF12 sécrété par l'hybridome CNCM I-4803, ont été déterminées à partir de leur ARN messager (ARNm) selon le protocole ci-après.

Les ARN ont été extraits des cellules à l'aide du kit Nucleospin® RNA (Macherey Nagel, Allemagne) en suivant les recommandations du fabriquant. Les concentrations en ARN purifiés ont été estimées par mesure de l'absorbance (A) à 260 nm et leur qualité par le rapport A260nm/280nm et visuellement après migration électrophorétique sur gel d'agarose.

Les ADN complémentaires des ARNm ont alors été synthétisés à l'aide d'un oligo-dT₁₈ comme amorce par réaction de rétrotranscription avec l'enzyme M-MLV (Réf. M1701, Promega, USA) en suivant les recommandations du fabriquant.

La synthèse du second brin d'ADN a été réalisée par une réaction de polymérisation en chaîne (PCR) selon le protocole suivant : à 4 µl de la réaction de rétrotranscription sont ajoutés dans un volume final de 50 µl ; le tampon de réaction (1X final), 200 µM de chaque dNTPs, 300 nM d'amorces sens et antisens, 1,25 U de GoTaq polymérase (Ref M3175, Promega, USA).

Pour l'amplification de la partie variable des chaînes légères, 5 couples d'amorces différents ont été utilisés (MKRev2 à 8 + MKC5For) et 2 couples pour celles des chaînes lourdes (VHRev1 ou VHRev2 + MµCFοr).

**Tableau 4 : Séquences nucléotidiques des amorces utilisées pour séquencer les chaînes lourdes (VH) et légères (VL) de l'anticorps CF12.**

| Anticorps CF12 | | |
|---|---|---|
| Chaîne lourde (VH) | | |
| **Nom** | **Séquence 5'-3'** | **SEQ ID NO** |
| **VHRev1** | CGGGATCCTCTAGAGGTCCAACTGCAGGAGTCA GG | SEQ ID NO : 12 |
| **VHRev2** | AGATCTAGAAAGCTTAGGTCAAGCTGCAGCAGTC AGG | SEQ ID NO : 13 |
| **MµCFor** | GGGGAAGACATTTGGGAAGG | SEQ ID NO : 14 |

| Chaîne légère (VL) | | |
|---|---|---|
| **MKRev2** | GATATTGTGATGACGCAGGCT | SEQ ID NO : 15 |
| **MKRev3** | GATATTGTGATAACCCAG | SEQ ID NO : 16 |
| **MKRev4** | GACATTGTGCTGACCCAATCT | SEQ ID NO : 17 |
| **MKRev5** | GACATTGTGATGACCCAGTCT | SEQ ID NO : 18 |
| **MKRev8** | GACATCCAGCTGACTCAGTCT | SEQ ID NO : 19 |
| **MKC5For** | GGATACAGTTGGTGCAGCATC | SEQ ID NO : 20 |

**Tableau 5 : Séquences nucléotidiques des amorces utilisées pour séquencer la partie 5' des chaînes lourdes (VH) et légères (VL) de l'anticorps CF12.**

| Anticorps CF12 | | |
|---|---|---|
| Chaîne lourde (VH) | | |
| **Nom** | **Séquence 5'-3'** | **SEQ ID NO** |
| **CF12VH_Fw** | CAGKAACTGCAGGTGTCCWCT | SEQ ID NO : 21 |
| **CF12VH_Rev** | CTGGAGGATGTGTCTACAGTCAG | SEQ ID NO : 22 |

| Chaîne légère (VL) | | |
|---|---|---|
| **CF12VL_Fw** | CTGCTATGGGTGCTGCTGCTC | SEQ ID NO : 23 |
| **CF12VL_Rev** | AGATTGGATGCAGCATAGATGAG | SEQ ID NO : 24 |

Le programme de PCR utilisé est composé d'une dénaturation initiale de 2 min à 95°C suivie de 30 cycles de dénaturation 30 sec à 95°C, hybridation 30 sec à 47°C et amplification 1 min à 72°C et enfin d'une amplification finale de 5 min à 72°C. Les produits de PCR obtenus ont été dessalés avec le kit QIAquick®Gel extraction kit (Ref 28704, Qiagen GmbH, Allemagne) puis ligaturés avec le plasmide pGEMT easy vector (Ref A1360, Promega, USA) pour être transformés en bactéries. L'ADN plasmidique extrait de différents clones bactériens a été envoyé pour analyse par séquençage (Macrogen Europe, Pays-Bas).

Les séquences nucléotidiques 5' terminales des VH et VL de l'anticorps CF12 ont par la suite été déterminées grâce à la conception d'amorces spécifiques ancrées dans les séquences leader des ADNc (amorce Fw). Ces amorces ont été conçues suite à l'identification d'homologie par alignement entre les séquences VL et VH obtenues précédemment et la base de données du logiciel IMGT/V-QUEST (Brochet et al., Nucl. Acids Res., 36 : W503-508, 2008; Giudicelli et al.,, Cold Spring Harb Protoc., 2011(6) : 695-715, 2011) [3, 4] et à l'extraction des séquences leader d'intérêt de IMGT/GENE-DB (Giudicelli et al., Nucl. Acids Res., 33 : D256-261, 2005) [5]. Les amorces anti-sens (Rev) ont été conçues dans les séquences VH et VL respectives précédemment déterminées de chacun des anticorps. Le protocole utilisé pour obtenir la partie 5'est le même que celui décrit au paragraphe précédent.

Les séquences nucléotidiques consensus ont été déduites de l'alignement des séquences en utilisant le logiciel MultAlin (Corpet, Nucl. Acids Res., 16(22) : 10881-10890, 1988) [6]. La transcription en séquences polypeptidiques et l'annotation des CDRs ont été réalisées à l'aide de logiciel IMGT/V-QUEST. Les résultats sont présentés dans les tableaux 6 et 7.

**Tableau 6 : Séquences nucléotidiques et peptidiques des parties variables lourdes (VH) et légères(VL) de l'anticorps CF12.**

| Anticorps CF12 | |
|---|---|
| Chaîne lourde (VH) | |
| Séquence nucléotidique SEQ ID NO : 1 | CAGGGTCAGATGCAGCAGTCTGGAGCTGAGCTGGTGAAGCCTGG GGCTTCAGTGAAGCTGTCCTGCAAGACTTCTGGCTTCACCTTCAG CAGTAGCTATATAAGTTGGTTGAAGCAAAAGCCTGGACAGAGTCT |
| | TGAGTGGATTGCATGGATTTATGCTGGAACTGGTGGTACTAGCTA TAATCAGAAGTTCACAGGCAAGGCCCAACTGACTGTAGACACATC CTCCAGCACAGCCTACATGCAATTCAGCAGCCTGACAACTGAGGA CTCTGCCATCTATTACTGTGCAAGACACGGGTCCTACTTTGACTA CTGGGGCCAAGGCACCACTCTCACAGTCTCCTCA |
| Séquence peptidique SEQ ID NO : 2 | QGQMQQSGAELVKPGASVKLSCKTSGFTFSSSYISWLKQKPGQSLE WIAWIYAGTGGTSYNQKFTGKAQLTVDTSSSTAYMQFSSLTTEDSAI YYCARHGSYFDYWGQGTTLTVSS |

| Chaîne légère (VL) | |
|---|---|
| Séquence nucléotidique SEQ ID NO :3 | GACATTGTGCTGACCCAATCTCCAGCTTCTTTGGCTGTGTCTCTA GGGCAGAGGGCCACCATCTCCTGCAAGGCCAGCCAAAGTGTTGA TTATGATGGTGATAGTTATATGAACTGGTACCAACAGAAACCAGG ACAGCCACCCAAACTCCTCATCTATGCTGCATCCAATCTAGAATCT GGGATCCCAGCCAGGTTTAGTGGCAGTGGGTCTGGGACAGACTT CACCCTCAACATCCATCCTGTGGAGGAGGAGGATGCTGCAACCT ATTACTGTCAGCAAAGTAATGAGGATCCGTACACGTTCGGAGGGG GGACCAAGCTGGAAATAAAA |
| Séquence peptidique SEQ ID NO : 4 | DIVLTQSPASLAVSLGQRATISCKASQSVDYDGDSYMNWYQQKPGQ PPKLLIYAASNLESGIPARFSGSGSGTDFTLNIHPVEEEDAATYYCQQ SNEDPYTFGGGTKLEIK |

**Tableau 7 : CDR des parties variables lourdes (VH) et légères(VL) de l'anticorps**

| | |
|---|---|
| VH-CDR1 (SEQ ID NO: 5) | GFTFSSSY |
| VH-CDR2 (SEQ ID NO: 6) | IYAGTGGT |
| VH-CDR3 (SEQ ID NO: 7) | ARHGSYFDY |
| VL-CDR1 (SEQ ID NO: 8) | QSVDYDGDSY |
| VL-CDR2 | AAS |
| VL-CDR3 (SEQ ID NO: 9) | QQSNEDPYT |

### 4/ Construction, production et caractérisation des scFv

### a/ Construction des fragments d'anticorps scFv

Les gènes de synthèse des fragments variables simple chaîne (scFv) dérivés de l'anticorps CF12 ont été synthétisés par ATG:Biosynthetics GmbH (Allemagne).

Chaque séquence a été conçue de la fusion des parties variables lourdes et légères (SEQ ID N0 : 1 / SEQ ID N0 : 3) liées par une séquence codant pour le peptide (Gly₄Ser)₃ assurant la fonctionnalité de la protéine et terminées par une séquence codant le peptide His₆ (peptide HIS-tag) qui autorisera la purification des scFvs. Afin de permettre leur insertion dans le plasmide d'expression, les séquences ont été flanquées par les sites enzymatiques de restriction Pstl et Sall. Une séquence additionnelle a été ajoutée entre l'extrémité 3' du VL et le site Sall autorisant la suppression du peptide His₆ si désiré. Les codons ont été optimisés pour l'expression en *E. coli.* Une représentation schématique de la construction des gènes de synthèse des scFvs est détaillée ci-dessous :

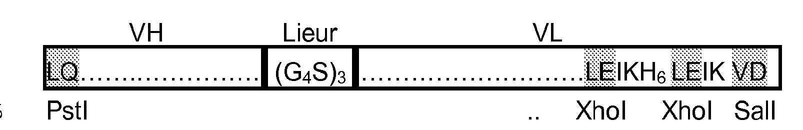

Les fragments d'anticorps ont été insérés entre les sites enzymatiques Pstl et Xhol du plasmide d'expression pSW1 (ATG:Biosynthetics GmbH, Allemagne) selon E. S. Ward et collaborateurs (Ward et al., Nature, 341: 544-546, 1989) [7] qui contient sous le contrôle d'un promoteur inductible LacZ, une séquence signal PelB qui fusionnée en phase de lecture avec le gène du fragment d'anticorps recombinant, permet l'adressage de la protéine synthétisée vers le périplasme bactérien. Dans le périplasme, cette séquence signal est éliminée par une peptidase.

Après contrôle par séquençage de la qualité des constructions, les plasmides pSW1-CA5, pSW1-CH10 et pSW1-CF12 ont été transformés par choc thermique en bactéries HB2151 (T53040, Interchim, France) rendues compétentes (Li et al., Afr. J. Biotechnol., 9(50) : 8549-8554, 2010) [8].

**Tableau 8 : Séquences nucléotidique et peptidique du scFv CF12.**

| scFv CF12 | |
|---|---|
| Séquence nucléotidique SEQ ID NO : 10 | CAGGTGCAGCTGCAGCAGTCGGGTGGCGCAGAGCTGGTGAAA CCGGGTGCGAGCGTTAAACTGAGCTGCAAAACTAGCGGCTTTA CCTTTAGCTCGTCATATATTTCGTGGCTGAAGCAGAAACCGGGC CAGTCACTGGAATGGATTGCGTGGATCTACGCAGGCACGGGTG GCACCTCATATAATCAGAAATTCACCGGTAAAGCGCAACTGACG GTCGATACCAGCAGCAGCACGGCGTACATGCAGTTCAGCTCGC |
| | TGACCACTGAAGATAGCGCAATCTACTATTGTGCACGCCATGGT TCGTACTTCGACTATTGGGGCCAGGGCACCACCCTGACCGTTTC AAGCGGTGGTGGTGGTAGCGGTGGTGGTGGTTCAGGTGGCGG CGGCTCAGATATTCAGATGACCCAGACCCCTGCGAGCCTGGCA GTGTCACTGGGCCAACGCGCAACCATCTCGTGTAAAGCCTCGC AGAGCGTGGATTATGACGGCGATAGCTACATGAACTGGTATCAG CAAAAGCCTGGTCAACCGCCGAAGCTGCTGATTTACGCCGCCA GCAACCTGGAATCGGGCATCCCGGCCCGTTTTAGCGGCTCAGG CTCGGGTACTGACTTCACGCTGAACATTCACCCGGTAGAAGAAG AAGACGCGGCCACGTATTACTGCCAGCAAAGCAATGAAGACCC GTACACTTTTGGCGGCGGCACGAAACTCGAGATCAAACACCATC ACCATCACCATTAACTCGAGATCAAGTAA |
| Séquence peptidique SEQ ID NO : 11 | QVQLQQSGGAELVKPGASVKLSCKTSGFTFSSSYISWLKQKPGQS LEWIAWIYAGTGGTSYNQKFTGKAQLTVDTSSSTAYMQFSSLTTED SAIYYCARHGSYFDYWGQGTTLTVSSGGGGSGGGGSGGGGSDIQ MTQTPASLAVSLGQRATISCKASQSVDYDGDSYMNWYQQKPGQP PKLLIYAASNLESGIPARFSGSGSGTDFTLNIHPVEEEDAATYYCQQ SNEDPYTFGGGTKLEIKHHHHHH |

### b/ Production des fragments d'anticorps recombinants

### - Culture bactérienne

Une pré-culture a été réalisée dans 5 ml de milieu 2xYT contenant 50 µg/ml d'ampicilline toute la nuit à 37°C. Le lendemain, 500 µl de cette préculture a été ensemencée dans 500 ml du même milieu et mis à croître à 37°C à 150 RPM jusqu'à obtenir une DO₆₀₀ₙₘ de 1,4. La synthèse du scFv a été induite par l'ajout de 0,1 mM d'IPTG 16 h à 16°C à 150 RPM.

### - Extraction

Le milieu de culture a été centrifugé 30 min à 4500 g à 4°C. La suite de la préparation a été réalisée à 4°C. Pour extraire le périplasme bactérien, le culot a été remis en suspension et incubé dans 10 ml de TES (Tris 0,2 M pH8, EDTA 0,5M, saccharose 0,5 M) pendant 30 min auxquels ont alors été ajoutés 15 ml de TES dilué au ¼ pour être de nouveau incubé 30 min. L'extrait bactérien a été centrifugé 30 min à 10 000g. Le surnageant a été mis à dialyser contre du PBS pendant une nuit. Le surnageant dialysé a été traité immédiatement pour purifier le scFv ou conservé à -20°C jusqu'à utilisation.

La production du scFv dans le périplasme a été analysée par Western blot en utilisant un anticorps anti - His-Tag HRP (Ref R93125 Life technologies, France) selon les conseils d'utilisation du fabricant.

### - Purification

Le périplasme a été centrifugé pendant 20 min à 5 000 g à 4°C. Le surnageant a été incubé avec HIS-Select® Nickel Affinity Gel (Sigma-Aldrich, MO, USA) sous agitation pendant 1h à 4°C. Le gel a été lavé avec un tampon phosphate de sodium 0,05 M, NaCl 0,3 M pH8 puis le même tampon additionné de 20 mM d'imidazole jusqu'à obtenir une DO₂₈₀ₙₘ proche de 0. Le scFv a alors été élué avec un tampon phosphate de sodium 0,05 M, NaCl 0,3 M, 250 mM imidazole pH8. L'éluat a été dialysé contre du PBS toute la nuit. Il est conservé à -20°C.

### - Contrôle qualité

Le scFv purifié a été analysé par électrophorèse sur gel de polyacrylamide à 15% après coloration au bleu de Coomassie et par chromatographie d'exclusion sur colonne Sephadex™ 75 10/300 GL (Ref 17-5174-01 GE Healthcare, Allemagne).

### 5/ Spécificité

La spécificité de l'anticorps CF12 et de son scFv a été étudiée par technique ELISA. Chaque hormone évaluée a été préparée à la concentration de 10 µg/ml dans du tampon carbonate de sodium 0,1M pH 9,6 et distribuée à raison de 100 µl par puits sur une plaque ELISA. Le temps d'adsorption a été de 18 heures à +4°C. Après cinq lavages, les puits ont été traités avec 100 µl de PBS additionné de Tween 0,1% et de BSA 1% pendant 45 min à 37°C, puis chaque anticorps ou scFv a été distribué à raison de 100 µl/puits et incubé 1 heure à 37°C. Sur chaque hormone évaluée, l'anticorps et le scFv ont été distribués à différentes concentrations selon une gamme de 10 à 250 µg/ml pour les anticorps et de 10 à 150 ou 200 µg/ml pour le scFv.

Après cinq lavages, un anticorps secondaire couplé à la peroxydase (HRP) a été distribué à raison de 100 µl/puits et incubé 1 heure à 37°C. Selon l'isotype de l'anticorps monoclonal étudié, l'anticorps secondaire a été un anti-IgG1 HRP (Réf. 115-035-205, Jackson ImmunoResearch Laboratories Inc), un anti-IgG2a HRP (Réf. 115-035-206, Jackson Laboratories) ou un anti-IgM HRP (Réf. 115-035-075, Jackson Laboratories). Pour les scFv, un anti-His Tag HRP (Réf. R93125 Life technologies, France) a été utilisé. Après cinq lavages, l'activité enzymatique a été révélée avec du TMB distribué à raison de 100 µl/puits. Le temps de révélation a été de 5 à 30 min à température ambiante selon la vitesse de la réaction. Après arrêt de la réaction avec H₂SO₄ 1M (50 µl/puits) l'intensité de la réaction colorée (Densité Optique) a été mesurée à l'aide d'un spectrophotomètre pour plaques ELISA.

### - Spécificité du scFv CF12

Le scFv CF12 a permis d'obtenir une liaison quantifiable par une méthode ELISA, basée sur la révélation de la liaison de l'anticorps préparé à des concentrations croissantes sur différentes hormones adsorbées (liaison jusqu'à saturation, Bmax). Les résultats sont exprimés en unités de densité optique obtenues après révélation.

Le tableau 9 représente les valeurs de densité optique obtenues avec le scFv CF12 incubé à la concentration de 200 µg/ml sur la FSH porcine (pFSH), ovine (oFSH) et sur différentes FSH humaines.

**Tableau 9**

| | oFSH | pFSH | hFSH (Gonal F) | hFSH (Puregon) | hFSH (Fostimon) | hMG (Menopur) |
|---|---|---|---|---|---|---|
| scFv CF12 | 2,3 | 2,5 | 0,5 | 0,9 | 0,5 | 0,8 |

Le scFv CF12 présente une forte liaison sur la pFSH et la oFSH adsorbées, et une liaison plus faible sur les hFSH et la hMG (Menopur).

Le tableau 10 représente les valeurs de densité optique obtenues avec le scFv CF12 incubé à la concentration de 200 µg/ml sur la LH porcine (pLH), ovine (oLH), bovine (bLH), la eCG et les hCG Chorulon et Endo 5000.

**Tableau 10**

| | oLH | pLH | bLH | eCG | Chorulon | Endo 5000 |
|---|---|---|---|---|---|---|
| scFv CF12 | 2 | 2,2 | 2,2 | 0,6 | 0,35 | 0,38 |

La liaison du scFv CF12 sur les LH animales est importante, à l'inverse des hCG et de la eCG adsorbées pour lesquelles la liaison est plus faible.

La liaison de CF12 et celle du scFv CF12 semblent donc extrêmement contraintes par la conformation de l'épitope particulièrement pour les hormones humaines. Etant donné les effets biologiques remarquables obtenus avec CF12 et son scFv, *in vitro* et *in vivo* sur l'activité des FSH humaines et des hCG Chorulon et Endo 5000 (voir résultats dans Exemple 2 et 3), il est probable que la liaison du scFv CF12 tout comme celle de l'anticorps entier soit totalement dépendante de la conformation de l'hormone. L'hypothèse d'une altération de la liaison de CF12 et du scFv CF12 due à une modification de la conformation des hormones adsorbées sur le plastique de la plaque ELISA peut expliquer ces résultats et renforce l'hypothèse que CF12 et son scFv sont spécifiques d'un épitope extrêmement conformationnel.

Une estimation de la constante de dissociation Kd du scFv, vis-à-vis des différentes FSH, LH et CG étudiées, a été calculée sur GraphPad Prism (GraphPad Software Inc., San Diego, CA, USA, version 5) en utilisant la fonction *"One site* - *Specific binding"* dans un modèle de liaison à saturation (*"saturation binding experiment model",* GraphPad PRISM software). Les différentes valeurs obtenues sont indiquées dans les tableaux 11 et 12.

**Tableau 11**

| | oFSH | pFSH | hFSH Gonal-F | hFSH Puregon | hFSH Fostimon | hMG Menopur |
|---|---|---|---|---|---|---|
| Kd (10⁻⁶M) | 3,779 | 2,628 | 4,874 | 25,22 | 2,634 | 14,01 |

**Tableau 12**

| | oLH | pLH | bLH | eCG | hCG Chorulon | hCG Endo 5000 |
|---|---|---|---|---|---|---|
| Kd (10⁻⁶M) | 5,813 | 4,729 | 5,033 | 6,116 | 5,074 | 6,238 |

La comparaison des constantes de dissociation Kd ainsi estimées indique une plus forte affinité du scFv pour les FSH ovine, porcine et humaines (Gonal-F et Fostimon) avec une valeur de Kd allant de 2,6 µM pour pFSH et hFSH Fostimon à 3,77 µM pour oFSH et 4,87 µM pour hFSH Gonal-F.

Les valeurs de Kd pour les LH animales, eCG et les hCG Chorulon et Endo 5000 sont relativement homogènes et varient entre 4,72 à 6,23 µM, témoignant d'une affinité un peu plus faible du scFv CF12 pour ces hormones comparativement aux FSH ci-dessus.

Vis-à-vis de la hFSH Puregon et de la hMG Menopur, le scFv CF12 présente une affinité encore plus faible avec un Kd de l'ordre de la dizaine de µM : 14 et 25,22 µM respectivement.

### EXEMPLE 2 : MESURE IN VITRO DE L'EFFET POTENTIALISANT DES LIGANDS DE L'INVENTION SUR LA BIOACTIVITE DE LA FSH

La mise en évidence de l'effet potentialisant des ligands de l'invention sur la bioactivité de la FSH a été réalisée en comparant la réponse biologique obtenue avec différentes types ou lignées cellulaires stimulées soit avec la FSH seule soit avec le complexe FSH / anticorps monoclonal (AcM).

Dans chacun des cas, la comparaison des courbes dose-réponse obtenues a permis de quantifier l'effet potentialisant *in vitro* de l'AcM sur l'activité biologique de la FSH complexée. L'analyse statistique des résultats a été faite par le logiciel Prism (GraphPad Software Inc., San Diego, CA, USA, version 5).

### 1/ Sur cultures primaires de cellules de granulosa bovines

L'effet potentialisant de l'AcM CF12 sur la FSH humaine (hFSH) a tout d'abord été caractérisé sur des cellules de granulosa bovines exprimant de façon endogène le récepteur FSH bovin.

Des surnageants d'hybridomes à la concentration finale de 0,1µg/ml d'anticorps CF12 ont été incubés avec une gamme de FSH humaine allant de 3 ng/ml à 25 ng/ml, 30 mn à 37°C.

Les cellules de granulosa bovines ont été ponctionnées sur des ovaires de vache à partir de follicules de diamètre allant de 2 à 6 mm, selon le protocole décrit par Chopineau et al. (Mol. Cell Endocrinol., 92(2) : 229-39, 1993) [8] et Wehbi et al. (Endocrinology, 151(6): 2788-2799, 2010) [9]. Les cellules de granulosa bovines en suspension dans un milieu McCoy's 5A (Lonza, Belgique, référence BE12-688F), préparées à raison de 80 000 cellules par 0,5 ml, ont été stimulées pendant 3 heures à 37°C, sous agitation, en présence d'IBMX 48 µg/ml (Sigma Aldrich, France, référence I5879), par une gamme de FSH allant de 3 ng/ml à 25 ng/ml, seule ou préalablement complexée à un anticorps monoclonal selon le protocole ci-dessus. La réponse biologique mesurée a été la sécrétion en AMPc.

Après centrifugation, l'AMPc produit a été dosé dans le surnageant de culture à l'aide d'un kit ELISA (Biomedical Technologies Inc., MA, USA, BT-730).

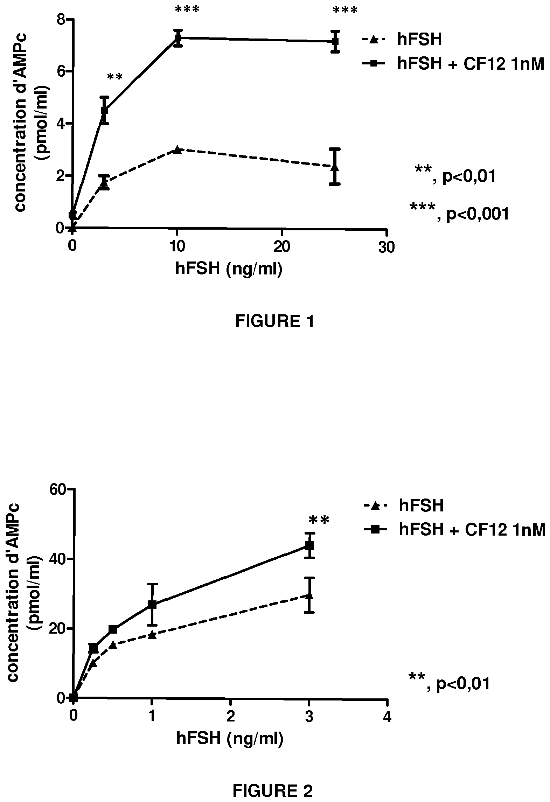

Les résultats sont présentés dans la figure 1.

Les résultats montrent une amplification de 2,5 fois pour CF12 sur l'activité de la FSH humaine. L'analyse statistique par analyse de variance à deux variables (*two-way* ANOVA, GraphPad PRISM software) montre un effet significatif allant de p<0,01 (**) à p<0,001 (***) pour CF12. L'anticorps CF12 a un effet significatif pour l'ensemble des concentrations de hFSH testées.

### 2/ Sur lignée cellulaire HEK293 transfectées de façon stable avec le récepteur FSH humain

L'effet potentialisant des AcM sur la FSH de différentes espèces a été mesuré sur des cellules HEK 293 exprimant de manière stable le récepteur FSH humain. Ce système a permis de mesurer la production d'AMPc suite à l'activation du récepteur de la FSH après une stimulation par la FSH seule ou par le complexe FSH / ACM pendant 1 heure à 37°C.

Pour cela, 60 000 cellules ont été réparties dans des puits de plaques 96 puits (Becton Dickinson, NJ, USA, référence 353072) et cultivées 24h à 37°C, 5% CO₂ en atmosphère humide, dans 100 µl de milieu MEM (Ozyme, France, référence BE12-611F) contenant du SVF 10% (Lonza, Belgique, référence DE14-801F), pénicilline/streptomycine 1% (Sigma Aldrich, France, référence P-4333) et G418 400 µg/ml (Sigma Aldrich, France, référence A1720). Après 2h de sevrage dans du milieu MEM, les cellules ont été stimulées pendant 1h à 37°C. Le surnageant de culture a été récupéré et dosé à l'aide d'un kit ELISA (Biomedical Technologies Inc., MA, USA, BT-730). Les résultats expriment la quantité d'AMPc sécrétée en point final. Ils ont été analysés grâce au logiciel Prism (GraphPad Software Inc., San Diego, CA, USA, version 5).

La figure 2 représente l'effet potentialisant de l'anticorps monoclonal CF12 sur la bioactivité de la FSH humaine *in vitro* sur des cellules HEK 293 transfectées de façon stable avec le récepteur FSH humain. Pour cela, les cellules ont été stimulées soit avec une gamme allant de 0,3 à 3 ng/ml pour la FSH humaine (Gonal-F, Laboratoire Serono), soit avec les mêmes points de gamme FSH préalablement incubés, 30 minutes à 37°C, avec l'anticorps monoclonal (concentration finale 0,1 µg/ml) avant la stimulation des cellules. Une analyse de variance à deux variables (*two-way* ANOVA, GraphPad PRISM software) a permis de comparer les courbes dose-réponse obtenues avec la FSH seule ou avec le complexe FSH/anticorps monoclonal. Les résultats obtenus avec la FSH humaine recombinante (Gonal-F, laboratoire Serono) montrent que l'anticorps CF12 a un effet potentialisant de l'activité hormonale de 140% à 0.5ng/ml et 160% pour les concentrations 1 et 3 ng/ml respectivement. Cet effet est significatif pour le point 3 ng/ml de FSH humaine (p<0,01) par un test-t apparié (test de Wilcoxon).

### 3/ Sur lignée cellulaire HEK293 transfectées de façon stable avec le récepteur FSH humain et avec le système Glosensor®

L'effet potentialisant des AcM sur les FSH de différentes espèces a été mesuré en temps réel sur des cellules HEK 293 exprimant de manière stable le récepteur FSH humain et le vecteur GloSensor™ (Promega, France). Ce système cellulaire a permis de suivre la production d'AMPc suite à la stimulation du récepteur FSH par l'agoniste (FSH seule ou complexe FSH / anticorps monoclonal) en temps réel. Suite à la liaison de l'AMPc sur la protéine GloSensor™, le substrat GloSensor™ (Promega, France, référence E1291) a été hydrolysé et conduit à une émission de luminescence mesuré grâce à un lecteur PolarStar Optima (BMG Labtech, Allemagne) et exprimée en RLU (Relative Luminescence Unit). Cette lignée stable a été développée par l'équipe de Biologie et BioInformatique des Systèmes de Signalisation au centre INRA Val de Loire, 37380 Nouzilly, France) et a été mise gracieusement à disposition pour ces essais.

Pour cela, les cellules HEK 293 ont été mises en culture à raison de 80 000 cellules par puits de microplaque 96 puits blanc à fond transparent (Dominique Dutscher, France, référence 655903) et cultivées dans 100 µl de milieu MEM (Ozyme, France, référence BE12-611F) supplémenté de SVF 10% (Lonza, Belgique, référence DE14-801F), pénicilline / streptomycine 1% (Sigma Aldrich, France, référence P-4333), Hygromycine B 200 µg/ml (Life Technologies™, France, référence 10687010) et G418 400 µg/ml (Sigma Aldrich, France, référence A1720) pendant une nuit. Après 2h de sevrage dans 100 µl de milieu MEM supplémenté de BSA 1% (PAA, France, référence K45012) et contenant 4% de substrat GloSensor™ pendant 2h à température ambiante à l'abri de la lumière, la plaque de cellules a été mise dans le lecteur PolarStar Optima et une première lecture a été faite pendant 5 minutes pour mesurer le niveau basal de luminescence. La plaque a ensuite été retirée du lecteur et 11 µl de ligand (FSH seule ou complexe FSH / anticorps monoclonal) y ont été ajoutés de manière à obtenir les concentrations indiquées. La luminescence émise a ensuite été mesurée pendant environ 1h30.

Les résultats obtenus ont été analysés grâce au logiciel Prism (GraphPad Prism Software Inc., San Diego, CA, USA, version 5). La fonction non linéaire « log (agoniste) versus response » a été utilisée pour tracer la réponse en fonction de la concentration de FSH. Ceci a permis de caractériser et comparer l'EC50 pour la FSH seule et la FSH complexée à l'anticorps monoclonal. Pour chaque exemple, l'effet significatif du complexe FSH / anticorps potentialisant a été mesuré par analyse de variance à deux variables (*two-way* ANOVA, GraphPad PRISM software) en comparant les deux courbes dans leur totalité.

### - Anticorps monoclonal CF12

L'effet potentialisant de l'anticorps monoclonal CF12 a été caractérisé sur la bioactivité de la FSH humaine, ovine et porcine.

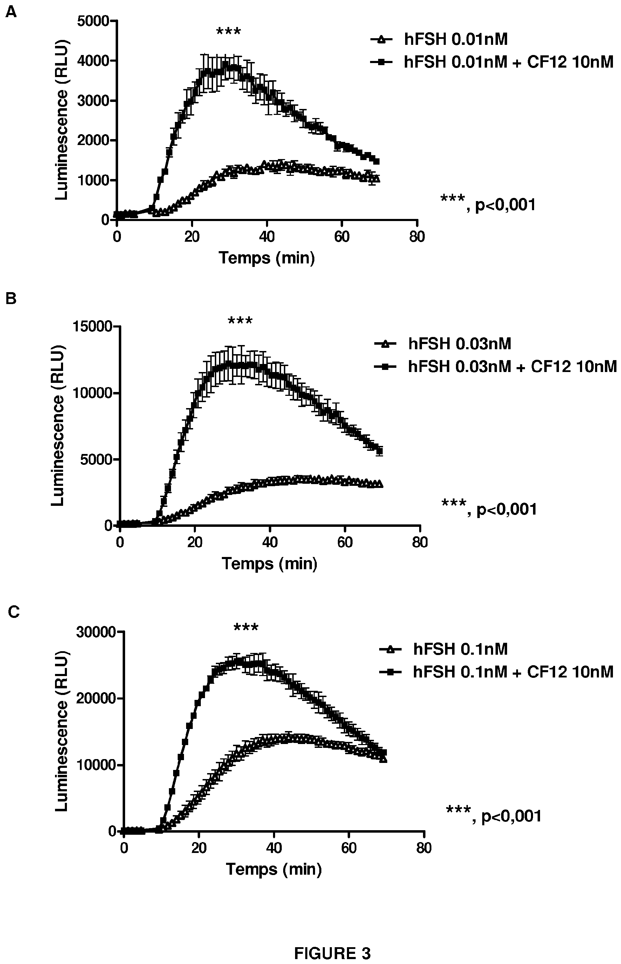

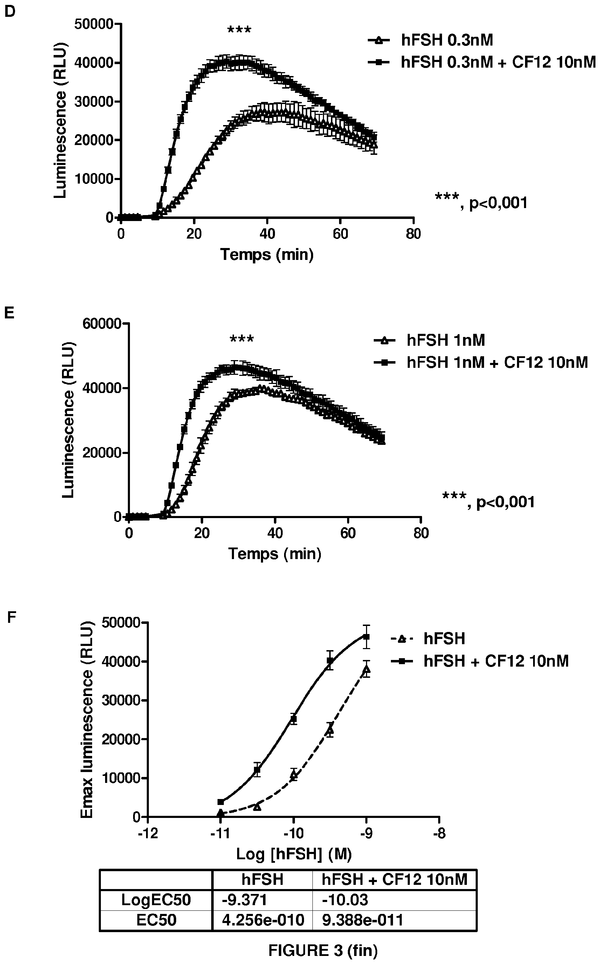

La Figure 3 illustre l'effet potentialisant remarquable de CF12 sur la bioactivité de la FSH humaine (Gonal-F, Laboratoire SERONO). Cet effet remarquable est parfaitement quantifiable aux concentrations faibles de 0,01 nM et 0,03 nM de hFSH pour lesquelles le système cellulaire n'est pas à saturation (courbes A et B). On observe ainsi une augmentation du signal de luminescence de 280% et 341% respectivement, hautement significatives (p<0,001). Pour les concentrations plus élevées (0,1 - 0,3 et 1 nM), l'augmentation de la réponse cellulaire est de 181%, 147% et 120% respectivement, en raison probablement d'une saturation progressive du signal luminescent jusqu'à 46000 RLU (courbes C, D et E). Pour les courbes C, D et E l'augmentation reste très significative (p<0,001). La valeur des EC50 mesurée par GraphPad Prism est de 4,25.10⁻¹⁰M pour la hFSH et de 9,38.10⁻¹¹M pour le complexe hFSH / CF12 traduisant une augmentation de la bioactivité de l'hormone de 0,7 unité de LogEC50 (de 10^{-9,37} à 10^{-10,03} respectivement) lorsqu'elle est complexée à l'anticorps potentialisant CF12 (courbe F).

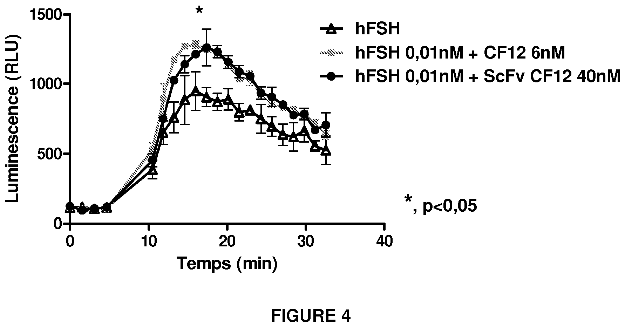

L'effet potentialisant du scFv CF12 (40 nM) a également été mesuré sur l'activité de la FSH humaine (Gonal F, Laboratoire Serono) préparée à la concentration de 0,01 nM (Figure 4). L'effet de l'anticorps entier CF12 (6 nM) a été mesuré parallèlement pour comparaison. Les courbes obtenues avec le complexe hFSH / scFv CF12 ou hFSH / anticorps CF12 se superposent parfaitement, indiquant un effet identique du fragment d'anticorps monovalent.

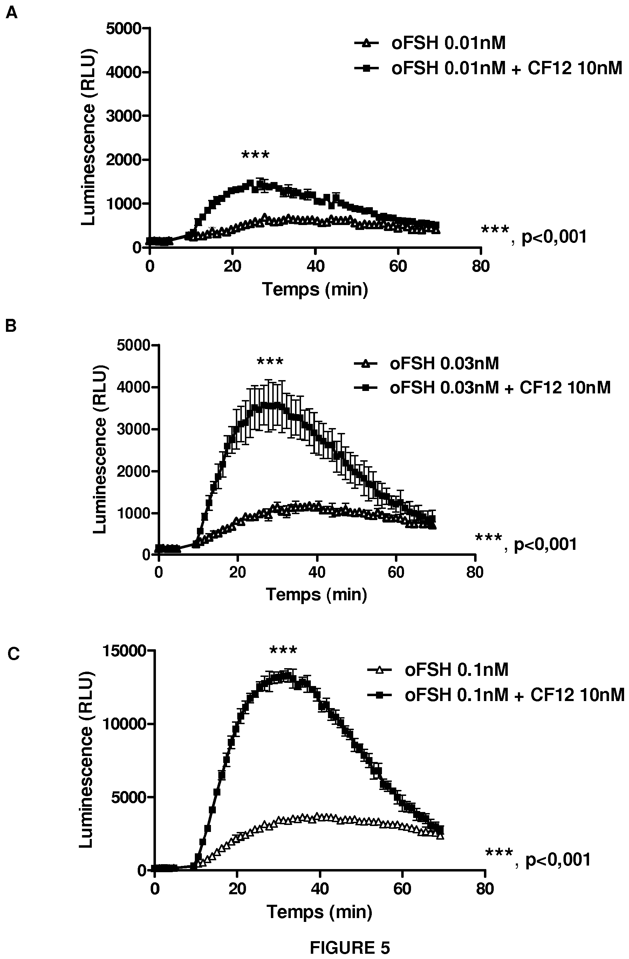

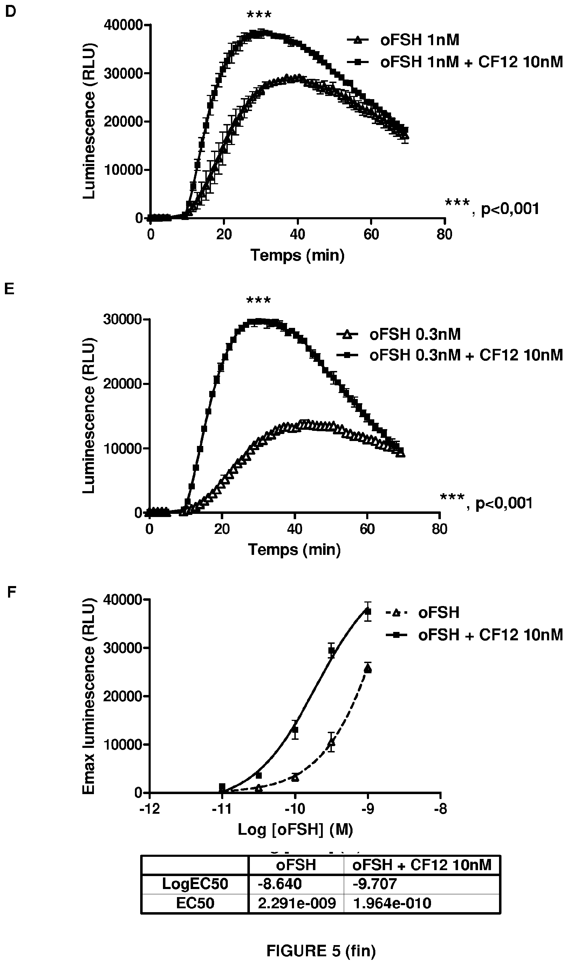

L'effet potentialisant exercé par CF12 est également très significatif sur la FSH ovine comme l'illustre la figure 5 où l'on observe une augmentation de la réponse cellulaire de 240%, 300% et 350% au cours d'une stimulation avec le complexe CF12 / oFSH pour les concentrations 0,01 nM - 0,03 nM et 0,1 nM d'hormone (courbes A, B, C). CF12 a été préparé à 10 nM. De même que pour la hFSH, pour les concentrations plus élevées 0,3 nM et 1nM (courbes D et E), l'augmentation de la réponse cellulaire est de 200% et 130% respectivement due à une saturation progressive du signal luminescent jusqu'à 40000 RLU. La valeur des EC50 mesurée par GraphPad Prism est de 2,29.10⁻⁹M pour la oFSH et de 1,96.10⁻¹⁰M pour le complexe oFSH / CF12 traduisant une augmentation de la bioactivité de l'hormone de 1,06 LogEC50 (de 8,64 à 9,7 respectivement) lorsqu'elle est complexée à l'anticorps potentialisant CF12 (courbe F). Les effets potentialisants observés sur la réponse cellulaire sont dans tous les cas hautement significatifs (p<0,001).

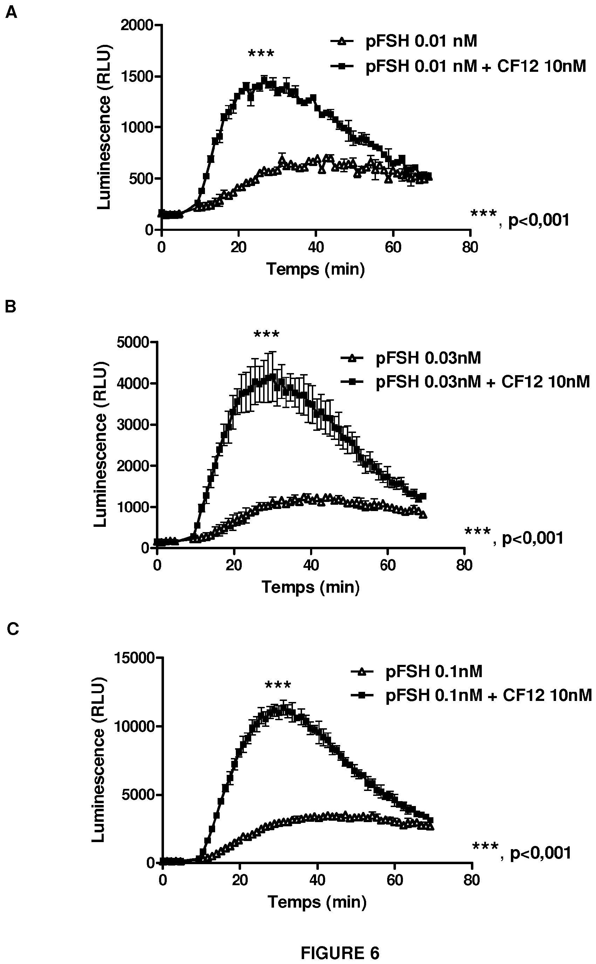

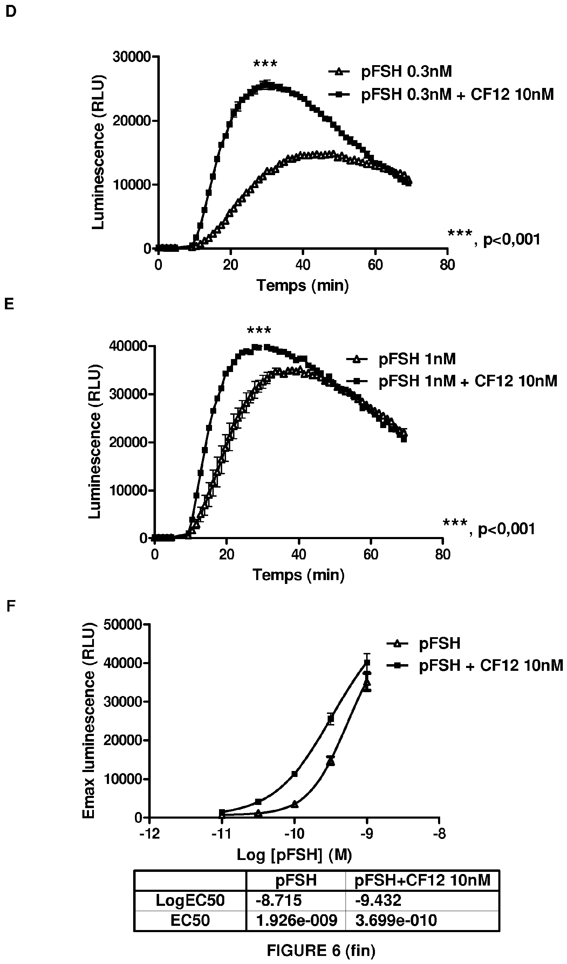

Les courbes de la Figure 6 illustrent l'effet potentialisant de CF12 (10 nM) sur la FSH porcine préparée aux concentrations 0,01 - 0,03 - 0,1 - 0,3 et 1 nM. Cet effet est parfaitement quantifiable aux concentrations les plus faibles 0,01 nM - 0,03 nM et 0,1 nM de pFSH pour lesquelles le système cellulaire n'est pas à saturation (courbes A, B, C). On observe ainsi une augmentation très significative et importante du signal de luminescence de 220%, 350% et 330% respectivement. Pour les concentrations plus élevées (0,3 et 1 nM), l'augmentation de la réponse cellulaire est moindre, respectivement de 175% et 114%, due à une saturation progressive du signal luminescent jusqu'à la limite de 40000 RLU (courbes D et E). La valeur des EC50 mesurée par GraphPad Prism est de 1,92.10⁻⁹M pour la pFSH et de 3,69.10⁻¹⁰M pour le complexe pFSH / CF12 traduisant une augmentation de la bioactivité de l'hormone de 0,717 LogEC50 (de 10^{-8,715} à 10^{-9,432} respectivement) lorsqu'elle est complexée à l'anticorps potentialisant CF12 (courbe F).

### 4/ Sur cultures primaires de cellules de granulosa humaines

Les cellules de granulosa humaines ont été récupérées et cultivées comme décrit dans Reverchon et collaborateurs (Reverchon et al., Human Reprod., 27(6) : 1790-1800, 2012) [11].

Ces cellules ont été récupérées à partir de liquides folliculaires récoltés à l'issue de ponction ovocytaires chez des femmes traitées pour une Fécondation In Vitro (FIV) dans le cadre d'une procréation médicalement assistée (PMA). Ces cellules ont été isolées par centrifugation sur un gradient de Percoll 40%, resuspendues dans un milieu McCoy's 5A complet (Lonza, Belgique, référence BE12-688F) puis ensemencées dans des plaques 24 puits (Becton Dickinson, NJ, USA, référence 353047) à raison de 30 000 cellules par puits dans un volume final de 500 µl et cultivées pendant 48 heures. Elles ont ensuite été stimulées soit avec de la FSH humaine seule soit avec le complexe FSH humaine / AcM pendant 48 heures. Les FSH humaines utilisées ont été principalement la Gonal-F, hormone recombinante commercialisée par le laboratoire pharmaceutique SERONO, (SERONO, Europe, Limited) et la Fostimon, FSH extraite à partir d'urine de femmes ménopausées, commercialisée par le laboratoire pharmaceutique GENEVRIER (France). Après la stimulation, les surnageants ont été récupérés et centrifugés. L'AMPc produit a été dosé dans chaque surnageant de culture à l'aide d'un kit ELISA (Biomedical Technologies Inc., MA, USA, BT-730).

Les cellules issues de 23 patientes ont été préparées séparément et mises en culture séparément selon la méthode décrite ci-dessus. Chaque culture cellulaire issue d'une patiente a été divisée en deux lots : l'un a été stimulé par une gamme de FSH allant de 10⁻¹¹M à 10⁻⁸M, l'autre a été stimulé par le complexe anticorps monoclonal CF12 / hFSH aux différentes concentrations de la gamme. L'anticorps a été préparé à la concentration finale de 0,1 nM ou à 4 nM. Pour chaque patiente, les courbes doses-réponses obtenues dans les deux conditions ont été comparées afin d'évaluer l'effet potentialisant de l'anticorps sur la bioactivité de l'hormone humaine utilisée.

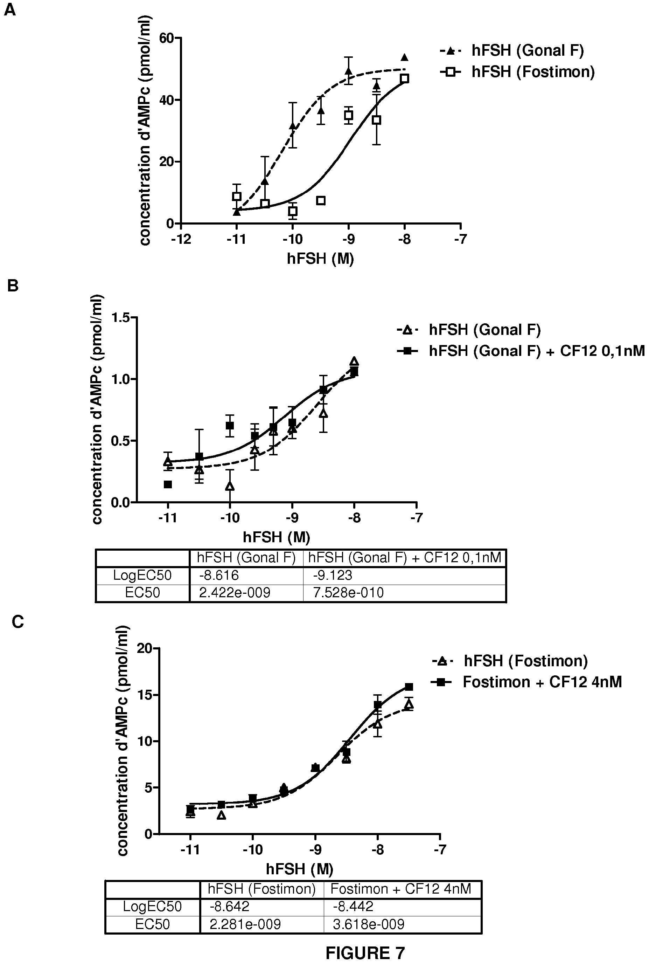

Les cultures cellulaires issues des 23 patientes ont montré des réponses à la stimulation par hFSH et/ou par le complexe hFSH / anticorps très différentes. Seules les cellules de 12 patientes sur les 23 totales ont répondu à une stimulation par la FSH seule, quel que soit la hFSH utilisée, et à une stimulation par le complexe FSH / anticorps (Figure 7, courbes A, B, C). Ces résultats sont illustrés sur la figure 7. La courbe A représente la réponse cellulaire d'une patiente à une stimulation par la Gonal F et par la Fostimon ; les EC50 mesurés ont été de 7.10⁻¹¹ et 1.10⁻⁹ respectivement. Les courbes B et C illustrent deux cas représentatifs de patientes dont les cellules de granulosa ont répondu à la fois à une stimulation par la hFSH seule (Gonal-F pour la courbe B et Fostimon pour la courbe C) et par le complexe CF12 / hFSH. Dans le cas de la courbe B, l'EC50 de la courbe dose-réponse obtenue avec le complexe est supérieur à celui obtenu avec l'hormone seule (7,52.10⁻¹⁰ M versus 2,42.10⁻⁹ M). Dans le cas de la courbe C, les EC50 ne sont pas différents (2,28.10⁻⁹ M versus 3,61.10⁻⁹ M).

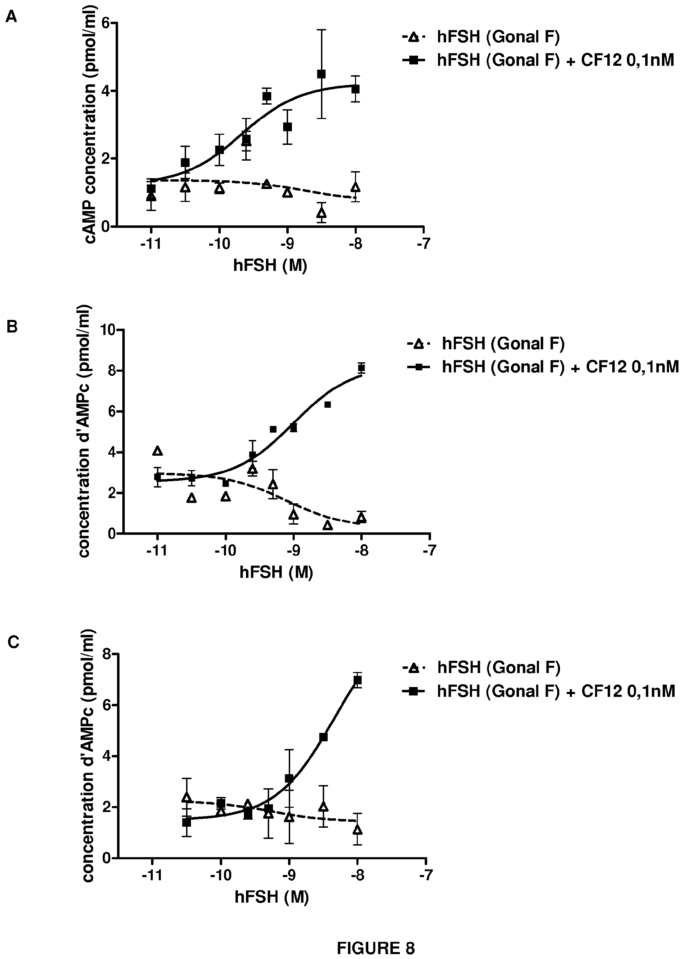

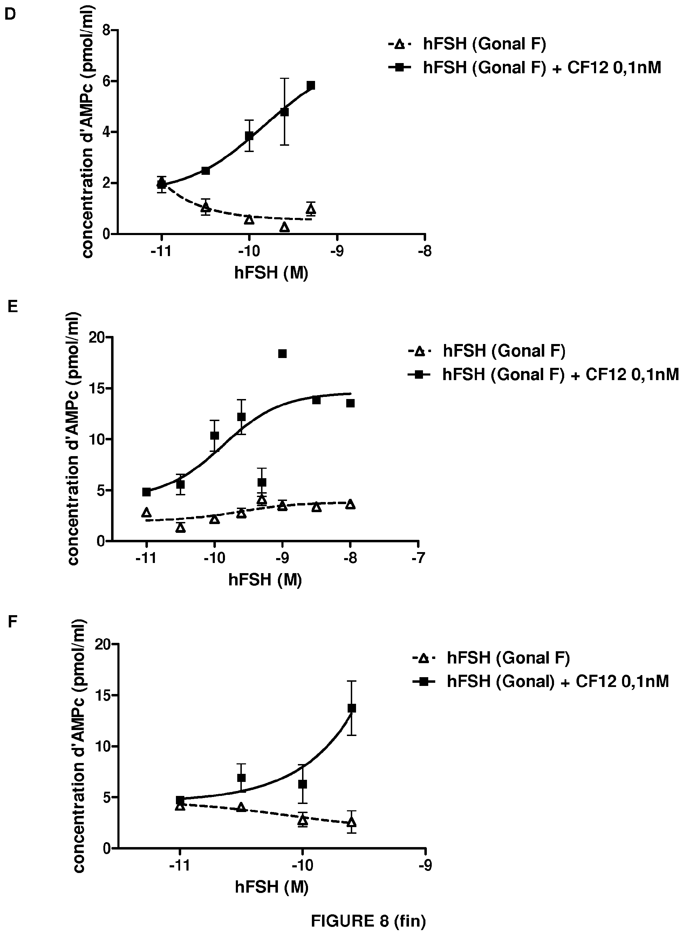

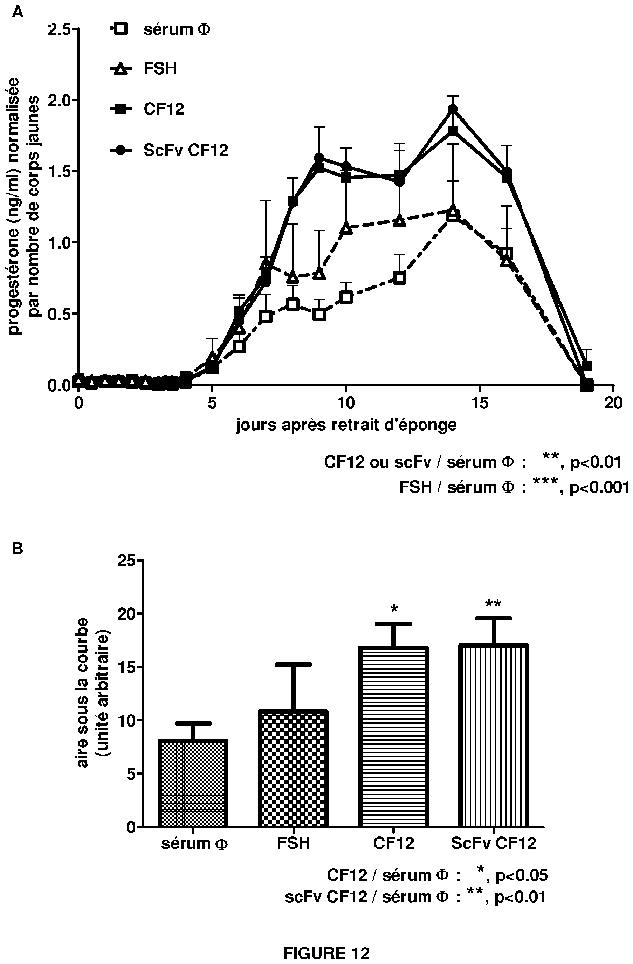

Sur les 11 patientes restantes, les cellules issues de quatre d'entre elles n'ont répondu ni à une stimulation par la FSH ni à une stimulation par le complexe FSH / CF12. A l'inverse, et de façon étonnante, les cellules issues des 7 autres patientes ont répondu uniquement à une stimulation par le complexe FSH / anticorps potentialisant alors qu'aucune augmentation de la sécrétion d'AMPc n'a été observée après stimulation avec la hFSH seule dans la même gamme de concentrations. Ces résultats remarquables sont illustrés par la Figure 8, courbes A à F, chaque courbe représentant la réponse des cellules de granulosa d'une patiente différente. Dans chacun des cas, l'anticorps CF12 a été utilisé à 0,1 nM. Deux patientes ont donné une même courbe dose-réponse illustrée par la courbe D. Ces résultats démontrent très clairement que chez ces patientes, seul le complexe hFSH / anticorps CF12 est capable d'induire une stimulation fonctionnelle du récepteur hFSHR à l'inverse de la FSH seule qui n'entraîne aucune activation du récepteur hFSHR. Le taux de sécrétion maximale en AMPc obtenu sous stimulation par le complexe hFSH / anticorps CF12 se situe entre 6 et 15 pmol/ml équivalent au taux de sécrétion maximale obtenu avec des cultures cellulaires ayant normalement répondu à la hFSH (Figure 12). Les EC50 mesurés par GraphPad Prism pour chacune des courbes dose-réponse sont illustrés dans le Tableau 13 :

**Tableau 13**

| patiente | A | B | C | D* | E | F |
|---|---|---|---|---|---|---|
| EC50 (M) | 2,15 10⁻¹⁰ | 1,11 10⁻⁹ | 4,27 10⁻¹⁰ | 1,71 10⁻¹⁰ | 8,91 10⁻¹¹ | ND |

| | | | | | | |
|---|---|---|---|---|---|---|
| * : l'EC50 obtenu pour la patiente G a été identique à celui de la patiente D. | | | | | | |

Le complexe hFSH / anticorps CF12 se comporte donc comme un nouveau ligand, comme un nouvel agoniste capable d'activer le hFSHR chez des patientes naturellement réfractaires à une stimulation classique par hFSH recombinante ou extraite. L'utilisation d'un mélange hFSH / anticorps potentialisant peut ainsi apporter une nouvelle alternative dans les traitements hormonaux d'induction de l'ovulation (mono ou poly ovulation) chez des patientes ne répondant pas aux traitements hormonaux classiques utilisés en biologie de la reproduction humaine.

### EXEMPLE 3 : MESURE IN VIVO DE L'EFFET POTENTIALISANT DES LIGANDS DE L'INVENTION SUR LA BIOACTIVITE DES FSH et LH/CG DANS LE MODELE RAT

Après avoir été caractérisé *in vitro,* l'effet potentialisant de l'anticorps monoclonal a été caractérisé *in vivo,* chez le rat femelle pour son effet sur la bioactivité de la FSH et chez le rat mâle pour leur effet sur la bioactivité de la LH/CG, qu'ils reconnaissent également.

Pour mesurer la bioactivité FSH, le protocole utilisé a été celui du dosage biologique décrit par Steelman et Pohley (Steelman SL, Pohley FM. Endocrinology, 53:604-616. 1953) [12]. Pour mesurer la bioactivité LH, le protocole utilisé a été celui du dosage décrit par Scobey et collaborateurs (Scobey et al, Reprod. Biol. Endocr. 3 :61, 2005) [13].

L'effet des anticorps sur l'activité FSH a été évalué en utilisant des FSH humaines. L'effet des anticorps sur l'activité LH a été évalué sur deux préparations de hCG (human Chorionic Gonadotropin).

L'analyse statistique a été réalisée avec le logiciel GraphPad Prism (GraphPad Software Inc., San Diego, CA, USA, version 5). Les résultats portant sur des expériences réalisées sur des lots de 5 animaux, une analyse de variance à une variable, non paramétrique (test de Kruskal Wallis), suivie d'une correction de Dunns, a été appliquée ou un test-t non-paramétrique (test de Mann-Whitney). Pour les résultats portant sur des effectifs plus importants (n>30) issus de la compilation de plusieurs bioassays, un test paramétrique (test t de Student non apparié) suivi d'une correction de Bonferroni a été appliqué.

### 1/ Effet potentialisant des anticorps sur la bioactivité de la FSH chez la Ratte

L'effet potentialisant de l'anticorps CF12 et de son scFv a été étudié sur différentes préparations de FSH humaine utilisées en reproduction humaine dans le cadre des traitements de procréation médicalement assistée : la Gonal-F et la Puregon (FSH recombinantes des laboratoires Merck Serono et Merck Schering-Plough respectivement), la Fostimon et la Menopur (FSH extraites commercialisées par les laboratoires Genevrier et Merck Schering-Plough respectivement).

Comme décrit dans le protocole de Steelman et Pohley, des rattes immatures de 21 jours ont reçu pendant trois jours consécutifs 2 injections matin et soir, de 100 µl d'un mélange de hCG et FSH comportant une quantité constante de hCG (3,5 UI) additionnée d'une quantité variable de FSH allant de 0,5 à 1,5 UI pour la FSH humaine (Gonal F, Puregon, Fostimon, Menopur). Les injections ont été réalisées par voie sous-cutanée au niveau de la nuque. Chaque expérience comprenait au minimum 4 lots : un lot traité avec du sérum physiologique (sérum Φ), un lot traité avec l'anticorps ou le scFv seul, un lot traité avec le mélange hCG+FSH, un lot traité avec le mélange hCG/FSH additionné de 2 µg d'anticorps ou de scFv purifié.

Dans le cas d'un traitement avec le complexe hormone/anticorps ou scFv, avant l'injection, le mélange FSH + anticorps a été préalablement incubé 20 min à 37°C ou à température ambiante indifféremment, puis ajouté à la hCG. La hCG peut indifféremment être mélangée à la FSH pendant l'incubation du complexe.

Le quatrième jour, les rattes ont été pesées, et leurs ovaires ont été prélevés, disséqués puis pesés. Les résultats sont exprimés en milligramme d'ovaire/100 grammes de poids corporel. L'augmentation du poids des ovaires est proportionnelle à la quantité de FSH bioactive injectée. Ceci permet de quantifier et de comparer la bioactivité d'une même quantité d'hormone injectée seule ou en complexe avec un anticorps.

La comparaison de la bioactivité de la FSH injectée seule ou complexée à l'anticorps ou au scFv permet de mesurer le différentiel de la réponse et de quantifier ainsi l'effet potentialisant de l'anticorps ou de son scFv.

### Effet potentialisant de l'anticorps CF12 et de son scFv.

L'effet *in vivo* de l'anticorps CF12, produit contre la FSH humaine, a été évalué sur la bioactivité de différentes préparations de FSH humaine et sur la bioactivité de la FSH ovine.

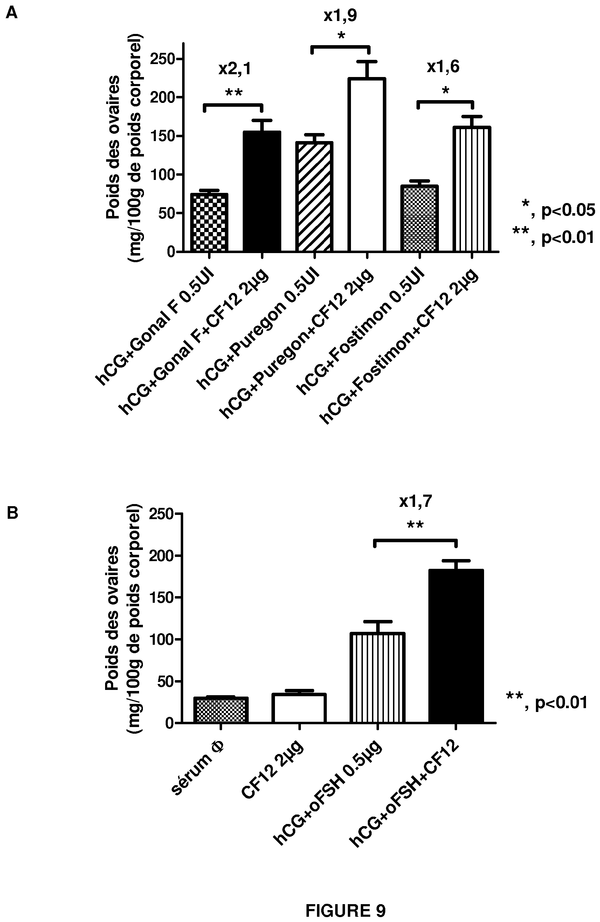

La figure 9A illustre l'effet potentialisant remarquable exercé par l'anticorps CF12 sur trois préparations différentes de FSH humaine. Les résultats sont ceux de bioassays représentatifs comportant des lots de 5 femelles. L'analyse statistique entre lots a été faite par un test-t non-paramétrique (test de Mann-Whitney). Un effet potentialisant important et significatif a été enregistré sur l'activité de la hFSH Gonal-F avec une augmentation de 210% du poids moyen des ovaires (74 versus 155 mg/100g de poids corporel, p<0.001). De même, sur la Puregon une augmentation de 190% a été obtenue (141 versus 224 mg, p<0.05) ainsi que sur la Fostimon avec laquelle une augmentation de 160% du poids moyen des ovaires a été enregistré (85 versus 161 mg, p<0,05).

Ces expériences ont été répétées entre 5 et 7 fois et les résultats ont été compilés (n=40 et 47 rattes pour les lots hCG+hFSH Gonal F et hCG+hFSH Gonal F+CF12). Un test paramétrique (test t de Student non apparié) suivi d'une correction de Bonferroni ont été appliqués. Comme illustré dans le tableau 14, elle fait apparaître une augmentation de 170% hautement significative entre le poids moyen des ovaires chez les femelles traitées classiquement par le mélange hCG+hFSH (Gonal F) et celui mesuré chez les femelles traitées avec le complexe hFSH Gonal F/CF12 : le poids moyen augmentant de 79,51 ± 2,178 mg d'ovaire/100gr de poids corporel à 134,8 ± 4,985 mg d'ovaire/100gr de poids corporel chez les femelles (***, p<0001).

**Tableau 14**

| Iot | Moyenne ± sem | effectif | statistiques |
|---|---|---|---|
| Sérum physiologique | 29,68 ± 5,88 | 10 | NS |
| CF12 2µg | 34,3 ± 7,9 | 12 | |
| hCG+hFSH | 79,51 ± 2,178 | 40 | *** |
| hCG+hFSH+CA5 | 134,8 ± 4,985 | 47 | p<0.0001 |

Une même approche a été conduite pour évaluer et quantifier l'effet potentialisant de l'anticorps CF12 sur la FSH d'origine ovine.

La figure 9B illustre un exemple représentatif de bioassay obtenu (lots de 5 femelles) en traitant les rattes avec 0,5 µg de FSH ovine + hCG ou avec hCG+ 0,5 µg de FSH ovine pré complexée avec CF12. Une augmentation de 170% du poids moyen des ovaires a été obtenu chez les femelles traitées avec le complexe oFSH/CF12 + hCG par rapport à celles ayant reçu un traitement sans CF12 : le poids moyen des ovaires passant de 107 mg à 183 mg/100gr de poids corporel (**, p<0.01).

La répétition de cette étude sur un effectif plus important (n=10) a confirmé de façon très significative l'effet potentialisant de CF12 sur la bioactivité de la oFSH (Tableau 15), le poids moyen augmentant de 115,5 ± 7,45 mg dans le cas du traitement classique à 166,4 ± 9,54 mg chez les traitées avec le complexe oFSH/CF12+ hCG (***, p<0.001).

**Tableau 15**

| Iot | Moyenne ± sem | effectif | statistiques |
|---|---|---|---|
| hCG+oFSH 0,5µg | 115,5 ± 7,45 | 10 | *** |
| hCG+oFSH+CF12 | 166,4 ± 9,54 | 10 | p<0.001 |

Le scFv CF12 développé à partir de la séquence des régions variables V_{H} et V_{L} de l'anticorps a été évalué de la même façon sur la bioactivité de la FSH humaine. Préalablement, plusieurs doses de scFv ont été évaluées de 0,06 µg par injection (correspondant à une quantité équimolaire avec l'anticorps entier de 2,5.10⁻⁹ mole) à 2 µg par injection correspondant à la même quantité injectée que l'anticorps entier. La comparaison de différentes doses de scFv dans le bioassay a mis en évidence qu'un effet potentialisant optimal est obtenu en injectant 2 µg de scFv par injection, soit 8.10⁻⁸ mole.

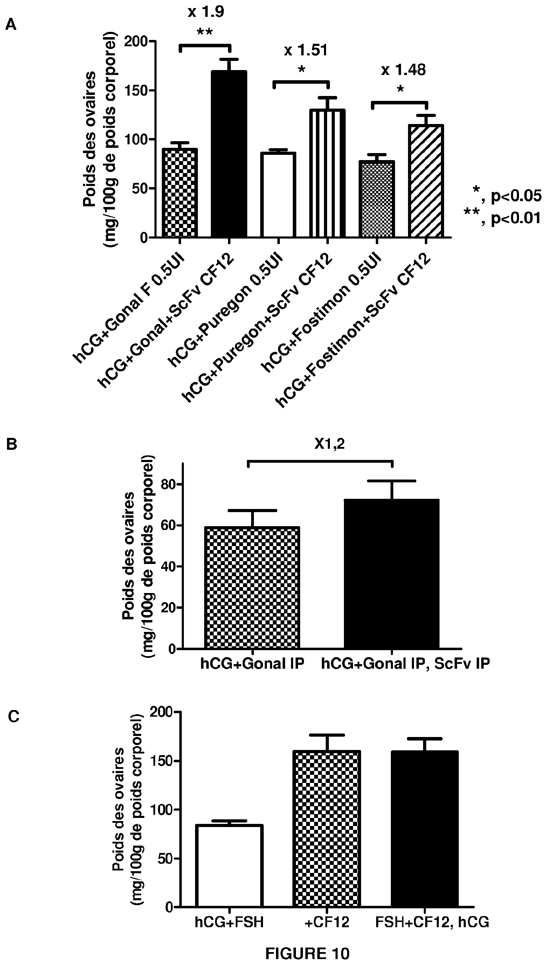

Les résultats obtenus sur les différentes préparations de FSH humaines sont illustrés sur la Figure 10A. Ils montrent que les effets enregistrés avec le scFv sont très proches de ceux mesurés avec l'anticorps entier sur les trois préparations de FSH humaines. Le poids moyen des ovaires a été systématiquement plus élevé chez les rattes traitées avec le complexe hFSH/scFv/ CF12 + hCG comparativement à celles traitées classiquement avec hFSH+hCG. Ainsi une augmentation de 190% a été enregistrée sur la hFSH Gonal F (avec un poids moyen de 170 mg dans le lot +scFv CF12 versus 89 mg dans le lot sans scFv CF12, p<0,01), une augmentation de 151% sur la hFSH Puregon (poids moyen de 130 mg dans le lot +scFv CF12 versus 86 mg dans le lot sans scFv, p<0,05) et une augmentation de 148% sur la hFSH Fostimon (poids moyen de 114 mg dans le lot +scFv CF12 versus 77 mg dans le lot sans scFv, p<0,05). L'analyse a été faite par un test-t non paramétrique (test de Mann-Whitney). Il est à noter que l'amplitude de l'augmentation de la réponse ovarienne est équivalente à celle obtenue avec l'anticorps CF12 entier (même facteur multiplicatif). Ce résultat significatif et majeur indique qu'un même effet potentialisant des gonadotropines circulantes peut être obtenu *in vivo* que ce soit par l'intermédiaire d'un scFv ou d'un anticorps entier, aboutissant à l'amplification d'une réponse physiologique tangible au niveau d'un organe.

Différents modes d'injection des mélanges hormone / anticorps ou scFv ont été évalués et comparés avec le protocole classique (injection par voie sous-cutanée) également dans le cas de CF12. Ainsi un bioassay a visé à comparer une injection du mélange hormonal par voie intra-péritonéale avec une injection du mélange hormonal par voie intra-péritonéale suivie d'une deuxième injection différée du scFv CF12 15 minutes plus tard. Les résultats sont présentés sur la Figure 10B, et indiquent une augmentation du poids des ovaires de 122% chez les femelles injectées par voie intra-péritonéale en deux temps: 1) avec le mélange hCG+hFSH puis 2) avec du scFv en injection différée 15 mn plus tard : 58,8 mg d'ovaire par 100 gr de poids corporel dans le lot hCG+hFSH Gonal F versus 72 mg d'ovaire par 100 gr de poids corporel dans le lot hCG+hFSH Gonal F suivi du scFv. La différence n'est pas significative entre les deux lots due au petit effectif mais les résultats indiquent une tendance à une potentialisation de la FSH en injectant l'hormone et le scFv en deux temps et à deux points d'injection différents. Il est donc envisageable pour des applications ultérieures d'injecter l'hormone à potentialiser puis l'anticorps ou le scFv indépendamment en temps et en points d'injection différents.

Une autre modalité d'injection a été évaluée chez les animaux traités avec le complexe hormone/anticorps : l'un ayant une seule injection sous-cutanée de hCG+FSH+CF12 et l'autre ayant une première injection sous-cutanée de FSH+CF12 puis 15 minutes plus tard de hCG en sous-cutanée également. Les résultats illustrés sur la figure 19C montrent que l'effet potentialisant observé dans les deux cas n'est pas différent : 160 mg versus 159 mg d'ovaires/100 gr de poids corporel chez les femelles traitées avec l'anticorps CF12 pour un poids moyen de 83 mg d'ovaires pour le lot traité sans anticorps.

### Effet potentialisant des anticorps sur la bioactivité de la LH/CG chez le Rat

En raison du coût très élevé de la LH ovine, ces dosages biologiques ont été réalisés avec de la hCG, facilement disponible, sous une forme très pure et peu onéreuse. L'effet des anticorps a été étudié sur deux préparations de hCG (human Chorionic Gonadotropin) humaines extraites, l'une utilisée en reproduction humaine dans le cadre des traitements de procréation médicalement assistée : l'ENDO 5000 (laboratoire Schering-Plough) et l'autre utilisée en médecine vétérinaire : la Chorulon (laboratoire MSD).

Selon le protocole de Scobey et collaborateurs [13], la bioactivité de la LH ou de la hCG a été quantifiée par rapport à l'augmentation du poids des vésicules séminales dont le développement est androgéno-dépendant. Le poids varie proportionnellement à l'activité de la hCG et permet donc de quantifier et comparer l'activité biologique de l'hormone injectée seule ou complexée avec l'anticorps étudié. Le protocole a été réalisé avec des ratons de 25 jours qui ont été injectés par voie sous-cutanée, une fois par jour pendant quatre jours avec 100 µl de 1,5 UI de hCG ou d'un mélange 1,5 UI hCG + 2 µg d'anticorps préalablement incubé 20 mn à 37°C. Le cinquième jour, les rats ont été pesés puis sacrifiés. Leurs vésicules séminales (VS) ont été prélevées, disséquées et pesées. Le poids des vésicules séminales est exprimé en mg/100g de poids corporel afin de pouvoir comparer et réunir les résultats obtenus avec différents lots. Dans chaque expérience, chacune des conditions a été testée sur un lot de 5 rats. Une même expérience a été répétée plusieurs fois.

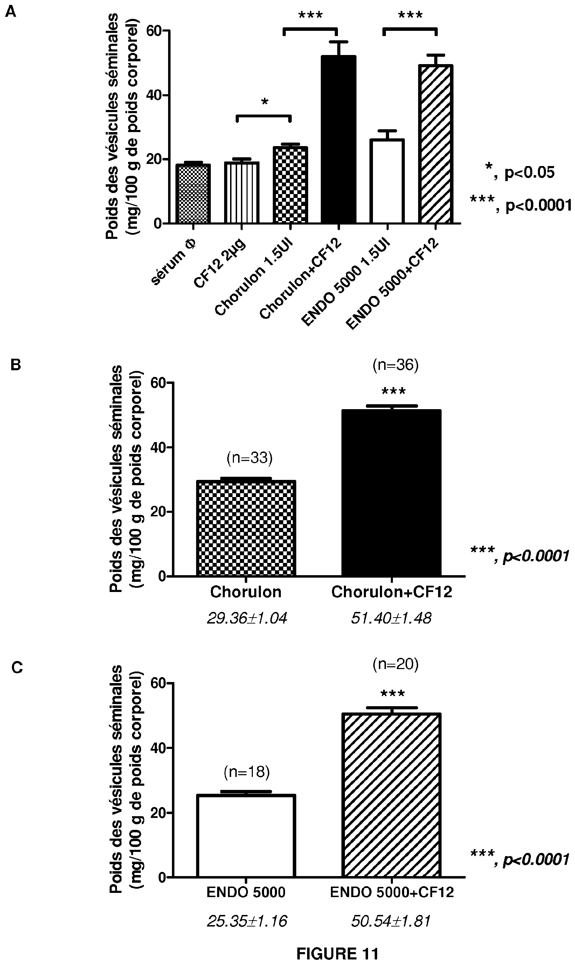

Les figures 11A, B, C illustrent les résultats obtenus chez les rats traités avec l'anticorps CF12 en complexe avec la hCG Chorulon et la hCG ENDO 5000. La figure 11A montre le résultat représentatif d'un bioassay réalisé sur 6 lots de 5 rats. Un effet potentialisant très significatif (p<0,0001, test de Krustal et Wallis) a été obtenu avec le complexe hCG Chorulon / CF12 avec une augmentation de 220% du poids des vésicules séminales par rapport au lot traité avec hCG seule. Un effet significatif a également été obtenu sur le lot traité avec le complexe hCG ENDO 5000 / CA5 avec une augmentation du poids de 189% (p<0,0001). On observe que le Iot traité avec l'anticorps CF12 seul ne présente aucun changement du poids des vésicules séminales par rapport aux animaux contrôles traités avec du sérum physiologique. CF12 non complexé à l'hormone n'a donc aucun effet propre sur l'organe cible.

La compilation des résultats issus des différents bioassays réalisés avec CF12 est représentée sur les histogrammes B et C de la figure 11. Un effet potentialisant hautement significatif (p<0,0001, Test-t non apparié) du complexe hormone / CF12 a été mesuré avec hCG Chorulon et hCG ENDO 5000:
- sur un effectif de 33 et 36 animaux respectivement, le poids moyen des VS a été de 29,36 mg/100 g chez les rats traités avec hCG Chorulon contre 51,40 mg/100 g chez les rats traités avec le complexe (augmentation de 175%) (Figure 11B)
- sur un effectif de 18 et 20 animaux respectivement, le poids moyen des VS a été de 25,35 mg/100 g chez les rats traités avec hCG ENDO 5000 et 50,54 mg/100 g chez les rats traités avec le complexe (augmentation de 208%) (Figure 11C).

### EXEMPLE 4 : MESURE IN VIVO DE L'EFFET POTENTIALISANT DES LIGANDS DE L'INVENTION SUR LA BIOACTIVITE DES GONADOTROPINES ENDOGENES CHEZ LA BREBIS

Après avoir démontré et caractérisé l'effet potentialisant *in vivo,* de l'anticorps monoclonal CF12, chez un rongeur (animal de petite taille), l'objectif a été d'étudier l'effet de chaque anticorps sur l'activité de la FSH chez un animal de rente, de plus grande taille : la brebis.

Pour cela, une étude a été réalisée sur des brebis Ile de France, pubères, toutes du même âge, dans le but d'évaluer l'effet potentialisant des anticorps sur les propres hormones des brebis traitées (hormones endogènes). L'étude de la spécificité a montré en effet une forte liaison de l'anticorps CF12 pour la FSH ovine et une liaison plus variable pour la LH ovine. Dans cet objectif, un traitement ne comprenant que l'injection d'un anticorps seul a été mis au point pour en évaluer son efficacité.

Dans les protocoles mis en place chez la brebis, l'anticorps a donc été injecté seul et non préalablement incubé avec la FSH exogène comme cela a été réalisé dans les études chez la Ratte. De plus, chaque anticorps a été injecté chez des brebis indemnes de toute stimulation préalable de l'ovaire : les animaux n'ont reçu aucun traitement hormonal de stimulation de l'ovulation avec une gonadotropine préalablement à l'injection de l'anticorps.

L'effet potentialisant de l'anticorps anti-FSH CF12 a été évalué au cours de protocoles réalisés en pleine saison sexuelle (janvier) ou en fin de saison sexuelle (fin du mois de mars). Les protocoles ont tous été réalisés sur des brebis dont le cycle ovulatoire a été préalablement synchronisé par la pose d'une éponge vaginale imprégnée d'un progestagène (45 mg d'acétate de fluorogestone (FGA) - MSD) pendant 14 jours. L'effet potentialisant a été analysé en comparant la réponse ovulatoire (nombre d'ovulations) et la mise en place d'un ou de plusieurs corps jaunes fonctionnels de bonne qualité (amplitude de la sécrétion de progestérone) chez des brebis contrôles (lot sérum physiologique), des brebis stimulées par un traitement de FSH porcine (lot FSH) et des brebis stimulées par un anticorps seul (lot anticorps).

Dans chaque protocole, un dosage de la LH plasmatique a été réalisé par méthode ELISA afin de détecter et dater le pic pré-ovulatoire de LH. Pour évaluer la réponse ovulatoire une observation endoscopique des ovaires a été réalisée par laparoscopie, sous anesthésie, huit jours après le retrait de l'éponge vaginale, afin de compter le nombre de corps jaunes et observer leur aspect.

Pour évaluer la fonctionnalité et la qualité du ou des corps jaunes, un dosage ELISA quantitatif de progestérone a été réalisé à partir de prises de sang quotidiennes du premier au 21^{ème} jour après le retrait de l'éponge.

Toutes les analyses statistiques ont été faites avec le logiciel GraphPad Prism Version 5.0 (GraphPad, San Diego, CA, USA).

### Anticorps CF12 et son scFv

L'effet potentialisant de CF12 (IgM) et de son scFv ont été étudiés et comparés en utilisant les paramètres de mesure de l'ovulation et de la qualité fonctionnelle du corps jaune mis en place. Les doses injectées ont été de 2 fois 1 mg.

Le protocole réalisé en saison sexuelle comportait quatre lots :
- le Iot anticorps CF12 (n=7) a reçu une injection intra-musculaire de 1 mg d'anticorps 24h avant retrait de l'éponge et une deuxième injection de 1 mg au moment du retrait de l'éponge
- le Iot scFv CF12 (n=5) a reçu une injection intra-musculaire de 1 mg de scFv 24h avant retrait de l'éponge et une deuxième injection de 1 mg au moment du retrait de l'éponge
- le Iot "contrôle" (n=9) a reçu une injection de sérum physiologique, par voie intramusculaire, 24h avant le retrait de l'éponge et au moment du retrait
- le lot "FSH" (n=11) a reçu une injection par voie intra-musculaire de 100 µg de FSH porcine (pFSH) 24h avant le retrait de l'éponge et de 90 µg 12h avant le retrait de l'éponge.

Les endoscopies des ovaires ont été réalisées 8 jours après retrait de l'éponge.

Des prises de sang quotidiennes du 1^{er} au 21^{ème} jour après retrait de l'éponge ont été faites pour doser la progestérone plasmatique par dosage ELISA.

L'analyse de la réponse ovulatoire a donné les résultats présentés dans le tableau 16 ci-dessous. L'analyse statistique a été faite par un test exact de Fisher.

**Tableau 16**

| | Lot sérum Φ | Lot FSH | Lot CF12 seul | Lot scFv CF12 seul |
|---|---|---|---|---|
| Nombre de brebis par Iot | 9 | 11 | 7 | 5 |
| Nombre de brebis ayant ovulé par Iot | 4/9 (44%) | 4/11 (36%) | **7/7 (100%) ***** | **5/5 (100%) ***** |
| Nombre de corps jaunes par brebis totales | 0,67 ± 0,3 | 0,9 ± 0,5 | 1,7 ± 0,4 | **2,2 ± 0,4 *** |
| Nombre de corps jaunes par brebis ayant ovulé | 1,5 ± 0,3 | 3 ± 1,4 | 1,7 ± 0,4 | 2,2 ± 0,4 |
| Moment du pic de LH (heures après retrait) | 64 ± 13 | 56 ± 5 | 52 ± 3,2 | 51,6 ± 4 |

| | | | | |
|---|---|---|---|---|
| *, p<0.05 ; ***, p<0.0001 | | | | |

Comparativement aux lots contrôle et FSH, les résultats obtenus dans le Iot CF12 et scFv CF12 montrent un effet très significatif de l'anticorps ou de son scFv injecté seul sur la réponse ovulatoire. En effet, 100% des femelles (7/7 pour CF12 et 5/5 pour le scFv CF12) ayant reçu deux injections de 1 mg d'anticorps ou de scFv ont ovulé contre 44% et 36% respectivement pour le lot sérum Φ et le lot FSH (p<0.0001, test exact de Fisher). Le nombre de corps jaunes obtenus par femelle sur l'effectif total du lot est significativement supérieur dans le lot scFv CF12 (p<0.05, test de Kruskall Wallis) par rapport aux lots FSH et sérum Φ: 2,2 corps jaunes versus 0,9 (FSH) et 0,67 (sérum Φ) respectivement. Il n'y a pas de différence significative entre le lot scFv CF12 et CF12.

Le moment moyen d'apparition du pic de LH n'est pas significativement différent entre les trois lots. On observe malgré tout une tendance à moins de variabilité dans la venue du pic de LH (donc du moment de l'ovulation) dans les lots CF12 et scFv CF12 par rapport aux lots FSH et surtout sérum Φ. Dans cette hypothèse, ceci indiquerait une meilleure synchronisation des ovulations chez les brebis ayant reçu l'anticorps ou son scFv.

Le profil de sécrétion de la progestérone au cours de la phase lutéale suite à l'ovulation, dans les différents lots est illustré dans la figure 12A. Pour chaque individu, les valeurs de concentration en progestérone (ng/ml) ont été normalisées par nombre de corps jaunes. Chaque courbe de la figure représente la moyenne des valeurs de progestérone mesurée à chaque prélèvement chez les femelles de chaque lot. Les courbes de sécrétion obtenues avec les lots CF12 et scFv CF12 sont très nettement au-dessus des lots FSH et sérum Φ. Les résultats obtenus indiquent des valeurs moyennes de progestérone de 1,46 - 1,4 - 1,1 et 0,6 ng/ml respectivement pour les lots scFv CF12, CF12, FSH et sérum Φ à J10 après retrait de l'éponge et de 1,93 - 1,78 - 1,22 et 1 ng/ml à J15.

La comparaison des quatre courbes a été faite par un test t non paramétrique apparié (test de Wilcoxon). Les courbes des lots CF12 et scFv CF12 sont significativement différentes de la courbe du lot sérum Φ (p < 0.01). De même, la courbe du lot FSH est significativement différente de celle du lot contrôle (p < 0.001). La différence entre les courbes des brebis traitées avec CF12 et scFv CF12 avec la courbe des brebis traitées avec FSH n'est pas significative et représente donc une tendance.

Une analyse de l'aire sous la courbe (AUC) a été réalisée avec le logiciel GraphPad Prism version 5.0 pour quantifier les différences entre courbes de sécrétion de la progestérone. Les résultats sont illustrés sur la figure 12B et indiquent que l'AUC de la courbe scFv CF12 (16,98 unités) est significativement plus élevé d'un facteur 1,55 de l'AUC de la courbe sérum Φ (8 unités) (p<0.01, test t non paramétrique de Mann-Whitney). De même, l'AUC des courbes CF12 (16,78 unités) et sérum Φ (8 unités) sont significativement différentes (p<0.05, test t non paramétrique de Mann-Whitney). Par contre, il n'y a pas de différence significative entre AUC de la courbe FSH (10,82 unités) et de la courbe sérum Φ indiquant que le traitement avec CF12 ou avec le scFv CF12 est plus efficace que le traitement avec FSH, dans le cadre de cette expérience.

En conclusion, l'ensemble des résultats indiquent que le fragment monovalent de CF12 a les mêmes effets potentialisants que l'anticorps bivalent CF12. Il n'a jamais été observé de différence significative entre les réponses des deux lots de brebis concernées. Le traitement avec l'une ou l'autre des deux molécules sous forme de deux injections intra-musculaires de 1 mg chacune a donné de façon très significative de meilleurs résultats qu'un traitement classique avec FSH :
- en termes d'efficacité sur l'induction de l'ovulation (100% des brebis ont ovulé et 1,3 et 0,8 corps jaunes supplémentaires sont obtenus par brebis sur l'effectif total)
- en termes de qualité des corps jaunes mis en place avec une sécrétion de progestérone plus élevée tout au long de la phase lutéale.

L'ensemble des résultats indique que l'anticorps potentialisant CF12, injectés *in vivo* chez la brebis, sont capables de complexer les hormones gonadotropes endogènes de l'animal et de potentialiser l'activité biologique des hormones propres à l'animal.

L'effet potentialisant de l'anticorps CF12 chez la brebis est capable d'induire une stimulation de l'ovaire plus forte que le traitement hormonal FSH classique : l'induction des ovulations est de 100% en saison sexuelle et dans tous les cas une augmentation importante de la concentration circulante de la progestérone est maintenue tout au long de la phase lutéale. Cet effet supplémentaire est majeur pour diminuer les taux d'échec du développement embryonnaire progestagène dépendant et les risques d'avortement.

Il a été montré que le fragment monovalent scFv de CF12 induit les mêmes effets potentialisants que l'anticorps entier aussi bien sur l'induction de l'ovulation que sur la qualité du corps jaune et l'augmentation de la sécrétion de progestérone chez la brebis. De plus, nous avons constaté que l'injection du scFv CF12 dans notre protocole n'a pas induit de sécrétion d'anticorps anti-scFv CF12 chez les brebis traitées. La perspective d'une utilisation de fragment monovalent diminue donc les risques de réponse immunitaire humorale pouvant être induite chez certaines brebis.

### EXEMPLE 5 : MESURE IN VIVO DE L'EFFET POTENTIALISANT DU LIGAND CF12 DE L'INVENTION SUR LA BIOACTIVITE DE LA FSH CHEZ LA GUENON

Après avoir démontré et caractérisé l'effet potentialisant de l'anticorps monoclonal CF12 *in vivo* chez le rat, la ratte et la brebis, son effet potentialisant a été étudié chez une espèce proche de l'humain : le singe Cynomolgus (*Macaca fascicularis*). Pour cela, une étude a été réalisée sur des guenons pubères de plus de 36 mois, dans le but d'évaluer l'effet potentialisant de l'anticorps sur la FSH humaine (hFSH).

Dans les protocoles mis en place, l'anticorps a été injecté soit en complexe avec la hFSH (l'anticorps ayant été préalablement incubé avec la FSH exogène), soit injecté seul, 20 minutes après une injection de hFSH.

Au premier jour des règles, les guenons ont reçu une injection de 1,5 mg de préparation de GnRH à libération prolongée (Décapeptyl® L.P. 3 mg - IPSEN Pharma) par voie intra-musculaire. Quinze jours après l'injection de GnRH, les guenons ont été traitées selon différents protocoles. Une seule guenon a été traitée par protocole.

Trente-six heures après la dernière injection de hFSH, 1000 UI de hCG (Gonadotrophine Chorionique ENDO 5000 - MSD) ont été injectées aux animaux. Les ovocytes ont été ponctionnés par laparotomie 36 heures après l'injection de hCG, et observés au microscope pour évaluer leur degré de maturité.

L'effet potentialisant a été analysé en comparant la croissance folliculaire induite (surface des follicules et amplitude de la sécrétion d'œstradiol) et la mise en place de corps jaunes de bonne qualité (amplitude de la sécrétion de progestérone). Pour cela, des échographies ovariennes trans-abdominales ont été pratiquées toutes les 48 heures afin de compter les follicules et mesurer leur surface (exprimée en mm²). Des prises de sang réalisées toutes les 48 heures du premier jour de traitement jusqu'à 30 jours après les ponctions folliculaires ont permis de réaliser des dosages ELISA quantitatifs de l'oestradiol (exprimés en pg/ml) et de la progestérone (exprimés en ng/ml).

Toutes les analyses statistiques ont été faites avec le logiciel GraphPad Prism Version 5.0 (GraphPad, San Diego, CA, USA).

### 1/ Effet du ligand CF12 administré seul après l'injection de 25UI de hFSH

L'effet potentialisant de CF12 a d'abord été évalué sur la hFSH. Pour cela, 3 protocoles, notés de 1 à 3, ont été réalisés :
1- animal traité avec une seule injection de 25UI de hFSH : une injection de 25 UI de FSH humaine (Gonal-f® stylo pré-rempli - Merck Serono) par voie sous-cutanée, le premier jour du traitement ("hFSH 25UI X1").
2- animal traité avec CF12 + hFSH : une injection d'anticorps CF12 (400µg) 20 minutes après une injection de 25 UI de FSH humaine (Gonal-f® stylo pré-rempli - Merck Serono) pendant 20 minutes, a été réalisée par voie sous-cutanée, le premier et le cinquième jour du traitement ("hFSH 25UI+CF12 X2")
3- animal traité avec 25UI de hFSH pendant 8 jours: une injection quotidienne de 25 UI de FSH humaine (Gonal-f® stylo pré-rempli - Merck Serono) par voie sous-cutanée, pendant 8 jours ("hFSH 25UI X8").

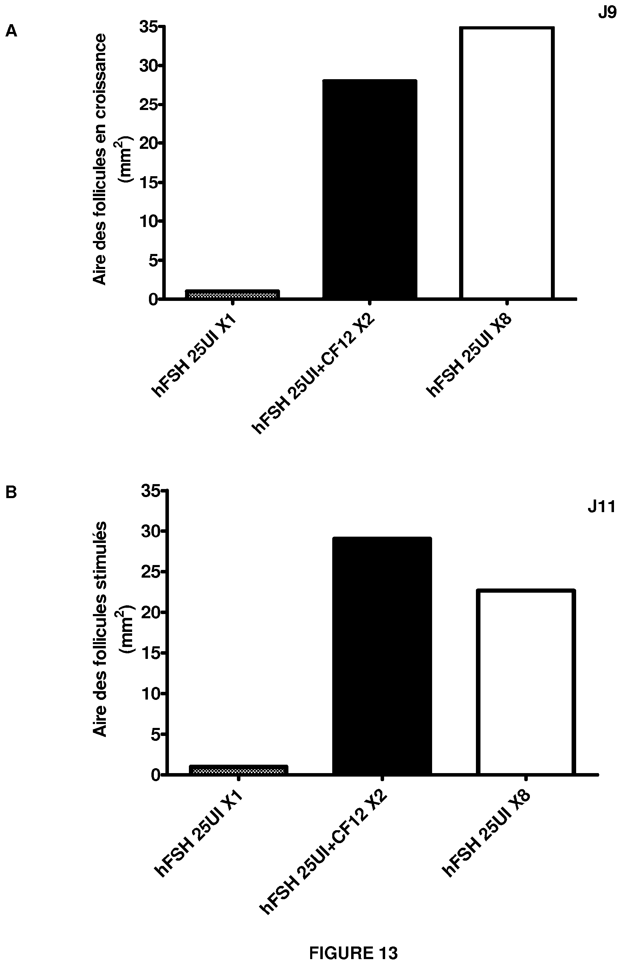

L'effet des trois traitements a été suivi en mesurant par échographie la croissance folliculaire induite (surface des follicules). Les résultats obtenus neuf jours (J9) après le début du traitement sont illustrés sur la figure 13A. Ils montrent qu'au 9^{ème} jour après le début du traitement FSH, la guenon traitée avec une seule injection de 25 UI de hFSH ne présentait aucun follicule stimulé (aire sous la courbe nulle). A l'inverse, la guenon traitée deux fois avec le mélange CF12 400µg+hFSH 25 UI, le 1^{er} et le 5^{ème} jour de traitement, présentait une aire totale des follicules stimulés de 28 mm². Chez la guenon ayant reçu 8 injections de hFSH, l'aire totale des follicules stimulés était de 35 mm².

Les résultats obtenus onze jours (J11) après le début du traitement sont illustrés sur la figure 13B. Ils montrent que la guenon traitée deux fois avec CF12+hFSH, présentait alors une aire totale des follicules de 29 mm² avec 6 follicules stimulés. Comparativement, l'aire des follicules mesurée chez la guenon ayant reçu 8 injections de hFSH était de 22,6 mm² avec 11 follicules stimulés. Deux injections de CF12 400µg+hFSH 25UI ont donc induit une meilleure croissance folliculaire que 8 injections de 25 UI de hFSH : 4,83 mm2/follicule versus 2,05 mm2/follicule respectivement. L'effet du complexe sur la croissance folliculaire a été constant jusqu'à J11 contrairement au traitement comportant 8 injections de FSH pour lequel on note une chute de l'aire des follicules stimulés. Ce résultat met en évidence un effet potentialisant de CF12 *in vivo* chez la guenon sur la FSH circulante, qu'elle soit endogène ou exogène. En effet, la demi-vie de la FSH étant inférieure à 1 heure, les effets observés lorsque CF12 est injecté avec 25UI de hFSH au 1er et au 5ème jour de traitement ne peuvent être attribués uniquement à un effet sur la FSH co-injectée, mais reflètent également un effet sur la FSH endogène de la guenon.

### 2/ Effet du ligand CF12 administré seul après l'injection de 37,5 UI de hFSH

L'effet potentialisant de CF12 injecté seul de façon différée après l'injection de 37,5 UI de hFSH a ensuite été étudié. Pour cela, 4 protocoles notés de 1 à 4, ont été mis en place quinze jours après l'injection de GnRH. Une guenon a été traitée par protocole :
1- animal traité avec 37,5UI de hFSH pendant 12 jours : une injection quotidienne de 37,5 UI de FSH humaine (Gonal-f® stylo pré-rempli - Merck Serono) par voie sous-cutanée, pendant 12 jours ("hFSH 37,5UI X12").
2- animal traité avec 37,5UI de hFSH tous les jours, et 400µg de CF12 tous les 2 jours : une injection quotidienne de 37,5 UI de FSH humaine (Gonal-f® stylo pré-rempli - Merck Serono) par voie sous-cutanée, pendant 12 jours, et une injection de l'anticorps CF12 à une dose de 400µg, tous les 2 jours, 20 minutes après l'injection de hFSH ("hFSH 37,5UI X12 + 400µg CF12 X6").
3- animal traité avec 37,5UI de FSH tous les jours, et 70µg de CF12 tous les 2 jours : une injection quotidienne de 37,5 UI de FSH humaine (Gonal-f® stylo pré-rempli - Merck Serono) par voie sous-cutanée, pendant 12 jours, et une injection de l'anticorps CF12 à une dose de 70 µg, tous les 2 jours, 20 minutes après l'injection de hFSH ("hFSH 37,5UI X12 + 70µg CF12 X6").
4- animal traité avec 75UI de FSH pendant 8 jours : une injection quotidienne de 75 UI de FSH humaine (Gonal-f® stylo pré-rempli - Merck Serono) par voie sous-cutanée, pendant 8 jours ("hFSH 75UI X8").

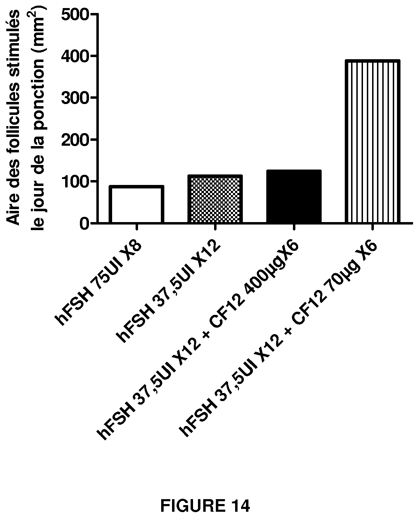

L'effet des quatre traitements a été suivi en mesurant la croissance folliculaire induite par échographie (surface des follicules en mm2). La figure 14 représente les surfaces des follicules stimulés obtenues à l'issue de chaque traitement, le jour de la ponction folliculaire (J15). L'intensité de la stimulation folliculaire varie selon le traitement. Elle a été maximale et très importante chez la guenon ayant reçu le traitement "hFSH 37,5UI X12 + 70µg CF12 X6" pour lequel une surface de 387,5 mm2 a été mesurée. Elle est 3,5 fois supérieure à la surface mesurée chez la guenon contrôle "hFSH 37,5UI X12" qui était de 112,3 mm2 et 3,2 fois supérieure à la surface de 124,2 mm2 obtenue chez la guenon traitée avec "hFSH 37,5UI X12 + 400µg CF12 X6". L'aire des follicules la plus faible (87,2 mm2) a été obtenue chez la guenon traitée pendant 8 jours avec hFSH 75UI.

Le nombre total de follicules obtenus le jour de la ponction est de 7 avec "hFSH 37,5UI X12 + 400µg CF12 X6" et "hFSH 75UI X8", et de 10 avec "hFSH 37,5 UI X12" et "37,5UI X12 + 70µg CF12 X6" (Tableau 17).

**Tableau 17 : Variation du nombre de follicules stimulés et de leur taille suite aux différents traitements.**

| | Follicules < 5 mm | Follicules entre 5 et 7 mm | Follicules ≥ 7 mm | Diamètre du plus gros follicule (mm) |
|---|---|---|---|---|
| hFSH 37,5 UI X12 | 10 | 2 | 0 | 5,61 |
| hFSH 37,5 UI X12 + 400 µg CF12 X6 | 4 | 3 | 0 | 6,82 |
| hFSH 37,5 UI X12 + 70µg CF12 X6 | 2 | 3 | 5 | 9,15 |
| hFSH 75 UI X8 | 4 | 3 | 0 | 6,14 |

Il est à souligner que la taille du plus gros follicule varie considérablement entre traitements. Ainsi, le traitement "37,5UI X12 + 70µg CF12 X6" a induit la formation de 5 follicules supérieurs à 7 mm de diamètre (dont le plus gros a un diamètre de 9,15 mm), alors que tous les autres traitements n'ont induit que des follicules inférieurs à 7 mm.

Le nombre d'ovocytes ponctionnés a été de 11 ovocytes avec "hFSH 37,5UI X12 + 400µg CF12 X6" et "hFSH 75UI X8", et de 8 ovocytes avec hFSH 37,5UI X12. La guenon traitée avec hFSH 37,5UI X12 + 70µg CF12 X6 présentait de jeunes corps jaunes sur les ovaires ce qui indique qu'elle a ovulé spontanément avant le jour de la ponction.

Ces résultats démontrent un effet potentialisant très important de CF12 administré à la dose de 70 µg se traduisant à la fois par une croissance folliculaire très supérieure à celle induite par la FSH seule et une réponse ovulatoire très stimulée et avancée. La différence de réponse observée entre les traitements "hFSH 37,5UI X12 + 400µg CF12 X6" et "hFSH 37,5UI X12 + 70µg CF12 X6" indique également que l'effet potentialisant exercé par CF12 est dose-dépendant, la dose 70µg induisant la plus forte stimulation des ovaires.

L'effet des quatre traitements a également été analysé et comparé en mesurant la sécrétion de l'oestradiol et de la progestérone toutes les 48 heures du premier jour de traitement jusqu'à 30 jours après les ponctions folliculaires.

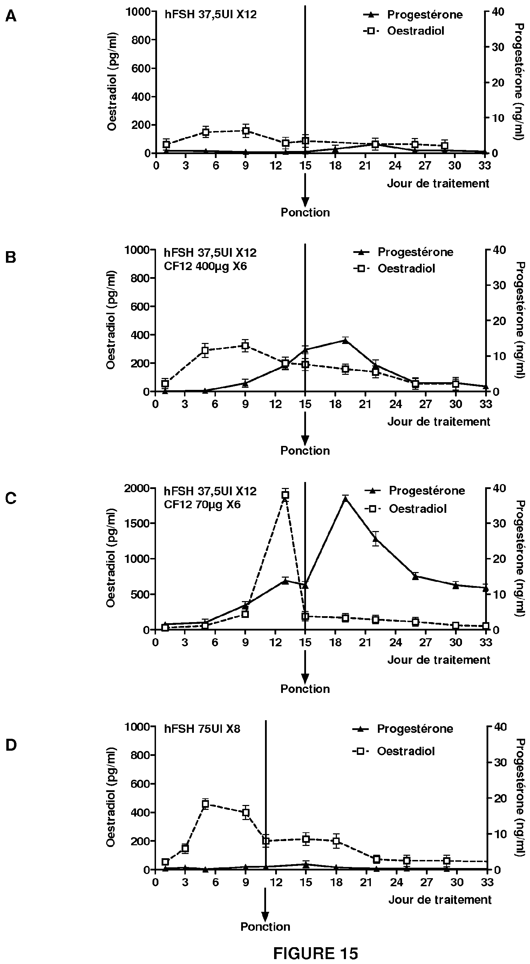

Les résultats sont illustrés sur la Figure 15, chaque traitement étant présenté par un graphe (A-B-C-D) montrant les profils de sécrétion de l'oestradiol et de la progestérone obtenus pour chaque guenon tout au long du cycle.

Les guenons traitées avec 37,5 UI de hFSH seule (Figure 15A) ou en association avec un traitement de CF12 à 400 (Figure 15B) et 70µg (Figure 15C) présentent des profils de sécrétion d'oestradiol tous significativement différents les uns des autres (**** p<0,0001, Two-way ANOVA). A J9, la concentration est respectivement de 157 pg/ml, 323 pg/ml et 220 pg/ml indiquant une meilleure réponse oestrogénique avec les traitements associant CF12. A J13, deux jours avant la ponction des follicules, la concentration en oestradiol est respectivement de 70 pg/ml (Fig 15A), 200 pg/ml (Fig 15B) et 1950 pg/ml (Fig 15C). Comparativement, elle est de 395 ng/ml chez la guenon traitée avec hFSH 75UI X8 (Fig 15D), deux jours avant la ponction folliculaire. Ces résultats démontrent un effet potentialisant très important de CF12 sur la réponse oestrogénique se traduisant par une augmentation du pic d'oestradiol d'un facteur 3 avec la dose 400 µg de CF12 et d'un facteur 28 avec la dose de 70 µg de CF12 par rapport au traitement contrôle avec FSH seule. Le pic d'oestradiol important observé à J13 dans le cas "hFSH 37,5UI X12 + 70 µg CF12 X6" peut expliquer l'induction d'une ovulation précoce survenue avant la ponction folliculaire.

Les profils de sécrétion de la progestérone, traduisant la mise en place de corps jaunes de bonne qualité, ont été mesurés. La comparaison des figures 15A, 15B et 15C montre très clairement l'augmentation dose dépendante du niveau de progestérone chez les guenons traitées avec CF12 et FSH par rapport à la guenon traitée avec FSH seule. Ainsi à J19, quatre jours après la ponction folliculaire, la concentration en progestérone est de 2,4 ng/ml avec FSH seule (Fig. 15A), 14,5 ng/ml (X6) avec CF12 400µg (Fig. 15B) pour atteindre 37 ng/ml (X15) avec CF12 70 µg (Fig. 15C). Par comparaison, le niveau de progestérone mesuré 4 jours après la ponction folliculaire chez la guenon traitée avec FSH seule à 75 UI X8 est de 1,5 ng/ml (Fig. 15D) et n'est pas statistiquement différente du traitement 37,5UI X12. A l'inverse les traitements avec CF12 400µg et 70µg induisent des niveaux de progestérone statistiquement différents des traitements avec FSH seule (**** p<0,0001, Two-way ANOVA).

Ces résultats démontrent très clairement un effet potentialisant de CF12 sur la progestéronémie traduisant une meilleure qualité de corps jaunes chez les guenons ayant reçu l'anticorps. Le rôle important de la progestérone dans la préparation de l'endomètre, l'implantation et le développement précoce de l'embryon en fait un atout important chez les espèces animales et pour une application chez la femme.

Etant donné la demi-vie très courte de la FSH (moins d'une heure), l'ensemble des résultats (avec 25 ou 37,5UI de hFSH) confirment que l'anticorps CF12 est capable d'exercer un effet potentialisant *in vivo* sur la FSH circulante, qu'elle soit endogène ou exogène. Les effets très importants observés chez la guenon traitée avec 70 µg d'anticorps sont de fait associés à une action "longue durée" de l'anticorps sur l'hormone endogène de la guenon.
Par ces résultats, nous démontrons également que l'anticorps CF12 pourrait ouvrir sur la mise en place d'un nouveau traitement d'induction de l'ovulation en clinique humaine, particulièrement dans les traitements de PMA, que ce soit pour induire une mono-ovulation dans le cadre de l'Insémination artificielle ou une poly-ovulation dans le cadre de la Fécondation In Vitro (FIV) en définissant un dosage approprié.

### EXEMPLE 6: PREDICTION DE L'EPITOPE RECONNU PAR LE LIGAND CF12 DE L'INVENTION ET PREDICTION DE SON PARATOPE.

L'épitope de l'anticorps CF12 a été déterminé sur les hormones gonadotropes de différentes espèces en utilisant un algorithme d'amarrage des protéines basé sur une modélisation de la structure des protéines par un diagramme de Voronoï et une optimisation par différentes méthodes d'apprentissages évolutionnaires de fonctions de score permettant de différencier les conformations natives et non-natives (Bernauer et al., Bioinformatics 2007, 5:555) [14], (Bernauer et al., Bioinformatics 2008, 24:652) [15], (Bourquard et al., PLoS One 2011, 6:e18541) [16] et (Bourquard et al., Sci. Reports 2015, 5 :10760) [17].

Chaque anticorps a été amarré avec la FSH humaine (hFSH), la LH humaine (hLH), la CG humaine (hCG), la FSH ovine (oFSH) et la LH ovine (oLH), la FSH porcine (pFSH) et la LH porcine (pLH). Les structures cristallographiques de la hFSH et de la hCG sont disponibles dans la Protein Data Bank (PDB) : 4MQW et 1QFW respectivement. La structure de la FSH humaine complexée avec le domaine extracellulaire du récepteur FSH humain a été utilisé (Fan et Hendrickson, Nature 2005, 433:269) [18]. Pour les autres hormones (hLH, oFSH, oLH, pFSH et pLH), des modèles par homologie ont été réalisés puis utilisés pour l'amarrage.

La structure 3D de l'anticorps CF12 n'étant pas disponible, l'étude a été faite à partir des séquences des fragments monovalents VH et VL de CF12. Pour cela, des modèles par homologie des parties variables ont été réalisés. Les modèles des VH et VL ont été réalisés séparément, à partir de structures différentes, et leur orientation relative a été déterminée à partir de la structure ayant servi de support pour la modélisation du VH. Les structures utilisées pour les modèles par homologie sont disponibles dans la Protein Data Bank (PDB) : 1PLV pour le VH de CF12 et 3TT1 pour le VL de CF12.

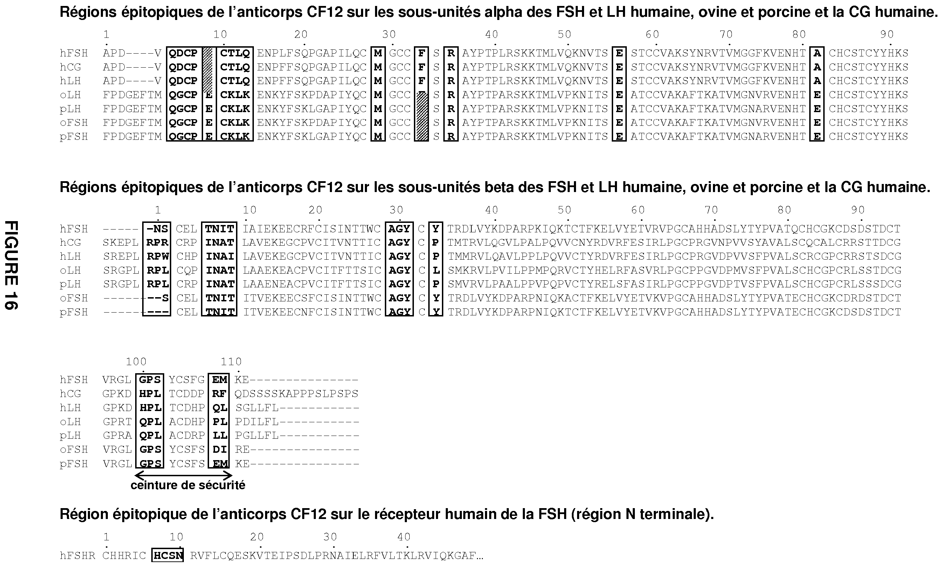

Les résultats d'amarrage sont illustrés sur la Figure 16. Il apparaît que le ligand CF12 s'amarre de manière similaire sur les sept hormones cibles. L'épitope est défini par plusieurs régions situées de façon discontinue sur les sous-unités alpha et béta des gonadotropines étudiées. L'épitope concerne également la séquence His7-Cys8-Ser9-Asn10 de l'ectodomaine du récepteur FSH humain. L'épitope du ligand CF12 est donc très conformationnel : il est constitué à la fois de plusieurs régions des sous-unités alpha et béta de l'hormone et d'une séquence du récepteur. L'ensemble de ces régions discontinues se trouvent spatialement proches dans la conformation native de l'hormone et de son récepteur activé.

Les différents résidus de l'hormone et du récepteur, impliqués dans l'interface avec le ligand CF12 sont entourés par des rectangles sur la figure 16. Les deux résidus notés en grisé sur la sous-unité alpha de hFSH sont impliqués dans l'interaction principale et ont donc un rôle majeur dans la reconnaissance anticorps/antigène : il s'agit de l'acide glutamique en position 9 (Glu9) et de la phenylalanine en position 33 (Phe33) de la sous-unité alpha de hFSH. Ces deux résidus sont identiques et reconnus dans la séquence des autres hormones cibles. Parmi les autres résidus de la sous-unité alpha, impliqués dans l'interface, on note une région comprenant 9 résidus dont Glu9. Il s'agit du motif Gln5-Asp6-Cys7-Pro8-Glu9-Cys10-Thr11-Leu12-Gln13.

On note également dans l'épitope la présence des résidus arginine en position 35 (Arg35) et acide glutamique en position 56 (Glu56) spatialement proches dans l'hormone native. Ces deux résidus fixent l'extrêmité C-terminale de la sous-unité béta, figeant ainsi la "ceinture de sécurité" autour de la sous-unité alpha. Ces deux résidus sont présents et reconnus de façon constante dans toutes les hormones cibles. Ils jouent un rôle important dans la stabilité et la bioactivité de l'hormone.. Par ce mécanisme, la liaison de l'anticorps sur ces résidus permettrait donc d'augmenter la stabilité du dimère FSH.

Le ligand CF12 reconnaît également les résidus 100 à 102 et 108-109 de l'extrêmité C-terminale de la sous-unité béta qui constitue la ceinture de sécurité. Le rôle de cette ceinture de sécurité ("seat belt") étant de stabiliser l'association du dimère alpha/béta de l'hormone, la liaison du ligand CF12 sur ces deux résidus contribuerait également à sécuriser la fermeture de la ceinture de sécurité permettant ainsi une meilleure stabilité du dimère, indispensable à la bioactivité de l'hormone.

Une autre caractéristique de l'épitope du ligand CF12 est l'implication des résidus 7 à 10 (His7-Cys8-Ser9-Asn10) de la région N-terminale du récepteur FSH humain dans l'interface reconnue par CF12. La liaison du ligand CF12 contribuerait également par ce mécanisme à favoriser l'interaction de l'hormone sur son récepteur et entraînant la mise en place d'un effet « potentialisant »

Le tableau 18, illustre les différentes régions du paratope du ligand CF12. La numérotation utilisée est celle des séquences SEQ ID N°2 et SEQ ID N°4.

Les deux chaînes VH et VL sont impliquées dans la reconnaissance de l'hormone, par leurs trois CDR et certains résidus de leurs frameworks.

Pour l'interaction principale, le résidu Glu9 de la sous-unité alpha de hFSH est reconnu respectivement par les résidus Tyr102, Asp104 du CDR3 de la chaîne VH et par le résidu Leu50 de la chaîne VL. Le résidu Phe33 de la sous-unité alpha est reconnu par les résidus Ser31 et Tyr33 du CDR1 de la chaîne VH et par le résidu Tyr52 du CDR2 de la chaîne VH.

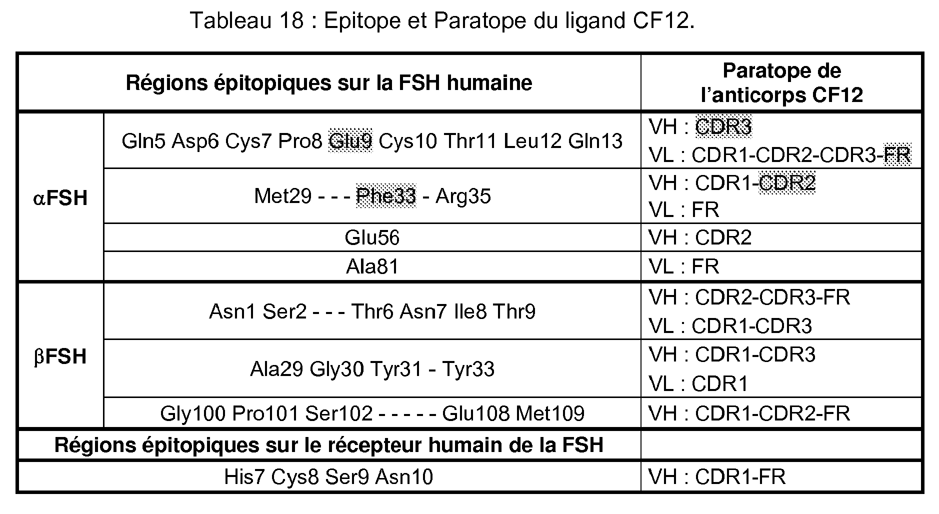

Tableau 18 : Différentes régions constituant l'épitope du ligand CF12 et celles constituant son paratope. Les résidus impliqués dans l'interaction principale sont indiqués en grisé.

L'interaction sur les résidus Arg35 et Glu56 de la sous-unité alpha implique plusieurs résidus de la chaîne VH. Les résidus Ser30 du CDR1 et Tyr52, Gly54-Thr55 du CDR2 interagissent avec Arg35. Le résidu Tyr52 du CDR2 interagit également avec Glu56 de la sous-unité alpha.

L'interaction sur l'extrémité C-terminale de la chaîne béta (ceinture de sécurité) implique plusieurs résidus de la chaîne VH : les résidus Ser30 du CDR1, Gly54 du CDR2 et Asp73-Thr74-Ser75 du framework 3.

Seule la chaîne VH est également impliquée dans la reconnaissance de l'ectodomaine du récepteur FSH, particulièrement son CDR1 avec la Glycine en position 26 (Gly26), et, certains résidus du framework 1 (Gln1-Gly2-Gln3, Lys23-Thr24-Ser25) et du framework 3 (Ser75).

En conclusion, le ligand CF12 est caractérisé par le fait qu'il reconnaît un épitope très conformationnel impliquant la sous-unité alpha de la hFSH, la sous-unité béta et particulièrement son extrémité C-terminale formant la ceinture de sécurité ainsi que l'ectodomaine du récepteur FSH. La chaîne VH est essentiellement impliquée dans l'interaction avec le récepteur et la chaîne VL dans l'interaction avec l'hormone. Cet épitope permet au ligand CF12, d'une part, de stabiliser l'association du dimère de l'hormone et, d'autre part, de stabiliser la liaison de l'hormone sur son récepteur. Ces deux mécanismes sont complémentaires pour aboutir à une meilleure interaction de l'hormone sur son récepteur et pourraient constituer les bases de l'effet potentialisant du ligand CF12 sur les gonadotropines.

### EXEMPLE 7: CONSTRUCTION, PRODUCTION ET CARACTERISATION DE DIFFERENTS FRAGMENTS DU LIGAND CF12 DE L'INVENTION.

Différents fragments de l'anticorps CF12 ont été construits afin d'évaluer leur capacité à potentialiser l'activité biologique de la FSH ovine et humaine. Il a été produit un fragment comportant la chaîne variable légère seule appelé "VL CF12", un fragment comportant la chaîne variable lourde seule appelé "VH CF12" et un "scFv CF12 inverse" construit dans un ordre VL-VH inversé par rapport à la séquence VH-VL du scFv CF12 (SEQ ID NO: 10 et SEQ ID NO: 11) de référence décrit dans l'exemple 1, paragraphe 4 de la présente invention.

### 1/ Construction et production des fragments d'anticorps

Les gènes de synthèse codant pour les fragments VL CF12 et scFv CF12 inverse dérivés de l'anticorps CF12 ont été synthétisés par ATG:Biosynthetics GmbH (Allemagne). Le scFv CF12 inverse est constitué de la fusion VL CF12 du scfv CF12-linker-VH CF12 du scFv CF12. Chaque gène de synthèse est conçu par la fusion de la séquence du plasmide pSW1 [7], comprise entre le site HindIII et la fin de la séquence codant pour la protéine PelB et la séquence de la protéine d'intérêt à synthétiser (SEQ ID NO : 27 et SEQ ID NO : 31), flanquée du site de restriction Xhol. Les séquences sont insérées entre les sites HindIII et XhoI du plasmide pSW1. Les codons ont été optimisés pour l'expression en *E. coli.*

Le plasmide d'expression pSW1VH CF12 a été obtenu par l'insertion, dans le plasmide pSW1 [7] aux sites Pstl-Xhol, du fragment issu de la digestion par ces mêmes enzymes du plasmide pSW1 scFv CF12 inverse.

Après contrôle par séquençage de la qualité des constructions, les plasmides pSW1-VL CF12, pSW1-VH CF12 et pSW1 scFv CF12 inverse ont été transformés par choc thermique en bactéries HB2151 (T53040, Interchim, France) rendues compétentes [8].

**Tableau 19 : Séquences nucléotidique et peptidique du VL CF12**

| VL CF12 | |
|---|---|
| Séquence nucléotidique SEQ ID NO : 27 | GATATTCAGATGACCCAGACCCCTGCGAGCCTGGCAGTGTCAC TGGGCCAACGCGCAACCATCTCGTGTAAAGCCTCGCAGAGCGT GGATTATGACGGCGATAGCTACATGAACTGGTATCAGCAAAAGC CTGGTCAACCGCCGAAGCTGCTGATTTACGCCGCCAGCAACCT GGAATCGGGCATCCCGGCCCGTTTTAGCGGCTCAGGCTCGGGT ACTGACTTCACGCTGAACATTCACCCGGTAGAAGAAGAAGACGC GGCCACGTATTACTGCCAGCAAAGCAATGAAGACCCGTACACTT TTGGCGGCGGCACGAAACTTGAGATCAAACACCATCACCATCAC CATTAA |
| Séquence peptidique SEQ ID NO : 28 | DIQMTQTPASLAVSLGQRATISCKASQSVDYDGDSYMNWYQQKP GQPPKLLIYAASNLESGIPARFSGSGSGTDFTLNIHPVEEEDAATYY CQQSNEDPYTFGGGTKLEIKHHHHHH* |

**Tableau 20 : Séquences nucléotidique et peptidique du VH CF12**

| VH CF12 | |
|---|---|
| Séquence nucléotidique SEQ ID NO : 29 | CAGGTGCAGCTGCAGCAGTCGGGTGGCGCAGAGCTGGTGAAA CCGGGTGCGAGCGTTAAACTGAGCTGCAAAACTAGCGGCTTTA CCTTTAGCTCGTCATATATTTCGTGGCTGAAGCAGAAACCGGGC CAGTCACTGGAATGGATTGCGTGGATCTACGCAGGCACGGGTG GCACCTCATATAATCAGAAATTCACCGGTAAAGCGCAACTGACG GTCGATACCAGCAGCAGCACGGCGTACATGCAGTTCAGCTCGC TGACCACTGAAGATAGCGCAATCTACTATTGTGCACGCCATGGT |
| | TCGTACTTCGACTATTGGGGCCAGGGCACCACCCTGACCGTTTC AAGCCACCATCACCATCACCATTAA |
| Séquence peptidique SEQ ID NO : 30 | QVQLQQSGGAELVKPGASVKLSCKTSGFTFSSSYISWLKQKPGQS LEWIAWIYAGTGGTSYNQKFTGKAQLTVDTSSSTAYMQFSSLTTED SAIYYCARHGSYFDYWGQGTTLTVSSHHHHHH* |

**Tableau 21 : Séquences nucléotidique et peptidique du scFv CF12 inverse**

| scFv CF12 inverse | |
|---|---|
| Séquence nucléotidique SEQ ID NO : 31 | GATATTCAGATGACCCAGACCCCTGCGAGCCTGGCAGTGTCAC TGGGCCAACGCGCAACCATCTCGTGTAAAGCCTCGCAGAGCGT GGATTATGACGGCGATAGCTACATGAACTGGTATCAGCAAAAGC CTGGTCAACCGCCGAAGCTGCTGATTTACGCCGCCAGCAACCT GGAATCGGGCATCCCGGCCCGTTTTAGCGGCTCAGGCTCGGGT ACTGACTTCACGCTGAACATTCACCCGGTAGAAGAAGACGC GGCCACGTATTACTGCCAGCAAAGCAATGAAGACCCGTACACTT TTGGCGGCGGCACGAAACTTGAGATCAAAGGTGGTGGTAG CGGTGGTGGTGGTTCAGGTGGCGGCGGCTCACAGGTGCAGCT GCAGCAGTCGGGTGGCGCAGAGCTGGTGAAACCGGGTGCGAG CGTTAAACTGAGCTGCAAAACTAGCGGCTTTACCTTTAGCTCGT CATATATTTCGTGGCTGAAGCAGAAACCGGGCCAGTCACTGGAA TGGATTGCGTGGATCTACGCAGGCACGGGTGGCACCTCATATA ATCAGAAATTCACCGGTAAAGCGCAACTGACGGTCGATACCAGC AGCAGCACGGCGTACATGCAGTTCAGCTCGCTGACCACTGAAG ATAGCGCAATCTACTATTGTGCACGCCATGGTTCGTACTTCGAC TATTGGGGCCAGGGCACCACCCTGACCGTTTCAAGCCACCATC ACCATCACCATTAA |
| Séquence peptidique SEQ ID NO : 32 | DIQMTQTPASLAVSLGQRATISCKASQSVDYDGDSYMNWYQQKP GQPPKLLIYAASNLESGIPARFSGSGSGTDFTLNIHPVEEEDAATYY CQQSNEDPYTFGGGTKLEIKGGGGSGGGGSGGGGSQVQLQQSG GAELVKPGASVKLSCKTSGFTFSSSYISWLKQKPGQSLEWIAWIYA GTGGTSYNQKFTGKAQLTVDTSSSTAYMQFSSLTTEDSAIYYCARH GSYFDYWGQGTTLTVSSHHHHHH* |

La production des fragments a été réalisée selon le procédé préalablement décrit dans l'exemple 1 de la présente invention.

### 2/ Mesure in vitro de l'effet des fragments VL CF12, VH CF12 et scFv CF12 inverse sur la bioactivité de la FSH

L'effet *in vitro* des fragments "VL CF12", "VH CF12" et "scFv CF12 inverse" sur la bioactivité de la FSH humaine a été étudié avec la lignée cellulaire HEK293 transfectée de façon stable avec le récepteur FSH humain et le système Glosensor® selon le protocole préalablement décrit dans l'exemple 2 de la présente invention. Les fragments "VL CF12" et "VH CF12" seuls ou en mélange ont été testés à 40 nM chacun. Le scFv CF12 inverse a été testé à la concentration de 80 nM tout comme le scFv CF12 de référence. La FSH humaine a été testée à 0,1 nM.

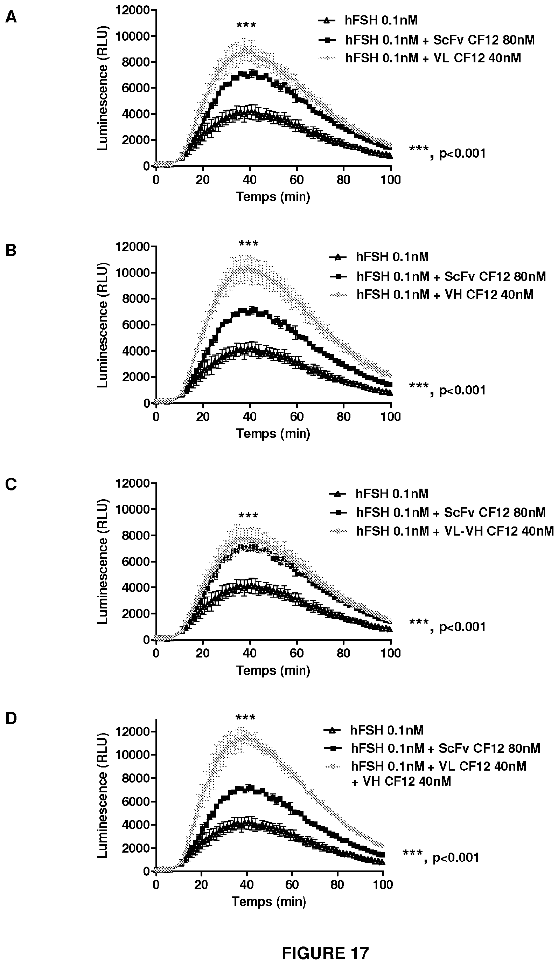

La figure 17 illustre les courbes de cinétique de production d'AMPc exprimées en unités relatives de luminescence en fonction du temps (en minutes) obtenues en présence de 0,1 nM de FSH humaine (hFSH) seule ou complexée aux différents fragments de CF12. Quatre conditions ont été comparées à la hFSH seule: le complexe hFSH+ VL CF12 (Fig. 17A), le complexe hFSH+VH CF12 (Fig. 17B), le complexe hFSH+scFv CF12 inverse (Fig. 17C) et le complexe de la hFSH avec un mélange équimolaire de 40 nM de VL CF12 et 40 nM de VH CF12 (Fig. 17D). Les niveaux de réponse luminescente sont comparés à 40 mn de stimulation.

On observe que le fragment "VL CF12" à la concentration de 40 nM complexé à hFSH 0,1 nM exerce un effet potentialisant très significatif (p<0,001) qui augmente la réponse cellulaire de 218% de façon comparable au scFv CF12 (180% d'augmentation) par rapport à la stimulation avec hFSH seule (Fig. 17A). Le fragment "VH CF12" à la concentration de 40 nM complexé à hFSH 0,1 nM exerce un effet potentialisant très significatif (p<0,001) augmentant la réponse cellulaire de 250% de façon plus importante que le scFv CF12 (180% d'augmentation) par rapport à la stimulation avec hFSH seule (Fig. 17B).

Le scFv CF12 inverse (Fig. 17C) potentialise l'action de la FSH de façon identique au scFv CF12 de référence, les deux donnant une augmentation du signal luminescent de 180% par rapport à la réponse à FSH; cet effet est significatif (p<0,001).

Enfin, le mélange des deux fragments VH CF12 + VL CF12 (Fig. 17D) complexés à la hFSH induit une augmentation significative (p<0,001) de la réponse cellulaire de 278%, plus importante que le scFv CF12 de référence (augmentation de 180%).

L'ensemble de ces résultats indique que les fragments VL ou VH isolés sont capables d'exercer un effet potentialisant sur la bioactivité de la FSH. Ils valident en cela la prédiction du modèle d'interaction, décrit dans l'exemple 6 de la présente invention, montrant l'implication des deux chaînes variables dans l'interaction sur le complexe FSH/récepteur. Le mélange des deux fragments VL et VH exerce l'effet potentialisant le plus important dans cet essai.

### 3/ Mesure in vivo de l'effet potentialisant des fragments VL CF12, VH CF12 et scFv CF12 inverse sur la bioactivité de la FSH chez la ratte

Après avoir été caractérisé *in vitro,* l'effet potentialisant des différents fragments de CF12 sur la bioactivité de la hFSH a été étudié *in vivo,* chez le rat femelle.

Pour mesurer la bioactivité FSH, le protocole utilisé a été celui du dosage biologique de Steelman et Pohley (Steelman SL, Pohley FM. Endocrinology, 53 :604-616. 1953) [12] tel que décrit dans l'exemple 3 de la présente invention. Chaque Iot comportait 5 rattes.

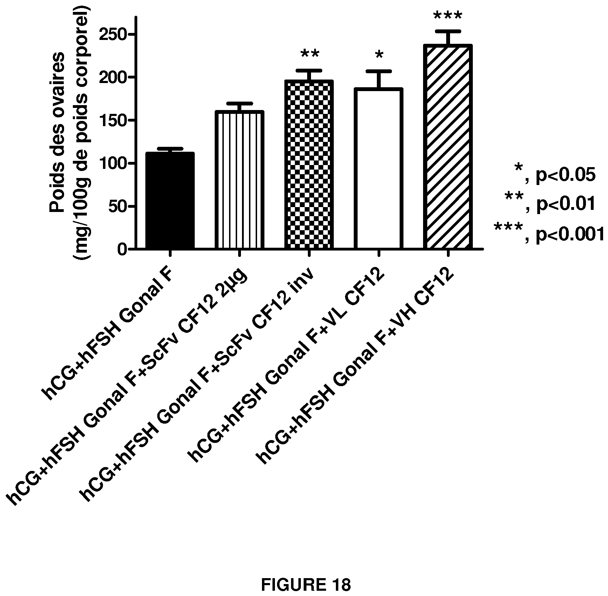

Les résultats sont illustrés sur la Figure 18. Le lot traité avec la hFSH complexée au scFv CF12 inverse a donné un poids moyen des ovaires de 195 ± 15 mg/100g de poids corporel, légèrement supérieur à celui du lot traité avec le complexe hFSH/scFv CF12 de référence (160 ± 5 mg), soit une augmentation de 175% par rapport au lot ayant reçu le traitement hormonal seul (p<0,01). Les lots traités avec les fragments VL CF12 et VH CF12 complexés à la hFSH ont eu un poids moyen des ovaires de 186 ± 24 mg et 237 ± 15 mg respectivement soit une augmentation de 167% et 213% par rapport au lot ayant reçu le traitement hormonal seul (p<0,05 et p<0,001).

Ces résultats démontrent que l'ordre de construction du scFv (VL-VH versus VH-VL) complexé à la hFSH n'affecte pas les propriétés de potentialisation du scFv sur la bioactivité de la FSH. Les résultats démontrent également de façon très significative que les chaînes variables de CF12, particulièrement le fragment VL, sont capables de potentialiser, *in vivo* comme *in vitro,* la bioactivité de l'hormone. Ceci traduit l'implication respective des deux chaînes dans cet effet comme le prédit le modèle d'interaction décrit dans l'exemple 7 de la présente invention.

### Listes des références

- 1-: Brevet EP 1518863
- 2-: Demande Internationale WO 2012/066519
- 3-: Brochet et al., Nucl. Acids Res., 36 : W503-508, 2008
- 4-: Giudicelli et al., Cold Spring Harb Protoc., 2011(6) : 695-715, 2011
- 5-: Giudicelli et al., Nucl. Acids Res., 33 : D256-261, 2005
- 6-: Corpet, Nucl. Acids Res., 16(22) : 10881-10890, 1988
- 7-: Ward et al. Nature, 341 : 544-546, 1989)
- 8-: Li et al., Afr. J. Biotechnol., 9(50) : 8549-8554, 2010
- 9-: Chopineau et al., Mol. Cell Endocrinol., 92(2) : 229-239, 1993
- 10-: Wehbi et al., Endocrinology, 151(6) : 2788-2799, 2010
- 11-: Reverchon et al., Human Reprod., 27(6) : 1790-1800, 2012
- 12-: Steelman SL, Pohley FM., Endocrinology, 53 :604-616, 1953
- 13-: Scobey et al, Reprod. Biol. Endocr. 3 :61, 2005
- 14-: Bernauer et al., Bioinformatics, 5 :555, 2007
- 15-: Bernauer et al., Bioinformatics, 24 :652, 2008
- 16-: Bourquard et al., PLoS One, 6:e18541, 2011
- 17-: Bourquard et al., Sci. Reports, 5 :10760, 2015
- 18-: Fan et Hendrickson, Nature, 433 :269, 2005.

## Revendications

1. Ligand de l'hormone folliculo-stimulante (FSH) potentialisant la bioactivité de la FSH, de l'hormone lutéinisante (LH) et de la gonadotropine chorionique (CG), **caractérisé en ce que** ledit ligand est un anticorps ou un fragment de celui-ci et **en ce que** :
le domaine variable de la chaine lourde contient les CDRs suivants :
- VH-CDR1, défini par la séquence GFTFSSSY (SEQ ID NO : 5) ;
- VH-CDR2, défini par la séquence IYAGTGGT (SEQ ID NO : 6) ;
- VH-CDR3, défini par la séquence ARHGSYFDY (SEQ ID NO : 7) ; et
le domaine variable de la chaine légère contient les CDRs suivants :
- VL-CDR1, défini par la séquence QSVDYDGDSY (SEQ ID NO : 8) ;
- VL-CDR2, défini par la séquence AAS ;
- VL-CDR3, défini par la séquence QQSNEDPYT (SEQ ID NO : 9).

2. Ligand de l'hormone folliculo-stimulante (FSH) potentialisant la bioactivité de la FSH, de l'hormone lutéinisante (LH) et de la gonadotropine chorionique (CG), **caractérisé en ce que** ledit ligand est un anticorps ou un fragment de celui-ci et **en ce que** :
le domaine variable de la chaine lourde contient la séquence SEQ ID NO : 2 ou 30 ;
et/ou
le domaine variable de la chaine légère contient la séquence SEQ ID NO : 4 ou 28.

3. Ligand selon la revendication 1 ou 2, **caractérisé en ce que** le ligand est choisi dans le groupe constitué de : Fab, Fab', F(ab')2, Fv, dsFv, scFv, diabodies, triabodies, tétrabodies, domaine variable de la chaine lourde, et domaine variable de la chaine légère.

4. Ligand selon la revendication 1, **caractérisé en ce que** le ligand est l'anticorps monoclonal CF12 produit par l'hybridome CNCM I-4803.

5. Ligand selon la revendication 1, **caractérisé en ce que** la séquence peptidique du scFv est la séquence SEQ ID NO : 11.

6. Ligand selon l'une quelconque des revendications 1 à 5, pour une utilisation comme médicament.

7. Complexe ligand-gonadotrophine choisi parmi :
un complexe d'un ligand selon l'une quelconque des revendications 1 à 5 avec la FSH ou un peptide actif de celle-ci ;
un complexe d'un ligand selon l'une quelconque des revendications 1 à 5 avec la LH ou l'hormone gonadotropine chorionique (CG) ou un peptide actif de celles-ci.

8. Complexe selon la revendication 7, pour utilisation comme médicament.

9. Ligand pour une utilisation selon la revendication 6 ou complexe pour une utilisation selon la revendication 8, où ledit médicament est destiné à induire l'ovulation ou une polyovulation chez un mammifère femelle.

10. Ligand pour une utilisation selon la revendication 6 ou complexe pour une utilisation selon la revendication 8, où ledit médicament est destiné à augmenter le taux de progestérone endogène circulant chez un mammifère femelle.

11. Composition pharmaceutique destinée à être utilisée pour l'induction de l'ovulation ou d'une polyovulation chez un mammifère femelle, **caractérisée en ce qu'**elle comprend un ligand selon l'une quelconque des revendications 1 à 5 et/ou un complexe selon la revendication 7 et un véhicule pharmaceutiquement acceptable.

12. Composition pharmaceutique selon la revendication 11, **caractérisée en ce qu'**elle comprend en outre une FSH et/ou une LH et/ou une CG.

13. Ligand selon l'une quelconque des revendications 1 à 5 ou complexe selon la revendication 7, pour une utilisation dans le traitement et/ou la prévention de l'infertilité ou de l'hypofertilité chez un mammifère.

14. Ligand selon l'une quelconque des revendications 1 à 5 ou complexe selon la revendication 7, pour une utilisation pour stimuler la procréation chez un mammifère femelle.

## Patentansprüche

1. Ligand von follikelstimulierendem Hormon (FSH), der die Bioaktivität von FSH, luteinisierendem Hormon (LH) und Choriogonadotropin (CG)-Hormon verstärkt, **dadurch gekennzeichnet, dass** es sich bei dem Liganden um einen Antikörper oder ein Fragment davon handelt und dass:
die variable Domäne der schweren Kette die folgenden CDRs enthält:
- VH-CDR1, definiert durch die Sequenz GFTFSSSY (SEQ ID NO: 5);
- VH-CDR2, definiert durch die Sequenz IYAGTGGT (SEQ ID NO: 6);
- VH-CDR3, definiert durch die Sequenz ARHGSYFDY (SEQ ID NO: 7); und
die variable Domäne der leichten Kette die folgenden CDRs enthält:
- VL-CDR1, definiert durch die Sequenz QSVDYDGDSY (SEQ ID NO: 8);
- VL-CDR2, definiert durch die Sequenz AAS;
- VL-CDR3, definiert durch die Sequenz QQSNEDPYT (SEQ ID NO: 9).

2. Ligand von follikelstimulierendem Hormon (FSH), der die Bioaktivität von FSH, luteinisierendem Hormon (LH) und Choriogonadotropin (CG)-Hormon verstärkt, **dadurch gekennzeichnet, dass** es sich bei dem Liganden um einen Antikörper oder ein Fragment davon handelt und dass:
- die variable Domäne der schweren Kette die Sequenz SEQ ID NO: 2 oder 30 enthält
und/oder
- die variable Domäne der leichten Kette die Sequenz SEQ ID NO: 4 oder 28 enthält.

3. Ligand nach einem der Ansprüche 1 oder 2, **dadurch gekennzeichnet, dass** der Ligand aus der Gruppe bestehend aus Fab, Fab', F(ab')2, Fv, dsFv, scFv, Diabodies, Triabodies, Tetrabodies, variabler Domäne der schweren Kette und variabler Domäne der leichten Kette ausgewählt ist.

4. Ligand nach Anspruch 1, **dadurch gekennzeichnet, dass** es sich bei dem Liganden um den durch das Hybridom CNCM I-4803 produzierten monoklonalen CF12-Antikörper handelt.

5. Ligand nach Anspruch 1, **dadurch gekennzeichnet, dass** es sich bei der Peptidsequenz von scFv um die Sequenz SEQ ID NO: 11 handelt.

6. Ligand nach einem der Ansprüche 1 bis 5 zur Verwendung als Medikament.

7. Ligand-Gonadotropin-Komplex, ausgewählt aus:
einem Komplex eines Liganden nach einem der Ansprüche 1 bis 5 mit FSH oder einem aktiven Peptid davon;
einem Komplex eines Liganden nach einem der Ansprüche 1 bis 5 mit LH oder Choriogonadotropin (CG)-Hormon oder einem aktiven Peptid davon.

8. Komplex nach Anspruch 7 zur Verwendung als Medikament.

9. Ligand zur Verwendung nach Anspruch 6 oder Komplex zur Verwendung nach Anspruch 8, wobei das Medikament zur Induktion der Ovulation oder einer Polyovulation bei einem weiblichen Säugetier vorgesehen ist.

10. Ligand zur Verwendung nach Anspruch 6 oder Komplex zur Verwendung nach Anspruch 8, wobei das Medikament zur Erhöhung des Spiegels von zirkulierendem endogenem Progesteron bei einem weiblichen Säugetier vorgesehen ist.

11. Pharmazeutische Zusammensetzung zur Verwendung zur Induktion der Ovulation oder einer Polyovulation bei einem weiblichen Säugetier, **dadurch gekennzeichnet, dass** sie einen Liganden nach einem der Ansprüche 1 bis 5 und/oder einen Komplex nach Anspruch 7 und ein pharmazeutisch unbedenkliches Vehikel umfasst.

12. Pharmazeutische Zusammensetzung zur Verwendung nach Anspruch 11, **dadurch gekennzeichnet, dass** sie außerdem ein FSH, ein LH und/oder ein CG umfasst.

13. Ligand nach einem der Ansprüche 1 bis 5 oder Komplex nach Anspruch 7 zur Verwendung bei der Behandlung und/oder Prävention von Unfruchtbarkeit oder verminderter Fruchtbarkeit bei einem Säugetier.

14. Ligand nach einem der Ansprüche 1 bis 5 oder Komplex nach Anspruch 7 zur Verwendung zur Stimulation der Fortpflanzung bei einem gesunden weiblichen Säugetier.

## Claims

1. Follicle-stimulating hormone (FSH) ligand which potentiates the bioactivity of FSH, of luteinizing hormone (LH), and of chorionic gonadotropin (CG), **characterized in that** said ligand is an antibody or a fragment thereof and **in that**:
the heavy chain variable domain contains the following CDRs:
- VH-CDR1, defined by the sequence GFTFSSSY (SEQ ID NO: 5);
- VH-CDR2, defined by the sequence IYAGTGGT (SEQ ID NO: 6);
- VH-CDR3, defined by the sequence ARHGSYFDY (SEQ ID NO: 7); and
the light chain variable domain contains the following CDRs:
- VL-CDR1, defined by the sequence QSVDYDGDSY (SEQ ID NO: 8);
- VL-CDR2, defined by the sequence AAS;
- VL-CDR3, defined by the sequence QQSNEDPYT (SEQ ID NO: 9).

2. Follicle-stimulating hormone (FSH) ligand which potentiates the bioactivity of FSH, of luteinizing hormone (LH), and of chorionic gonadotropin (CG), **characterized in that** said ligand is an antibody or a fragment thereof and **in that**:
the heavy chain variable domain contains the sequence of SEQ ID NO: 2 or 30;
and/or
the light chain variable domain contains the sequence SEQ ID NO: 4 or 28.

3. Ligand according to claim 1 or 2, **characterized in that** the ligand is chosen from the group consisting of: Fab, Fab', F(ab')2, Fv, dsFv, scFv, diabodies, triabodies, tetrabodies, heavy chain variable domain, and light chain variable domain.

4. Ligand according to claim 1, **characterized in that** the ligand is the CF12 monoclonal antibody produced by the CNCM 1-4803 hybridoma.

5. Ligand according to claim 1, **characterized in that** the peptide sequence of the scFv is the sequence of SEQ ID NO: 11.

6. Ligand according to any one of claims 1 to 5, for use as a medicament.

7. Ligand-gonadotropin complex chosen from:
a complex of a ligand according to any one of claims 1 to 5 with FSH or an active peptide thereof;
a complex of a ligand according to any one of claims 1 to 5 with LH or the chorionic gonadotropin (CG) hormone or an active peptide thereof.

8. Complex according to claim 7, for use as a medicament.

9. Ligand for use according to claim 6 or complex for use according to claim 8, wherein the medicament is intended for inducing ovulation or polyovulation in a female mammal.

10. Ligand for use according to claim 6 or complex for use according to claim 8, wherein the medicament is intended for increasing circulating endogenous progesterone level in a female mammal.

11. Pharmaceutical composition intended to be used for inducing ovulation or polyovulation in a female mammal, **characterized in that** it comprises a ligand according to any one of claims 1 to 5 and/or a complex according to claim 7 and a pharmaceutically acceptable carrier.

12. Pharmaceutical composition according to claim 11, **characterized in that** it also comprises an FSH and/or an LH and/or a CG.

13. Ligand according to any one of claims 1 to 5 or complex according to claim 7, for use in the treatment and/or prevention of infertility or hypofertility in a mammal.

14. Ligand according to any one of claims 1 to 5 or complex according to claim 7, for use for stimulating procreation in a female mammal.


### SEQUENCE LISTING

<110> REPROPHARM
   KARA, Elodie
   DECOURTYE, Jérémye
   CASTERET, Sophie
   MAUREL, Marie-Christine
<120> LIGANDS POTENTIALISANTS DE LA BIOACTIVITE DES GONADOTROPHINES
<130> BNT213486PC00
<150> FR 1458469
   <151> 2014-09-10
<160> 43
<170> PatentIn version 3.5
<210> 1
   <211> 348
   <212> DNA
   <213> Artificial Sequence
<220>
   <223> VH CF12
<400> 1 
cagggtcaga tgcagcagtc tggagctgag ctggtgaagc ctggggcttc agtgaagctg 60

tcctgcaaga cttctggctt caccttcagc agtagctata taagttggtt gaagcaaaag 120

cctggacaga gtcttgagtg gattgcatgg atttatgctg gaactggtgg tactagctat 180

aatcagaagt tcacaggcaa ggcccaactg actgtagaca catcctccag cacagcctac 240

atgcaattca gcagcctgac aactgaggac tctgccatct attactgtgc aagacacggg 300

tcctactttg actactgggg ccaaggcacc actctcacag tctcctca 348
<210> 2
   <211> 116
   <212> PRT
   <213> Artificial Sequence
<220>
   <223> VH CF12
<400> 2 
Gln Gly Gln Met Gln Gln Ser Gly Ala Glu Leu Val Lys Pro Gly Ala
1               5                   10                  15

Ser Val Lys Leu Ser Cys Lys Thr Ser Gly Phe Thr Phe Ser Ser Ser
            20                  25                  30

Tyr Ile Ser Trp Leu Lys Gln Lys Pro Gly Gln Ser Leu Glu Trp Ile
        35                  40                  45
 
Ala Trp Ile Tyr Ala Gly Thr Gly Gly Thr Ser Tyr Asn Gln Lys Phe
    50                  55                  60

Thr Gly Lys Ala Gln Leu Thr Val Asp Thr Ser Ser Ser Thr Ala Tyr
65                  70                  75                  80

Met Gln Phe Ser Ser Leu Thr Thr Glu Asp Ser Ala Ile Tyr Tyr Cys
                85                  90                  95

Ala Arg His Gly Ser Tyr Phe Asp Tyr Trp Gly Gln Gly Thr Thr Leu
            100                 105                 110

Thr Val Ser Ser
        115
<210> 3
   <211> 333
   <212> DNA
   <213> Artificial **Sequence**
<220>
   <223> VL CF12
<400> 3 
gacattgtgc tgacccaatc tccagcttct ttggctgtgt ctctagggca gagggccacc 60

atctcctgca aggccagcca aagtgttgat tatgatggtg atagttatat gaactggtac 120

caacagaaac caggacagcc acccaaactc ctcatctatg ctgcatccaa tctagaatct 180

gggatcccag ccaggtttag tggcagtggg tctgggacag acttcaccct caacatccat 240

cctgtggagg aggaggatgc tgcaacctat tactgtcagc aaagtaatga ggatccgtac 300

acgttcggag gggggaccaa gctggaaata aaa 333
<210> 4
   <211> 111
   <212> PRT
   <213> Artificial Sequence
<220>
   <223> VL CF12
<400> 4 
Asp Ile Val Leu Thr Gln Ser Pro Ala Ser Leu Ala Val Ser Leu Gly
1               5                   10                  15

Gln Arg Ala Thr Ile Ser Cys Lys Ala Ser Gln Ser Val Asp Tyr Asp
            20                  25                  30

Gly Asp Ser Tyr Met Asn Trp Tyr Gln Gln Lys Pro Gly Gln Pro Pro
        35                  40                  45
 
Lys Leu Leu Ile Tyr Ala Ala Ser Asn Leu Glu Ser Gly Ile Pro Ala
    50                  55                  60

Arg Phe Ser Gly Ser Gly Ser Gly Thr Asp Phe Thr Leu Asn Ile His
65                  70                  75                  80

Pro Val Glu Glu Glu Asp Ala Ala Thr Tyr Tyr Cys Gln Gln Ser Asn
                85                  90                  95

Glu Asp Pro Tyr Thr Phe Gly Gly Gly Thr Lys Leu Glu Ile Lys
            100                 105                 110
<210> 5
   <211> 8
   <212> PRT
   <213> Artificial Sequence
<220>
   <223> VH-CDR1 CF12
<400> 5 
Gly Phe Thr Phe Ser Ser Ser Tyr
1               5
<210> 6
   <211> 8
   <212> PRT
   <213> Artificial Sequence
<220>
   <223> VH-CDR2 CF12
<400> 6 
Ile Tyr Ala Gly Thr Gly Gly Thr
1               5
<210> 7
   <211> 9
   <212> PRT
   <213> Artificial Sequence
<220>
   <223> VH-CDR3 CF12
<400> 7 
Ala Arg His Gly Ser Tyr Phe Asp Tyr
1               5
<210> 8
   <211> 10
   <212> PRT
   <213> Artificial Sequence
<220>
   <223> VL-CDR1 CF12
<400> 8 
Gln Ser Val Asp Tyr Asp Gly Asp Ser Tyr
1               5                   10
<210> 9
   <211> 9
   <212> PRT
   <213> Artificial Sequence
<220>
   <223> VL-CDR3 CF12
<400> 9 
Gln Gln Ser Asn Glu Asp Pro Tyr Thr
1               5
<210> 10
   <211> 765
   <212> DNA
   <213> Artificial Sequence
<220>
   <223> ScFv CF12
<400> 10 
caggtgcagc tgcagcagtc gggtggcgca gagctggtga aaccgggtgc gagcgttaaa 60

ctgagctgca aaactagcgg ctttaccttt agctcgtcat atatttcgtg gctgaagcag 120

aaaccgggcc agtcactgga atggattgcg tggatctacg caggcacggg tggcacctca 180

tataatcaga aattcaccgg taaagcgcaa ctgacggtcg ataccagcag cagcacggcg 240

tacatgcagt tcagctcgct gaccactgaa gatagcgcaa tctactattg tgcacgccat 300

ggttcgtact tcgactattg gggccagggc accaccctga ccgtttcaag cggtggtggt 360

ggtagcggtg gtggtggttc aggtggcggc ggctcagata ttcagatgac ccagacccct 420

gcgagcctgg cagtgtcact gggccaacgc gcaaccatct cgtgtaaagc ctcgcagagc 480

gtggattatg acggcgatag ctacatgaac tggtatcagc aaaagcctgg tcaaccgccg 540

aagctgctga tttacgccgc cagcaacctg gaatcgggca tcccggcccg ttttagcggc 600

tcaggctcgg gtactgactt cacgctgaac attcacccgg tagaagaaga agacgcggcc 660

acgtattact gccagcaaag caatgaagac ccgtacactt ttggcggcgg cacgaaactc 720

gagatcaaac accatcacca tcaccattaa ctcgagatca agtaa 765
<210> 11
   <211> 249
   <212> PRT
   <213> Artificial Sequence
<220>
   <223> ScFv CF12
<400> 11 
Gln Val Gln Leu Gln Gln Ser Gly Gly Ala Glu Leu Val Lys Pro Gly
1               5                   10                  15

Ala Ser Val Lys Leu Ser Cys Lys Thr Ser Gly Phe Thr Phe Ser Ser
            20                  25                  30

Ser Tyr Ile Ser Trp Leu Lys Gln Lys Pro Gly Gln Ser Leu Glu Trp
        35                  40                  45

Ile Ala Trp Ile Tyr Ala Gly Thr Gly Gly Thr Ser Tyr Asn Gln Lys
    50                  55                  60

Phe Thr Gly Lys Ala Gln Leu Thr Val Asp Thr Ser Ser Ser Thr Ala
65                  70                  75                  80

Tyr Met Gln Phe Ser Ser Leu Thr Thr Glu Asp Ser Ala Ile Tyr Tyr
                85                  90                  95

Cys Ala Arg His Gly Ser Tyr Phe Asp Tyr Trp Gly Gln Gly Thr Thr
            100                 105                 110

Leu Thr Val Ser Ser Gly Gly Gly Gly Ser Gly Gly Gly Gly Ser Gly
        115                 120                 125

Gly Gly Gly Ser Asp Ile Gln Met Thr Gln Thr Pro Ala Ser Leu Ala
    130                 135                 140

Val Ser Leu Gly Gln Arg Ala Thr Ile Ser Cys Lys Ala Ser Gln Ser
145                 150                 155                 160

Val Asp Tyr Asp Gly Asp Ser Tyr Met Asn Trp Tyr Gln Gln Lys Pro
                165                 170                 175

Gly Gln Pro Pro Lys Leu Leu Ile Tyr Ala Ala Ser Asn Leu Glu Ser
            180                 185                 190

Gly Ile Pro Ala Arg Phe Ser Gly Ser Gly Ser Gly Thr Asp Phe Thr
        195                 200                 205

Leu Asn Ile His Pro Val Glu Glu Glu Asp Ala Ala Thr Tyr Tyr Cys
    210                 215                 220
 
Gln Gln Ser Asn Glu Asp Pro Tyr Thr Phe Gly Gly Gly Thr Lys Leu
225                 230                 235                 240

Glu Ile Lys His His His His His His
                245
<210> 12
   <211> 35
   <212> DNA
   <213> Artificial Sequence
<220>
   <223> VHRev1
<400> 12
   cgggatcctc tagaggtcca actgcaggag tcagg 35
<210> 13
   <211> 37
   <212> DNA
   <213> Artificial Sequence
<220>
   <223> VHRev2
<400> 13
   agatctagaa agcttaggtc aagctgcagc agtcagg 37
<210> 14
   <211> 20
   <212> DNA
   <213> Artificial Sequence
<220>
   <223> MuCFor
<400> 14
   ggggaagaca tttgggaagg 20
<210> 15
   <211> 21
   <212> DNA
   <213> Artificial Sequence
<220>
   <223> MKRev2
<400> 15
   gatattgtga tgacgcaggc t 21
<210> 16
   <211> 18
   <212> DNA
   <213> Artificial Sequence
<220>
   <223> MKRev3
<400> 16
   gatattgtga taacccag 18
<210> 17
   <211> 21
   <212> DNA
   <213> Artificial Sequence
<220>
   <223> MKRev4
<400> 17
   gacattgtgc tgacccaatc t 21
<210> 18
   <211> 21
   <212> DNA
   <213> Artificial Sequence
<220>
   <223> MKRev5
<400> 18
   gacattgtga tgacccagtc t 21
<210> 19
   <211> 21
   <212> DNA
   <213> Artificial Sequence
<220>
   <223> MKRev8
<400> 19
   gacatccagc tgactcagtc t 21
<210> 20
   <211> 21
   <212> DNA
   <213> Artificial Sequence
<220>
   <223> MKC5For
<400> 20
   ggatacagtt ggtgcagcat c 21
<210> 21
   <211> 21
   <212> DNA
   <213> Artificial Sequence
<220>
   <223> CF12VH_Fw
<400> 21
   cagkaactgc aggtgtccwc t 21
<210> 22
   <211> 23
   <212> DNA
   <213> Artificial Sequence
<220>
   <223> CF12VH_Rev
<400> 22
   ctggaggatg tgtctacagt cag 23
<210> 23
   <211> 21
   <212> DNA
   <213> Artificial Sequence
<220>
   <223> CF12VL_Fw
<400> 23
   ctgctatggg tgctgctgct c 21
<210> 24
   <211> 23
   <212> DNA
   <213> Artificial Sequence
<220>
   <223> CF12VL-Rev
<400> 24
   agattggatg cagcatagat gag 23
<210> 25
   <211> 14
   <212> PRT
   <213> Artificial Sequence
<220>
   <223> Lieur
<400> 25 
Gly Gly Gly Gly Ser Gly Gly Gly Gly Ser Gly Gly Gly Ser
1               5                   10
<210> 26
   <211> 16
   <212> PRT
   <213> Artificial Sequence
<220>
   <223> Fragment VL de scFv
<400> 26 Leu Glu Ile Lys His His His His His His Leu Glu Ile Lys Val Asp 1 5 10 15
<210> 27
   <211> 354
   <212> DNA
   <213> Artificial Sequence
<220>
   <223> VL CF12
<400> 27 
gatattcaga tgacccagac ccctgcgagc ctggcagtgt cactgggcca acgcgcaacc 60

atctcgtgta aagcctcgca gagcgtggat tatgacggcg atagctacat gaactggtat 120

cagcaaaagc ctggtcaacc gccgaagctg ctgatttacg ccgccagcaa cctggaatcg 180

ggcatcccgg cccgttttag cggctcaggc tcgggtactg acttcacgct gaacattcac 240

ccggtagaag aagaagacgc ggccacgtat tactgccagc aaagcaatga agacccgtac 300

acttttggcg gcggcacgaa acttgagatc aaacaccatc accatcacca ttaa 354
<210> 28
   <211> 117
   <212> PRT
   <213> Artificial Sequence
<220>
   <223> VL CF12
<400> 28 
Asp Ile Gln Met Thr Gln Thr Pro Ala Ser Leu Ala Val Ser Leu Gly
1               5                   10                  15

Gln Arg Ala Thr Ile Ser Cys Lys Ala Ser Gln Ser Val Asp Tyr Asp
            20                  25                  30

Gly Asp Ser Tyr Met Asn Trp Tyr Gln Gln Lys Pro Gly Gln Pro Pro
        35                  40                  45

Lys Leu Leu Ile Tyr Ala Ala Ser Asn Leu Glu Ser Gly Ile Pro Ala
    50                  55                  60

Arg Phe Ser Gly Ser Gly Ser Gly Thr Asp Phe Thr Leu Asn Ile His
65                  70                  75                  80

Pro Val Glu Glu Glu Asp Ala Ala Thr Tyr Tyr Cys Gln Gln Ser Asn
                85                  90                  95

Glu Asp Pro Tyr Thr Phe Gly Gly Gly Thr Lys Leu Glu Ile Lys His
            100                 105                 110
 
His His His His His
        115
<210> 29
   <211> 372
   <212> DNA
   <213> Artificial Sequence
<220>
   <223> VH CF12
<400> 29 
caggtgcagc tgcagcagtc gggtggcgca gagctggtga aaccgggtgc gagcgttaaa 60

ctgagctgca aaactagcgg ctttaccttt agctcgtcat atatttcgtg gctgaagcag 120

aaaccgggcc agtcactgga atggattgcg tggatctacg caggcacggg tggcacctca 180

tataatcaga aattcaccgg taaagcgcaa ctgacggtcg ataccagcag cagcacggcg 240

tacatgcagt tcagctcgct gaccactgaa gatagcgcaa tctactattg tgcacgccat 300

ggttcgtact tcgactattg gggccagggc accaccctga ccgtttcaag ccaccatcac 360

catcaccatt aa 372
<210> 30
   <211> 123
   <212> PRT
   <213> Artificial Sequence
<220>
   <223> VH CF12
<400> 30 
Gln Val Gln Leu Gln Gln Ser Gly Gly Ala Glu Leu Val Lys Pro Gly
1               5                   10                  15

Ala Ser Val Lys Leu Ser Cys Lys Thr Ser Gly Phe Thr Phe Ser Ser
            20                  25                  30

Ser Tyr Ile Ser Trp Leu Lys Gln Lys Pro Gly Gln Ser Leu Glu Trp
        35                  40                  45

Ile Ala Trp Ile Tyr Ala Gly Thr Gly Gly Thr Ser Tyr Asn Gln Lys
    50                  55                  60

Phe Thr Gly Lys Ala Gln Leu Thr Val Asp Thr Ser Ser Ser Thr Ala
65                  70                  75                  80

Tyr Met Gln Phe Ser Ser Leu Thr Thr Glu Asp Ser Ala Ile Tyr Tyr
                85                  90                  95

Cys Ala Arg His Gly Ser Tyr Phe Asp Tyr Trp Gly Gln Gly Thr Thr
 
100                 105                 110

Leu Thr Val Ser Ser His His His His His His
        115                 120
<210> 31
   <211> 750
   <212> DNA
   <213> Artificial Sequence
<220>
   <223> ScFv CF12 inverse
<400> 31 
gatattcaga tgacccagac ccctgcgagc ctggcagtgt cactgggcca acgcgcaacc 60

atctcgtgta aagcctcgca gagcgtggat tatgacggcg atagctacat gaactggtat 120

cagcaaaagc ctggtcaacc gccgaagctg ctgatttacg ccgccagcaa cctggaatcg 180

ggcatcccgg cccgttttag cggctcaggc tcgggtactg acttcacgct gaacattcac 240

ccggtagaag aagaagacgc ggccacgtat tactgccagc aaagcaatga agacccgtac 300

acttttggcg gcggcacgaa acttgagatc aaaggtggtg gtggtagcgg tggtggtggt 360

tcaggtggcg gcggctcaca ggtgcagctg cagcagtcgg gtggcgcaga gctggtgaaa 420

ccgggtgcga gcgttaaact gagctgcaaa actagcggct ttacctttag ctcgtcatat 480

atttcgtggc tgaagcagaa accgggccag tcactggaat ggattgcgtg gatctacgca 540

ggcacgggtg gcacctcata taatcagaaa ttcaccggta aagcgcaact gacggtcgat 600

accagcagca gcacggcgta catgcagttc agctcgctga ccactgaaga tagcgcaatc 660

tactattgtg cacgccatgg ttcgtacttc gactattggg gccagggcac caccctgacc 720

gtttcaagcc accatcacca tcaccattaa 750
<210> 32
   <211> 249
   <212> PRT
   <213> Artificial Sequence
<220>
   <223> ScFv CF12 inverse
<400> 32 
Asp Ile Gln Met Thr Gln Thr Pro Ala Ser Leu Ala Val Ser Leu Gly
1               5                   10                  15

Gln Arg Ala Thr Ile Ser Cys Lys Ala Ser Gln Ser Val Asp Tyr Asp
            20                  25                  30

Gly Asp Ser Tyr Met Asn Trp Tyr Gln Gln Lys Pro Gly Gln Pro Pro
        35                  40                  45
 
Lys Leu Leu Ile Tyr Ala Ala Ser Asn Leu Glu Ser Gly Ile Pro Ala
    50                  55                  60

Arg Phe Ser Gly Ser Gly Ser Gly Thr Asp Phe Thr Leu Asn Ile His
65                  70                  75                  80

Pro Val Glu Glu Glu Asp Ala Ala Thr Tyr Tyr Cys Gln Gln Ser Asn
                85                  90                  95

Glu Asp Pro Tyr Thr Phe Gly Gly Gly Thr Lys Leu Glu Ile Lys Gly
            100                 105                 110

Gly Gly Gly Ser Gly Gly Gly Gly Ser Gly Gly Gly Gly Ser Gln Val
        115                 120                 125

Gln Leu Gln Gln Ser Gly Gly Ala Glu Leu Val Lys Pro Gly Ala Ser
    130                 135                 140

Val Lys Leu Ser Cys Lys Thr Ser Gly Phe Thr Phe Ser Ser Ser Tyr
145                 150                 155                 160

Ile Ser Trp Leu Lys Gln Lys Pro Gly Gln Ser Leu Glu Trp Ile Ala
                165                 170                 175

Trp Ile Tyr Ala Gly Thr Gly Gly Thr Ser Tyr Asn Gln Lys Phe Thr
            180                 185                 190

Gly Lys Ala Gln Leu Thr Val Asp Thr Ser Ser Ser Thr Ala Tyr Met
        195                 200                 205

Gln Phe Ser Ser Leu Thr Thr Glu Asp Ser Ala Ile Tyr Tyr Cys Ala
    210                 215                 220

Arg His Gly Ser Tyr Phe Asp Tyr Trp Gly Gln Gly Thr Thr Leu Thr
225                 230                 235                 240

Val Ser Ser His His His His His His
                245
<210> 33
   <211> 92
   <212> PRT
   <213> Homo sapiens
<220>
   <221> misc_feature
   <223> Sous-unité alpha hFSH, hCG et HLH
<220>
   <221> misc_feature
   <222> (17)..(17)
   <223> Xaa = Leu ou Phe
<400> 33 
Ala Pro Asp Val Gln Asp Cys Pro Glu Cys Thr Leu Gln Glu Asn Pro
1               5                   10                  15

Xaa Phe Ser Gln Pro Gly Ala Pro Ile Leu Gln Cys Met Gly Cys Cys
            20                  25                  30

Phe Ser Arg Ala Tyr Pro Thr Pro Leu Arg Ser Lys Lys Thr Met Leu
        35                  40                  45

Val Gln Lys Asn Val Thr Ser Glu Ser Thr Cys Cys Val Ala Lys Ser
    50                  55                  60

Tyr Asn Arg Val Thr Val Met Gly Gly Phe Lys Val Glu Asn His Thr
65                  70                  75                  80

Ala Cys His Cys Ser Thr Cys Tyr Tyr His Lys Ser
                85                  90
<210> 34
   <211> 96
   <212> PRT
   <213> Ovis aries
<220>
   <221> misc_feature
   <223> Sous-unité alpha oLH et oFSH
<400> 34 
Phe Pro Asp Gly Glu Phe Thr Met Gln Gly Cys Pro Glu Cys Lys Leu
1               5                   10                  15

Lys Glu Asn Lys Tyr Phe Ser Lys Pro Asp Ala Pro Ile Tyr Gln Cys
            20                  25                  30

Met Gly Cys Cys Phe Ser Arg Ala Tyr Pro Thr Pro Ala Arg Ser Lys
        35                  40                  45

Lys Thr Met Leu Val Pro Lys Asn Ile Thr Ser Glu Ala Thr Cys Cys
    50                  55                  60

Val Ala Lys Ala Phe Thr Lys Ala Thr Val Met Gly Asn Val Arg Val
65                  70                  75                  80
 
Glu Asn His Thr Glu Cys His Cys Ser Thr Cys Tyr Tyr His Lys Ser
                85                  90                  95
<210> 35
   <211> 96
   <212> PRT
   <213> Sus scrofa
<220>
   <221> misc_feature
   <223> sous-unité alpha pLH et pFSH
<400> 35 
Phe Pro Asp Gly Glu Phe Thr Met Gln Gly Cys Pro Glu Cys Lys Leu
1               5                   10                  15

Lys Glu Asn Lys Tyr Phe Ser Lys Leu Gly Ala Pro Ile Tyr Gln Cys
            20                  25                  30

Met Gly Cys Cys Phe Ser Arg Ala Tyr Pro Thr Pro Ala Arg Ser Lys
        35                  40                  45

Lys Thr Met Leu Val Pro Lys Asn Ile Thr Ser Glu Ala Thr Cys Cys
    50                  55                  60

Val Ala Lys Ala Phe Thr Lys Ala Thr Val Met Gly Asn Ala Arg Val
65                  70                  75                  80

Glu Asn His Thr Glu Cys His Cys Ser Thr Cys Tyr Tyr His Lys Ser
                85                  90                  95
<210> 36
   <211> 111
   <212> PRT
   <213> Homo sapiens
<220>
   <221> misc_feature
   <223> sous-unité bêta hFSH
<400> 36 
Asn Ser Cys Glu Leu Thr Asn Ile Thr Ile Ala Ile Glu Lys Glu Glu
1               5                   10                  15

Cys Arg Phe Cys Ile Ser Ile Asn Thr Thr Trp Cys Ala Gly Tyr Cys
            20                  25                  30

Tyr Thr Arg Asp Leu Val Tyr Lys Asp Pro Ala Arg Pro Lys Ile Gln
        35                  40                  45
 
Lys Thr Cys Thr Phe Lys Glu Leu Val Tyr Glu Thr Val Arg Val Pro
    50                  55                  60

Gly Cys Ala His His Ala Asp Ser Leu Tyr Thr Tyr Pro Val Ala Thr
65                  70                  75                  80

Gln Cys His Cys Gly Lys Cys Asp Ser Asp Ser Thr Asp Cys Thr Val
                85                  90                  95

Arg Gly Leu Gly Pro Ser Tyr Cys Ser Phe Gly Glu Met Lys Glu
            100                 105                 110
<210> 37
   <211> 132
   <212> PRT
   <213> Homo sapiens
<220>
   <221> misc_feature
   <223> sous-unité bêta hCG
<400> 37 
Ser Lys Glu Pro Leu Arg Pro Arg Cys Arg Pro Ile Asn Ala Thr Leu
1               5                   10                  15

Ala Val Glu Lys Glu Gly Cys Pro Val Cys Ile Thr Val Asn Thr Thr
            20                  25                  30

Ile Cys Ala Gly Tyr Cys Pro Thr Met Thr Arg Val Leu Gln Gly Val
        35                  40                  45

Leu Pro Ala Leu Pro Gln Val Val Cys Asn Tyr Arg Asp Val Arg Phe
    50                  55                  60

Glu Ser Ile Arg Leu Pro Gly Cys Pro Arg Gly Val Asn Pro Val Val
65                  70                  75                  80

Ser Tyr Ala Val Ala Leu Ser Cys Gln Cys Ala Leu Cys Arg Arg Ser
                85                  90                  95

Thr Thr Asp Cys Gly Gly Pro Lys Asp His Pro Leu Thr Cys Asp Asp
            100                 105                 110

Pro Arg Phe Gln Asp Ser Ser Ser Ser Lys Ala Pro Pro Pro Ser Leu
        115                 120                 125

Pro Ser Pro Ser
    130
<210> 38
   <211> 123
   <212> PRT
   <213> Homo sapiens
<220>
   <221> misc_feature
   <223> sous-unité bêta hLH
<400> 38 
Ser Arg Glu Pro Leu Arg Pro Arg Pro Trp Cys His Pro Ile Asn Ala
1               5                   10                  15

Ile Leu Ala Val Glu Lys Glu Gly Cys Pro Val Cys Ile Thr Val Asn
            20                  25                  30

Thr Thr Ile Cys Ala Gly Tyr Cys Pro Thr Met Met Arg Val Leu Gln
        35                  40                  45

Ala Val Leu Pro Pro Leu Pro Gln Val Val Cys Thr Tyr Arg Asp Val
    50                  55                  60

Arg Phe Glu Ser Ile Arg Leu Pro Gly Cys Pro Arg Gly Val Asp Pro
65                  70                  75                  80

Val Val Ser Phe Pro Val Ala Leu Ser Cys Arg Cys Gly Pro Cys Arg
                85                  90                  95

Arg Ser Thr Ser Asp Cys Gly Gly Pro Lys Asp His Pro Leu Thr Cys
            100                 105                 110

Asp His Pro Gln Leu Ser Gly Leu Leu Phe Leu
        115                 120
<210> 39
   <211> 121
   <212> PRT
   <213> Ovis aries
<220>
   <221> misc_feature
   <223> sous-unité bêta oLH
<400> 39 
Ser Arg Gly Pro Leu Arg Pro Leu Cys Gln Pro Ile Asn Ala Thr Leu
1               5                   10                  15
 
Ala Ala Glu Lys Glu Ala Cys Pro Val Cys Ile Thr Phe Thr Thr Ser
            20                  25                  30

Ile Cys Ala Gly Tyr Cys Leu Ser Met Lys Arg Val Leu Pro Val Ile
        35                  40                  45

Leu Pro Pro Met Pro Gln Arg Val Cys Thr Tyr His Glu Leu Arg Phe
    50                  55                  60

Ala Ser Val Arg Leu Pro Gly Cys Pro Pro Gly Val Asp Pro Met Val
65                  70                  75                  80

Ser Phe Pro Val Ala Leu Ser Cys His Cys Gly Pro Cys Arg Leu Ser
                85                  90                  95

Ser Thr Asp Cys Gly Gly Pro Arg Thr Gln Pro Leu Ala Cys Asp His
            100                 105                 110

Pro Pro Leu Pro Asp Ile Leu Phe Leu
        115                 120
<210> 40
   <211> 121
   <212> PRT
   <213> Sus scrofa
<220>
   <221> misc_feature
   <223> sous-unité bêta pLH
<400> 40 
Ser Arg Gly Pro Leu Arg Pro Leu Cys Arg Pro Ile Asn Ala Thr Leu
1               5                   10                  15

Ala Ala Glu Asn Glu Ala Cys Pro Val Cys Ile Thr Phe Thr Thr Ser
            20                  25                  30

Ile Cys Ala Gly Tyr Cys Pro Ser Met Val Arg Val Leu Pro Ala Ala
        35                  40                  45

Leu Pro Pro Val Pro Gln Pro Val Cys Thr Tyr Arg Glu Leu Ser Phe
    50                  55                  60

Ala Ser Ile Arg Leu Pro Gly Cys Pro Pro Gly Val Asp Pro Thr Val
65                  70                  75                  80

Ser Phe Pro Val Ala Leu Ser Cys His Cys Gly Pro Cys Arg Leu Ser
                85                  90                  95
 
Ser Ser Asp Cys Gly Gly Pro Arg Ala Gln Pro Leu Ala Cys Asp Arg
            100                 105                 110

Pro Leu Leu Pro Gly Leu Leu Phe Leu
        115                 120
<210> 41
   <211> 110
   <212> PRT
   <213> Ovis aries
<220>
   <221> misc_feature
   <223> sous-unité bêta oFSH
<400> 41 
Ser Cys Glu Leu Thr Asn Ile Thr Ile Thr Val Glu Lys Glu Glu Cys
1               5                   10                  15

Ser Phe Cys Ile Ser Ile Asn Thr Thr Trp Cys Ala Gly Tyr Cys Tyr
            20                  25                  30

Thr Arg Asp Leu Val Tyr Lys Asp Pro Ala Arg Pro Asn Ile Gln Lys
        35                  40                  45

Ala Cys Thr Phe Lys Glu Leu Val Tyr Glu Thr Val Lys Val Pro Gly
    50                  55                  60

Cys Ala His His Ala Asp Ser Leu Tyr Thr Tyr Pro Val Ala Thr Glu
65                  70                  75                  80

Cys His Cys Gly Lys Cys Asp Arg Asp Ser Thr Asp Cys Thr Val Arg
                85                  90                  95

Gly Leu Gly Pro Ser Tyr Cys Ser Phe Ser Asp Ile Arg Glu
            100                 105                 110
<210> 42
   <211> 109
   <212> PRT
   <213> Sus scrofa
<220>
   <221> misc_feature
   <223> sous-unité bêta pFSH
<400> 42 
Cys Glu Leu Thr Asn Ile Thr Ile Thr Val Glu Lys Glu Glu Cys Gly
1               5                   10                  15
 
Phe Cys Ile Ser Ile Asn Thr Thr Trp Cys Ala Gly Tyr Cys Tyr Thr
        20                  25                  30

Arg Asp Leu Val Tyr Lys Asp Pro Ala Arg Pro Asn Ile Gln Lys Thr
    35                  40                  45

Cys Thr Phe Lys Glu Leu Val Tyr Glu Thr Val Lys Val Pro Gly Cys
    50                  55                  60

Ala His His Ala Asp Ser Leu Tyr Thr Tyr Pro Val Ala Thr Glu Cys
65                  70                  75                  80

His Cys Gly Lys Cys Asp Ser Asp Ser Thr Asp Cys Thr Val Arg Gly
                85                  90                  95

Leu Gly Pro Ser Tyr Cys Ser Phe Ser Glu Met Lys Glu
        100                 105
<210> 43
   <211> 49
   <212> PRT
   <213> Homo sapiens
<220>
   <221> misc_feature
   <223> Région N terminale du récepteur de la hFSH
<400> 43

Cys His His Arg Ile Cys His Cys Ser Asn Arg Val Phe Leu Cys Gln
1               5                   10                  15

Glu Ser Lys Val Thr Glu Ile Pro Ser Asp Leu Pro Arg Asn Ala Ile
            20                  25                  30

Glu Leu Arg Phe Val Leu Thr Lys Leu Arg Val Ile Gln Lys Gly Ala
        35                  40                  45

Phe